# EUROPEAN PATENT SPECIFICATION

(11) **EP 2 139 475 B1**
(45) Date of publication and mention of the grant of the patent: **17.12.2014**
(21) Application number: 08744515.1
(22) Date of filing: 27.03.2008
(51) Int. Cl.: A61K 31/435, A61K 31/195, A61K 31/433, A61K 31/519, A61K 31/527

(54) **PDE7 inhibitors for use in the treatment of movement disorders**
PDE7-Inhibitoren zur Verwendung bei der Behandlung von Bewegungsstörungen
Inhibiteurs de PDE7 pour le traitement de troubles du mouvement

(30) Priority: 27.03.2007 US 920496 P
(43) Date of publication of application: 06.01.2010
(73) Proprietor: Omeros Corporation, Seattle, WA 98119 (US)
(72) Inventor: BERGMANN, John, E., Mercer Island, WA 98040 (US); CUTSHALL, Neil, S., Snohomish, WA 98290 (US); DEMOPULOS, Gregory, A., Mercer Island, WA 98040 (US); FLORIO, Vincent, A., Seattle, WA 98105 (US); GAITANARIS, George, Seattle, WA 98102 (US); GRAY, Patrick, Seattle, WA 98122 (US); HOHMANN, John, LaConner, WA 98257 (US); ONRUST, Rene, Mercer Island, WA 98040 (US); ZENG, Hongkui, Shoreline, WA 98133 (US)
(74) Representative: Cole, William Gwyn
(86) International application number: PCT/US2008/058530
(87) International publication number: WO 2008/119057

(56) References cited:
- EP-A1- 1 454 897
- WO-A1-02/074754
- WO-A1-2006/092691
- US-A1- 2003 045 557
- US-A1- 2003 069 169
- US-A1- 2003 195 140
- US-A1- 2004 082 061
- US-A1- 2004 162 294
- US-A1- 2004 162 294
- US-A1- 2005 148 604
- US-A1- 2005 222 138
- US-A1- 2005 250 739
- MENNITI F S ET AL: "Phosphodiesterases in the CNS: Targets for drug development", NATURE REVIEWS. DRUG DISCOVERY, NATURE PUBLISHING GROUP, GB, vol. 5, no. 8, 1 August 2006 (2006-08-01), pages 660-670, XP002601485, ISSN: 1474-1784, DOI: 10.1038/NRD2058
- M.A. GIEMBYCZ ET AL: "Phosphodiesterase 7 (PDE7) as a therapeutic target", DRUGS OF THE FUTURE, vol. 31, no. 3, 1 January 2006 (2006-01-01), page 207, XP55017087, ISSN: 0377-8282, DOI: 10.1358/dof.2006.031.03.966246
- VERGNE F. ET AL.: 'Discovery of thiadiazoles as a novel structural class of potent and selective PDE7 inhibitors. Part 1: Design, synthesis and structure-activity relationship studies' BIOORGANIC & MEDICINAL CHEMISTRY LETTERS vol. 14, 2004, pages 4607 - 4613, XP008119454
- Maurizio Giorelli ET AL: "Dopamine Fails to Regulate Activation of Peripheral Blood Lymphocytes from Multiple Sclerosis Patients: Effects of IFN-[beta]", Journal of Interferon & Cytokine Research, vol. 25, no. 7, 1 July 2005 (2005-07-01), pages 395-406, XP055046863, ISSN: 1079-9907, DOI: 10.1089/jir.2005.25.395
- Jose A. Morales-Garcia ET AL: "Phosphodiesterase 7 Inhibition Preserves Dopaminergic Neurons in Cellular and Rodent Models of Parkinson Disease", PLoS ONE, vol. 6, no. 2, 1 February 2011 (2011-02-01), page e17240, XP055102530, ISSN: 1932-6203, DOI: 10.1371/journal.pone.0017240
- DAVID P ROTELLA: 'Phosphodiesterase enzymes - target overview' 2007, pages 22 - 23

## Description

### FIELD OF THE INVENTION

The disclosure herein relates to methods of treating a movement abnormality associated with the pathology of a movement disorder comprising administering to a patient in need thereof an amount of a PDE7 inhibitory agent effective to inhibit the enzymatic activity of PDE7.

### BACKGROUND

Parkinson's disease ("PD") is a progressive disorder that affects a small group of neurons (called the substantia nigra) in the midbrain. PD is associated with the depletion of dopamine, which is important for maintaining movement control through interaction with cells in the corpus striatum. Approximately one out of every 1,000 people contract the illness and about 1% of the population over the age of 65 suffers from PD. The common symptoms of PD include tremor at rest, stiffness (or rigidity) of muscles, slowness of movement (bradykinesia) and loss of balance (postural dysfunction).

Parkinson's disease is one of three distinct conditions that can be categorized together as Parkinsonism. Parkinson's disease, or paralysis agitans, is the most common form of Parkinsonism, afflicting approximately 75% of the cases and is of unknown origin or cause. A second type of Parkinsonism is caused by drugs and toxins, including carbon monoxide, manganese and a chemical compound known as MPTP (methylphenyltetrahydropyridine). A third form of Parkinsonism, referred to as Vascular Parkinsonism, may be caused by multiple small strokes that damage the dopamine-producing brain cells.

Many treatments have been tried since James Parkinson named and described the condition in 1817. Most treatments are symptomatic therapies, such as using pharmacologic therapy (e.g., levodopa, dopamine receptor agonists, MAO-B inhibitors, COMT inhibitors) or deep brain stimulation therapy, to alleviate the symptoms of the disease. Recently, neuroprotective therapies have been the subject of intense research and development efforts.

The therapeutic combination of levodopa (L-dopa), a dopamine precursor, and a dopa decarboxylase inhibitor (carbidopa), is considered to be one of the most effective treatments for symptoms of Parkinson's disease (The Medical Letter 35:31-34, 1993). However, certain limitations of the combination become apparent within two to five years of initiating the combination therapy. As the disease progresses, the benefit from each dose becomes shorter (the "wearing off effect") and some patients fluctuate unpredictably between mobility and immobility (the "on-off effect"). "On" periods are usually associated with high plasma levodopa concentrations and often include abnormal involuntary movements (i.e., dyskinesias). "Off" periods have been correlated with low plasma levodopa concentrations and bradykinetic episodes. Therefore, a need exists for additional effective treatments for Parkinson's disease.

The salient pathologic feature of Parkinson's disease is the degeneration of dopaminergic neurons in the substantia nigra pars compacta (SNc) that project to the striatum. Forno L.S., J. Neuropathol Exp Neurol 55:259-272, 1996. It is thought that the relatively selective dopamine depletion in the striatum and other basal ganglia results in increased and disordered discharge and synchronization in motor areas of the basal ganglia-thalamocorticol motor loops. Wichmann and Delong, Neuropsychopharmacology: The Fifth Generation of Progress, Chapter 122, "Neurocircuitry of Parkinson's Disease," 2002. In addition to Parkinson's disease, abnormal function of the basal ganglia has also been implicated in a variety of neurological disorders with movement abnormalities. Such neurological disorders include restless leg(s) syndrome (Hening, W., et al., Sleep 22:970-999, 1999) and Huntington's disease (Vonsattel, J.P., et al., J. Neuropathol. Exp. Neurol. 44:559-577, 1985). The study of the consequences of the pathophysiologic changes in the basal ganglia that result from the loss of dopaminergic transmission in the basal ganglia has been facilitated by the discovery that primates and rodents treated with MPTP develop behavioral and anatomic changes that closely mimic the features of Parkinson's disease in humans. See, e.g., Bankiewicz, K.S., et al., Life Sci. 39:7-16, 1986, Bums, R.S., et al., PNAS 80:4546-4550, 1983.

Cyclic nucleotide phosphodiesterases (PDEs) represent a family of enzymes that hydrolyze the ubiquitous intracellular second messengers, adenosine 3',5'-monophosphate (cAMP) and guanosine 3',5'-monophosphate (cGMP), to their corresponding inactive 5'-monophosphate metabolites. At least 11 distinct classes of PDE isozymes (PDE1-11) are believed to exist, each possessing unique physical and kinetic characteristics and representing unique gene families. Within each distinct class of PDE, there may be up to four distinct sub-types. (Crocker, I., et al., Drugs Today 35(7):519-535, 1999; Fawcett, L., et al., PNAS 97(7):3702-3703, 2000; and Yuasa, K., et al., J. Biol. Chem. 275(40):31496-31479, 2000).

Virtually all of the phosphodiesterases are expressed in the central nervous system ("CNS"), making this gene family a particularly attractive source of new targets for the treatment of psychiatric and neurodegenerative disorders. However, all neurons express multiple phosphodiesterases, which differ in cyclic nucleotide specificity, affinity, regulatory control, and subcellular compartmentalization, making linking the target for a specific disease with the treatment of the disease difficult. Therefore, there is a need to identify a target from the family of phosphodiesterases with the treatment of a specific CNS disease, such as Parkinson's disease and other neurological disorders with movement abnormalities.

Despite the advances in the research and treatment of Parkinson's disease, a need exists for new treatments for this disease and other neurological disorders with movement abnormalities. The present invention seeks to fulfill this need and provides further related advantages.

Menniti S et al, Nature Reviews, 5, 660-670 (2006) discloses that phosphodiesterases in the CNS can be targets for drug development and refers to PDE7B and to Parkinson's disease.

### SUMMARY

The present invention is defined in the appended claims. Subject-matters which are not encompassed by the scope of the claims do not form part of the present invention.

In accordance with the foregoing, described herein are compounds for use in a method of treating a movement abnormality associated with the pathology of a neurological movement disorder. The described method comprises administering to a patient in need thereof an amount of a PDE7 inhibitory agent effective to inhibit the enzymatic activity of PDE7, wherein such inhibition of PDE7 enzymatic activity is the principal therapeutic mode of action of the PDE7 inhibitor in the treatment of the movement abnormality.

As noted above, described herein are compounds for use in a method of treating a movement abnormality associated with the pathology of a neurological disorder. The described method comprises administering to a patient in need thereof an amount of a PDE7 inhibitory agent effective to inhibit the enzymatic activity of PDE7, wherein such inhibition of PDE7 enzymatic activity is the principal therapeutic mode of action of the PDE7 inhibitor in the treatment of the movement abnormality.

Described herein are compounds for use in a method for identifying an agent that inhibits PDE7 activity useful for treating a movement abnormality associated with the pathology of a neurological movement disorder in a mammalian subject in need thereof. The described method comprises (a) determining the IC₅₀ for inhibiting PDE7A and/or PDE7B activity of each of a plurality of agents; (b) selecting agent(s) from the plurality of agents having an IC₅₀ for inhibition of PDE7A and/or PDE7B activity of less than about 1 µM; (c) determining the IC₅₀ for inhibiting PDE4 activity of the agent(s) having an IC₅₀ for inhibiting PDE7 activity of less than about 1 µM; (d) identifying agent(s) useful for treating a movement disorder by selecting compounds having an IC₅₀ for inhibiting PDE4 activity greater than 10 times the lesser of the IC₅₀ for inhibiting PDE7A activity and the IC₅₀ for inhibiting PDE7B activity; and (e) evaluating the activity of the identified compound(s) in a neurological movement disorder model assay, wherein an agent that has an IC₅₀ for PDE7A and/or PDE7B activity inhibition of less than about 1 µM, and an IC₅₀ for inhibiting PDE4 activity greater than 10 times the lesser of the IC₅₀ for inhibiting PDE7A activity and the IC₅₀ for inhibiting PDE7B activity, and is determined to be effective to treat at least one movement abnormality in a model assay, is indicative of a PDE7 inhibitory agent useful for treating a movement abnormality associated with the pathology of a neurological movement disorder in a mammalian subject.

Described herein are compounds for use in a method of treating a movement abnormality associated with the pathology of a neurological movement disorder. This method comprises administering to a patient in need thereof a therapeutically effective amount of a chemical compound that is a PDE7 inhibitor, the chemical compound characterized in that: (i) the chemical compound has an IC₅₀ for inhibiting PDE7A and/or PDE7B activity of less than about 1 µM; and (ii) the chemical compound has an IC₅₀ for inhibiting PDE 3 greater than 10 times the lesser of the IC₅₀ for inhibiting PDE7A activity and the IC₅₀ for inhibiting PDE7B activity.

Various methods described herein are useful to treat a movement abnormality associated with a neurological disorder. Various methods also useful to treat a neurological movement disorder. Various methods are further useful to treat a movement abnormality associated with a neurological movement disorder.

In some cases of the various aspects of the description, the methods are useful to treat a neurological movement disorder, a movement abnormality associated with a neurological disorder, and/or a movement abnormality associated with a neurological movement disorder, that is treatable with a dopamine receptor agonist or a precursor of a dopamine receptor agonist. In some cases, the methods are useful to treat a neurological movement disorder selected from the group of Parkinson's disease, Post-Encephalitic Parkinsonism, Dopamine-Responsive Dystonia, Shy-Drager Syndrome, Periodic Limb Movement Disorder (PLMD), Periodic Limb Movements in Sleep (PLMS), and Restless Leg(s) Syndrome (RLS).

In some cases, the methods are useful to treat a movement abnormality associated with the pathology of a neurological movement disorder and/or the pathology of a neurological disorder. In some cases, the methods are useful to treat a movement abnormality associated with the pathology of a neurological movement disorder that is treatable with a dopamine receptor agonist or a precursor of a dopamine receptor agonist. In some cases, the methods are useful to treat a movement abnormality associated with the pathology of a neurological movement disorder selected from the group of Parkinson's disease, Post-Encephalitic Parkinsonism, Dopamine-Responsive Dystonia, Shy-Drager Syndrome, Periodic Limb Movement Disorder (PLMD), Periodic Limb Movements in Sleep (PLMS), and Restless Leg(s) Syndrome (RLS).

### DESCRIPTION OF THE DRAWINGS

The foregoing aspects and many of the attendant advantages of this disclosure will become more readily appreciated as the same become better understood by reference to the following detailed description, when taken in conjunction with the accompanying drawing. In certain of these figures, statistical significance is indicated by a marking in which "*" refers to a p value of less than 0.05, "**" refers to a p value of less than 0.01, and "***" refers to a p value of less than 0.005. In the figures:
FIGURE 1 is a flowchart illustrating the neurotransmission pathway in the basal ganglia in a mid-brain of a healthy mammalian subject, with excitatory pathways labeled "+" with hatched arrows, and with inhibitory pathways labeled "-" with open arrows;
FIGURE 2A illustrates a proposed model of the dopamine receptor activated pathway in a healthy subject, illustrating the new discovery that the dopamine receptor activated intracellular signaling pathway is downregulated or antagonized by PDE7, which hydrolyzes cAMP to its 5' monophosphate (5'AMP);
FIGURE 2B illustrates a model proposed by the present inventors of the dopamine receptor activated pathway in an untreated subject with Parkinson's disease (PD), showing that the reduced amount of dopamine receptor activated intracellular signaling pathway is further downregulated or antagonized by PDE7, which hydrolyzes cAMP to its 5' monophosphate (5'AMP), leading to low levels of activated PKA and reduced neuronal activation as compared to a healthy subject;

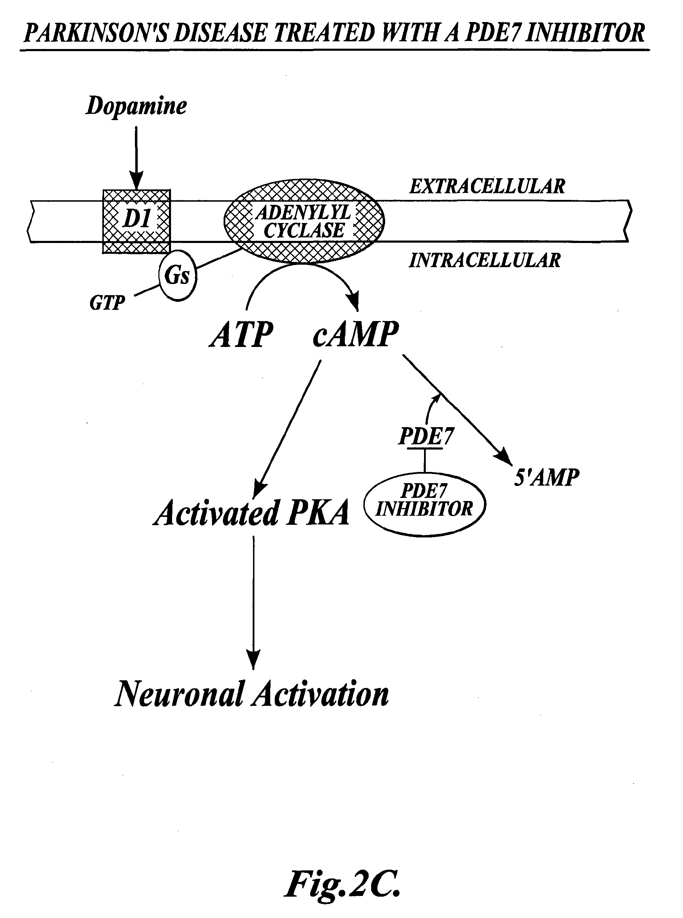

FIGURE 2C illustrates a model proposed by the present inventors of the dopamine receptor activated pathway in a subject with Parkinson's disease (PD) treated with a PDE7 inhibitory agent, showing that the presence of a PDE7 inhibitory agent that is effective to inhibit PDE7 enzymatic activity blocks the hydrolysis of cAMP, effectively increasing the intracellular cAMP levels, activating protein kinase A ("PKA"), which modulates phosphorylation of downstream elements in intracellular signaling pathways, leading to an increase in neuronal activation in accordance with various cases of the methods of the invention;
FIGURE 3A is a graph illustrating the PDE7A inhibitory activity (IC₅₀), expressed as counts per minute ("CPM"), of a representative PDE7 inhibitory agent (OM69) useful in the methods of the invention;
FIGURE 3B is a graph illustrating the PDE7B inhibitory activity (IC₅₀), expressed as CPM, of a representative PDE7 inhibitory agent (OM69) useful in the methods of the invention;
FIGURE 4A is a graph illustrating the PDE7A inhibitory activity (IC₅₀), expressed as CPM, of a representative PDE7 inhibitory agent (OM955) useful in the methods of the invention;
FIGURE 4B is a graph illustrating the PDE7B inhibitory activity (IC₅₀), expressed as CPM, of a representative PDE7 inhibitory agent (OM955) useful in the methods of the invention;
FIGURE 5A is a graph illustrating the PDE7A inhibitory activity (IC₅₀), expressed as CPM, of a representative PDE7 inhibitory agent (OM956) useful in the methods of the invention;
FIGURE 5B is a graph illustrating the PDE7B inhibitory activity (IC₅₀), expressed as CPM, of a representative PDE7 inhibitory agent (OM956) useful in the methods of the invention;
FIGURE 6A is a graph illustrating the PDE7A inhibitory activity (IC₅₀), expressed as CPM, of a representative PDE7 inhibitory agent (OM056) useful in the methods of the invention;
FIGURE 6B is a graph illustrating the PDE7B inhibitory activity (IC₅₀), expressed as CPM, of a representative PDE7 inhibitory agent (OM056) useful in the methods of the invention;
FIGURE 7 is a graph comparing the concentration (ng/g) in plasma and brain tissue over time of a representative PDE7 inhibitor (OM69) useful in the method of the invention;
FIGURE 8 is a flow diagram illustrating an experiment carried out in a methylphenyltetrahydropyridine ("MPTP") mouse model of Parkinson's disease to initially evaluate a representative PDE7 inhibitory agent (OM69) useful in the methods of the invention, administered alone or in combination with L-dopa, as compared to the effect of L-dopa alone, as described in Example 5;
FIGURE 9 is a bar graph illustrating the testing of inked paw stride length in the MPTP mouse model following the protocol illustrated in FIGURE 8, demonstrating that a representative PDE7 inhibitory agent (OM69) useful in the method of the invention increases stride length in MPTP-treated mice, when administered alone or in combination with L-dopa, and compares the effectiveness of this inhibitor to L-dopa alone and to a saline control, as described in Example 5;
FIGURE 10 is a bar graph illustrating a subset of the data shown in FIGURE 9, comparing the effect on stride length in the MPTP mouse model of various dosages of a representative PDE7 inhibitory agent (OM69 (compound 1)) useful in the method of the invention, various dosages of L-dopa , and combinations of OM69 and L-dopa, as described in Example 5;
FIGURE 11 is a bar graph illustrating a subset of the data shown in FIGURE 9, comparing the effect on stride length in the MPTP mouse model of a representative PDE7 inhibitor (OM69) useful in the method of the invention, L-dopa, and combinations thereof, as compared to saline control (i.e., non-MPTP-treated) mice, as described in Example 5;
FIGURE 12 is a flow diagram illustrating an experiment carried out in the MPTP mouse model of Parkinson's disease to confirm that the representative PDE7 inhibitor (OM69) increases stride length in MPTP-treated mice, as described in Example 6;
FIGURE 13A is a bar graph illustrating that the vehicle control dimethylacetamide: polyethylene glycol: methane sulfonic acid (DMA:PEG:MSA) did not alter stride length in MPTP-treated mice when administered alone, as described in Example 7;
FIGURE 13B is a bar graph illustrating that the vehicle control Tartaric Acid (TA) did not alter stride length in MPTP-treated mice when administered alone, as described in Example 7;
FIGURE 14 is a bar graph illustrating the testing of inked paw stride length in the MPTP mouse model demonstrating that the representative PDE7 inhibitory agent OM955 (compound 2) increases stride length in MPTP mice, with full recovery of baseline stride length at 20 minutes after a dose of 0.5 mg/kg, as described in Example 7;
FIGURE 15A is a bar graph illustrating the testing of inked paw stride length in the MPTP mouse model demonstrating that 1 mg/kg of L-dopa does not increase stride length in MPTP mice to a significant level at 20 minutes after administration, as described in Example 7;
FIGURE 15B is a bar graph illustrating the testing of inked paw stride length in the MPTP mouse model, demonstrating that 0.1 mg/kg of OM955 (compound 2) does not increase stride length in MPTP mice to a significant level at 20 minutes after administration, as described in Example 7;
FIGURE 15C is a bar graph illustrating the testing of inked paw stride length in the MPTP mouse model, demonstrating that mice administered the combination of 0.1 mg/kg of OM955 (compound 2) and 1 mg/kg L-dopa showed full recovery of stride length in MPTP-treated mice to a significant level at 20 minutes after administration, thus demonstrating synergistic results of the combination, as described in Example 7;
FIGURE 16 is a bar graph illustrating the testing of inked paw stride length in the MPTP mouse model, demonstrating that the representative PDE7 inhibitory agent OM956 (compound 3) increases stride length in MPTP-treated mice, with full recovery of baseline stride length at 20 minutes after a dose of 0.5 mg/kg, as described in Example 7;
FIGURE 17 is a bar graph illustrating the testing of inked paw stride length in the MPTP mouse model, demonstrating that the representative PDE7 inhibitory agent OM056 (compound 4) increases stride length in MPTP-treated mice, with full recovery of baseline stride length at 20 minutes after a dose of 0.05 mg/kg, as described in Example 7; and
FIGURE 18 is a bar graph illustrating the testing of inked paw stride length in the MPTP mouse model, demonstrating that the representative PDE7 inhibitory agent OM69 (compound 1) increases stride length in MPTP-treated mice in a dose-dependent manner, and further demonstrating that the combination of OM69 and L-dopa provides a greater than additive (i.e., synergistic) increase in stride length in MPTP-treated mice, as described in Example 6.

### DETAILED DESCRIPTION

The present invention is based upon the surprising discovery by the present inventors that selective inhibitors of the type 7 cyclic nucleotide phosphodiesterase (PDE7) cause a striking improvement in motor function in the mouse 1-methyl, 4-phenyl, 1,2,3,6-tetrahydropyridine (MPTP) model of Parkinson's disease (PD). Through the use of the MPTP animal model, the present inventors have shown that administration of selective PDE7 inhibitory agents in MPTP-lesioned mice is effective to restore stride length in these animals in a manner comparable to treatment with L-dopa, but at a surprisingly low dosage as compared to the dosage of L-dopa required to achieve an equivalent level of response. Furthermore, the inventors have demonstrated that the combination of suboptimal doses of L-dopa and a selective PDE7 inhibitor, when administered together, provide a greater than additive (i.e., synergistic) effect, again restoring stride length in MPTP-lesioned mice to normal values.

Accordingly, the present invention provides a compound for use in the treatment of movement abnormalities in Parkinson's disease, Post-Encephalitic Parkinsonism, Dopamine-Responsive Dystonia, Shy-Drager Syndrome, Period Limb Movement Disorder (PLMD), Periodic Limb Movements in Sleep (PLMS), and Restless Leg(s) Syndrome (RLS), by inhibition of the enzymatic activity of PDE7, which PDE7 inhibitory agent has an IC₅₀ for inhibiting the activity of PDE1, PDE2, PDE3, PDE4, PDE8, PDE10 and PDE11 of greater than 10 times the lesser of the IC₅₀ for inhibiting PDE7A activity and the IC₅₀ for inhibiting PDE7B activity.

### I. Definitions

Unless specifically defined herein, all terms used herein have the same meaning as would be understood by those of ordinary skill in the art of the present invention. The following definitions are provided in order to provide clarity with respect to the terms as they are used in the specification and claims to describe the present invention.

As used herein, the term "neurological movement disorder" refers to a movement disorder characterized by a deficiency or defect in dopamine signaling that is clinically manifested by one or more movement abnormalities associated with the pathology of the movement disorder, such as abnormal involuntary movements, tremor at rest, alterations in muscle tone, difficulty in the initiation of movement (bradykinesia) and/or disturbances in postural stability.

As used herein, the term "Parkinson's Disease" refers to a clinical syndrome marked by four cardinal signs: (1) tremor at rest; (2) rigidity, (3) bradykinesia, and (4) deficiency of postural reflexes.

As used herein, the term "Post-Encephalitic Parkinsonism" refers to Parkinsonism occurring after and presumably as a result of encephalitis.

As used herein, the term "Parkinsonism" refers to any of a group of neurological disorders similar to Parkinson's disease, marked by the four cardinal signs of Parkinson's disease: tremor at rest, muscular rigidity, bradykinesia and deficiency in postural reflexes.

As used herein, the term "bradykinesia" or "akinesia" refers to a paucity of automatic or spontaneous movement.

As used herein, the term "hyperkinesia" or "dyskinesia" refers to excessive or abnormal involuntary movement.

As used herein, the term "tremor" refers to relatively rhythmic oscillatory movements, which can, for example, result from alternating contractions of opposing muscle groups (e.g., Parkinson's tremor).

As used herein, the term "dystonia" refers to involuntary movements with sustained contractions at the end of the movement.

As used herein, the term "Dopamine-responsive Dystonia" refers to a neurological movement disorder in which sustained muscle contractions cause twisting and repetitive movements or abnormal postures, and which can be alleviated by agents that increase dopamine levels or enhance signaling through dopaminergic pathways. Such a disorder may be associated with Parkinson's disease, juvenile parkinsonism, progressive supranuclear palsy, cortical basal ganglionic degeneration, certain types of multiple system atrophy, or DYT3 X-linked recessive dystonia-parkinsonism.

As used herein, the term "Periodic Limb Movement in Sleep" (PLMS) refers to a condition in which the patient's legs move or twitch involuntarily during sleep. If these movements result in sleep disturbance, this syndrome is referred to as Periodic Limb Movement Disorder (PLMD).

As used herein, the term "Restless Leg(s) Syndrome" (RLS) refers to a neurological disorder of uncertain pathophysiology that is characterized by aching, burning, crawling, or creeping sensations of the legs that occur especially at night, usually when lying down (as before sleep) and cause a compelling urge to move the legs and that is often accompanied by difficulty in falling or staying asleep and by involuntary twitching of the legs during sleep.

As used herein, the term "Shy-Drager Syndrome" refers to a degenerative neurological disorder characterized by orthostatic hypotension, autonomic dysfunction, bladder dysfunction, and Parkinson's-like deficits in movement.

As used herein, the term "dopaminergic agent" refers to an agent which functions to enhance or replicate the effects mediated by dopamine in the central nervous system, including dopamine (if a clinically effective method of delivery should be developed), dopamine precursors, such as L-dopa, dopamine cofactors, inhibitors of enzymes that metabolize dopamine, other dopamine receptor agonists and precursor compounds that are metabolically converted to a dopamine receptor agonist, as well as dopamine reuptake inhibitors.

As used herein, the term "dopamine receptor agonist" refers to any molecule that causes the activation of one or more of the subtypes of the dopamine receptor protein family.

As used herein, the term "molecular target(s) known to be involved with the pathology of Parkinson's disease" includes catechol-O-methyltransferase (COMT), monamine oxidase B (MAO-B), dopamine transporters (DAT), tyrosine hydroxylase, dopamine receptors, adenosine A_{2A} receptors, and gabapentin receptors.

As used herein, the term "molecular target(s) known to be associated with the dopamine signaling pathway" includes catechol-O-methyltransferase (COMT), monamine oxidase B (MAO-B), dopamine transporters (DAT), tyrosine hydroxylase, dopa decarboxylase, dopamine receptors, N-methyl D-aspartate (NMDA) receptors, muscarinic acetylcholine receptors, gamma amino butyric acid (GABA) receptors, adenylyl cyclase, protein kinase A (PKA), dopamine and cyclic AMP- regulated phosphoprotein of molecular weight 32,000 (DARPP32), and protein phosphatase-1.

As used herein, the term "treatment" includes symptomatic therapy to lessen, alleviate, or mask the symptoms of the disease or disorder, as well as therapy for preventing, lowering, stopping, or reversing the progression of severity of the condition or symptoms being treated. As such, the term "treatment" includes both medical therapeutic treatment of an established condition or symptoms and/or prophylactic administration, as appropriate.

As used herein, the term "treating a movement abnormality associated with the pathology of a neurological movement disorder" refers to reversing, alleviating, ameliorating, or inhibiting one or more movement abnormalities associated with the neurological movement disorder.

As used herein, the term "treating a neurological movement disorder" includes: (1) treating a movement abnormality associated with the pathology of a neurological movement disorder; and/or (2) treating a neurological movement disorder

As used herein, the term "treating a neurological disorder" includes: (1) treating a movement abnormality associated with the pathology of a neurological disorder; and/or (2) treating a neurological disorder

As used herein, the term "treating" also encompasses, depending on the condition of the subject in need thereof, preventing the neurological movement disorder or preventing the movement abnormality associated with the pathology of the neurological movement disorder or preventing the neurological disorder or preventing the movement abnormality associated with the pathology of the neurological disorder, including onset of the movement abnormality or of any symptoms associated therewith, as well as reducing the severity of the movement abnormality, or preventing a recurrence of a movement abnormality.

As used herein the term "PDE7" is used generically to refer to all translation products coded by transcripts of either or both of these two genes (PDE7A and/or PDE7B).

As used herein, the term "PDE7 inhibitory agent" refers to an agent, such as a chemical compound, a peptide, or a nucleic acid molecule, that directly or indirectly inhibits or blocks the phosphodiesterase activity of PDE7A, PDE7B, or PDE7A and PDE7B. In some cases, the agent may bind or interact directly with PDE7 protein. An agent that binds to PDE7 may act to inhibit or block the PDE7 activation by any suitable means, such as by inhibiting the binding of cAMP or substrate ligand with PDE7. In other cases, the PDE7 inhibitory agent may inhibit PDE7 activity indirectly, such as by decreasing expression of the PDE7 protein.

As used herein, the term "mammalian subject" includes all mammals, including without limitation humans, non-human primates, dogs, cats, horses, sheep, goats, cows, rabbits, pigs, and rodents.

### II. PDE7 Inhibitory Agents to Treat a Movement Abnormality Associated With the Pathology of a Neurological Movement Disorder

The dopaminergic system is strongly implicated in the regulation of locomotor activity and movement in general. See, e.g., Tran, A.H., et al., PNAS 102:2117-2122, 2005; Tran, A.H., et al., PNAS 99:8986-8991, 2002. For example, evidence shows that a dopaminergic dysfunction plays a critical role in Parkinson's disease, Parkinsonism, Restless Leg(s) Syndrome ("RLS"), Periodic Limb Movement Disorder (PLMD), Periodic Limb Movement in Sleep ("PLMS"), and other movement disorders. In Parkinson's disease there is a deficiency of dopamine in the striatum, which results from a loss of pigmented neurons in the substantia nigra and locus ceruleus with corresponding loss of their dopamine and norepinephrine neurotransmitters. In postencephalitic Parkinsonism, the midbrain is affected, with loss of substantia nigra neurons. Wyngaarden and Smith, Cecil Textbook of Medicine, 17th Ed. "Neurological and Behavioral Disease: Section 5: The Extrapyramidal Disorders: Parkinsonism," 1985.

It is thought that the relatively selective dopamine depletion in the striatum and other basal ganglia results in increased and disordered discharge and synchronization in motor areas of the basal ganglia-thalamocorticol motor loops. Wichmann and Delong, Neuropsychopharmacology: The Fifth Generation of Progress, Chapter 122, "Neurocircuitry of Parkinson's Disease," 2002.

The basal ganglia serves as a major input to the pyramidal tract motor system. The basal ganglia comprise five paired nuclei including: the caudate nucleus, putamen, pallidum, subthalamic nucleus, and substantia nigra. The subthalamic nucleus is in the diencephalon. The substantia nigra is located in the midbrain. The caudate nucleus, putamen, and pallidum lie within the cerebral hemispheres and are collectively referred to as the corpus striatum. The caudate and putamen are considered collectively as the striatum, which serves as the main site of neural input into the basal ganglia. The striatum receives afferents from all parts of the cerebral cortex and from the nucleus centrum medianum of the thalamus. The major output of the striatum is to the pallidum and the zona reticulata portion of the substania nigra. The dorsal part of the substantia nigra sends efferents to the striatum (the dopaminergic nigrostriatal pathway), and the ventral part of the substantia nigra receives fibers from the striatum.

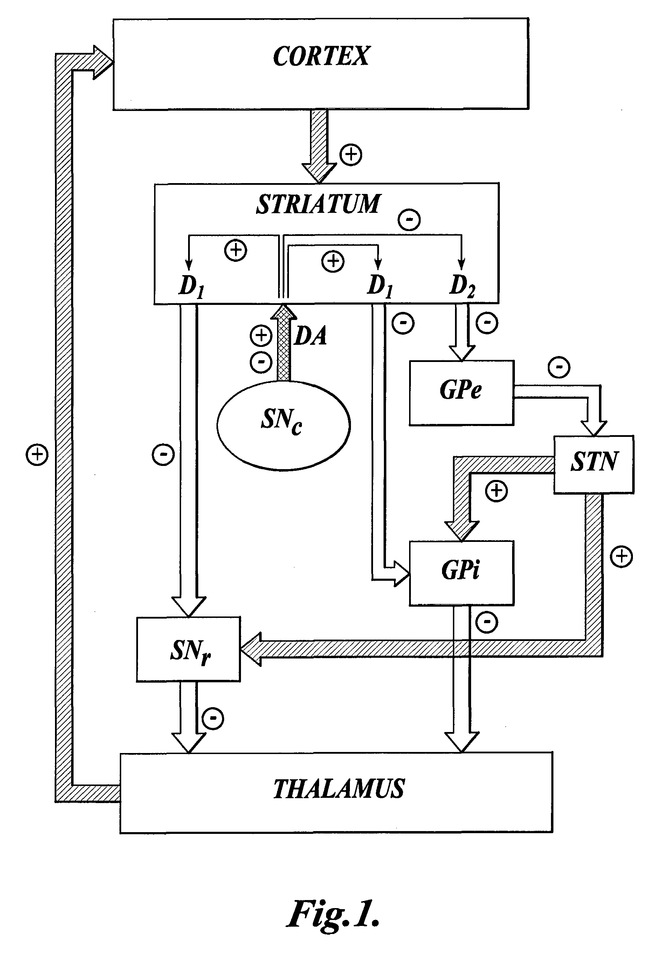

FIGURE 1 illustrates the neurotransmission pathway in the basal ganglia in the mid-brain of a healthy mammalian subject with excitatory pathways labeled "+" with hatched arrows, and with inhibitory pathways labeled "-" with open arrows. As shown in FIGURE 1, neural pathways connect the output pathways of the basal ganglia, a group of functionally related subcortical nuclei that include the external portion of the globulus pallidus ("GPe"), the internal portion of the globulus pallidus ("GPi"), the substantia nigra pars compacta ("SNc"), and the substantia nigra pars reticulata ("SNr") to the striatum. FIGURE 1 also illustrates the pathways connecting the subthalamic nucleus ("STN") to the GPe, the GPi and the SNr. As shown in FIGURE 1, in a healthy subject, Dopamine ("DA") from dopamine producing cells in the SNc sends an excitatory signal to the dopamine D1 receptors ("D1"), which, once activated, send an inhibitory signal to the SNr and an inhibitory signal to the GPi. As further shown in FIGURE 1, DA from dopamine producing cells in the SNc also sends an inhibitory signal to the dopamine D2 receptors ("D2"), which inhibits the D2 receptors from sending an inhibitory signal to the GPe.

The prominent pathologic feature of Parkinson's disease ("PD") is the degeneration of dopaminergic neurons in the substantia nigra pars compacta (SNc) that project to the striatum. Forno, L.S., J. Neuropathol Exp Neurol 55:259-272, 1996. In the early stages of Parkinson's disease, it has been determined that dopamine depletion is greatest in the sensorimotor territory of the striatum, consistent with the early manifestation of motor dysfunction. Kish, S.J., et al., N. Engl. J. Med. 318:876-880, 1988.

In PD and Parkinsonism diseases, dopamine producing cells in the SNc are lost, leading to a deficit in dopaminergic signaling to the striatum. Because DA usually activates the inhibitory striatal output to the SNr via D1 receptors in a healthy subject (as shown in FIGURE 1), this pathway is attenuated in PD. Conversely, because DA inhibits the inhibitory striatal output to the GPe via D2 receptors in a healthy subject (as shown in FIGURE 1), this pathway is augmented in PD. Therefore, a deficit in dopaminergic signaling to the striatum in PD has the net effect of causing net inhibition of the excitatory pathway from the thalamus to the cortex.

Cyclic adenosine monophosphate (cAMP) is a second messenger that mediates the biological response of cells to a wide range of extracellular stimuli. When the appropriate agonist binds to a specific cell surface receptor, adenylyl cyclase is activated to convert adenosine triphosphate (ATP) to cAMP. It is theorized that the agonist induced actions of cAMP within the cell are predominately mediated by the action of cAMP-dependent protein kinases. The intracellular actions of cAMP are terminated by either transporting the nucleotide outside of the cell, or by enzymatic cleavage by cyclic nucleotide phosphodiesterases (PDEs), which hydrolyze the 3'-phosphodiester bond to form 5'-adenosine monophosphate (5'-AMP), which is an inactive metabolite. Therefore, the intracellular enzyme family of PDEs regulates the level of cAMP in cells.

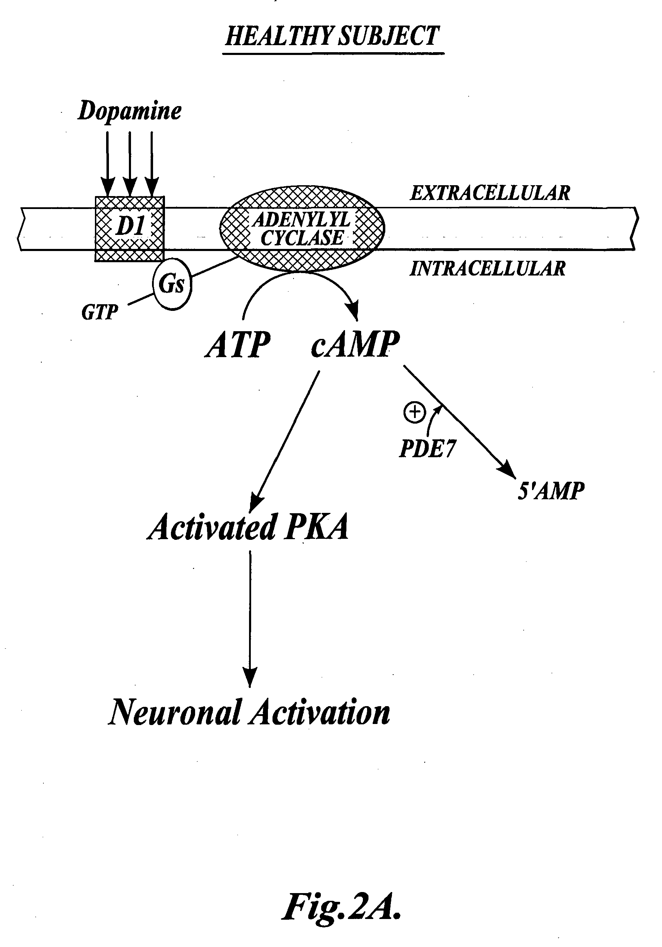

FIGURE 2A illustrates a proposed model of the dopamine receptor activated pathway in a healthy subject. As shown in FIGURE 2A, in healthy subjects Dopamine (DA) (depicted as three arrows) that is produced by the dopaminergic neurons in the substantia nigra pars compacta (SNc), binds to and activates the Dopamine D1 receptor which leads to adenylyl cyclase activation and increased cAMP levels. cAMP activates protein kinase A ("PKA"), which modulates phosphorylation of downstream elements in intracellular signaling pathways, leading to neuronal activation. As shown in FIGURE 2A, it is theorized that the dopamine receptor activated intracellular signaling pathway is downregulated or antagonized by PDE7, which hydrolyzes cAMP to its 5' monophosphate (5'AMP).

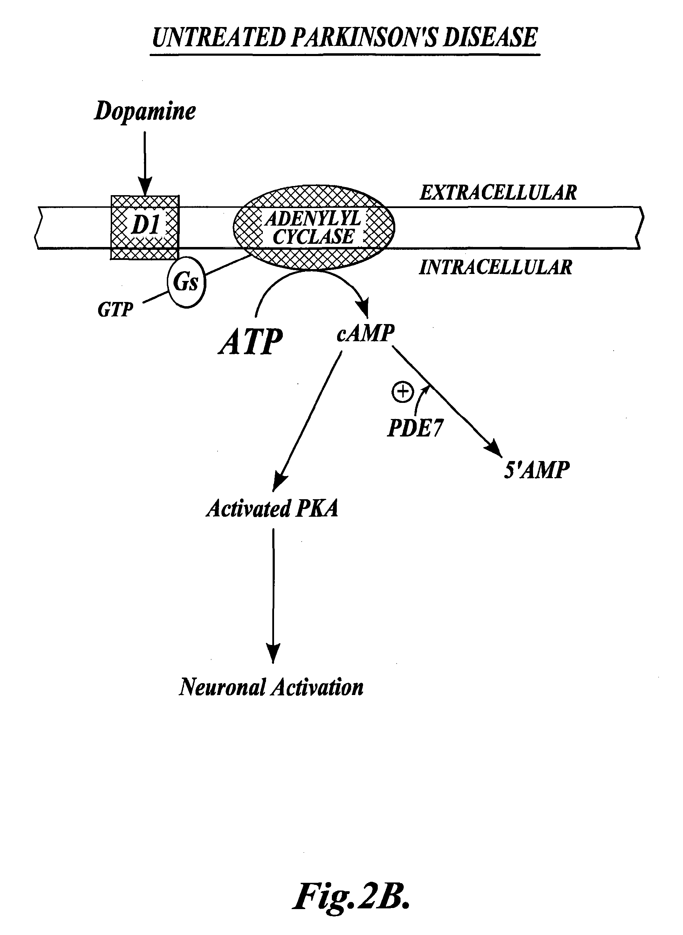

FIGURE 2B illustrates a proposed model of the dopamine receptor activated pathway in an untreated subject with Parkinson's disease (PD). As shown in FIGURE 2B, in the PD subject a reduced amount of dopamine (DA) (depicted as one arrow as compared to three arrows in the healthy subject) is available for binding to the dopamine receptor (D1) because, as described with reference to FIGURE 1, dopamine producing cells in the SNc are lost, leading to a deficit in dopaminergic signaling to the striatum. The reduced level of DA binds to and activates Dopamine D1 receptor to a lesser degree in the PD subject, which leads to minimal adenylyl cyclase activation and an attenuated increase in cAMP levels. As a result, the degree of activation of protein kinase A ("PKA") is less, which in turn leads to less phosphorylation of downstream elements in intracellular signaling pathways, and a lower degree of neuronal activation. As shown in FIGURE 2B, it is theorized that the reduced amount of dopamine receptor activated intracellular signaling pathway is further downregulated or antagonized by PDE7, which hydrolyzes cAMP to its 5' monophosphate (5'AMP), leading to low levels of activated PKA and reduced neuronal activation as compared to a healthy subject.

FIGURE 2C illustrates a proposed model of the dopamine receptor activated pathway in a subject with Parkinson's disease (PD) treated with a PDE7 inhibitory agent. As shown in FIGURE 2C, in the PD subject a reduced amount of dopamine (DA) (depicted as one arrow as compared to three arrows in the healthy subject) is available for binding to the dopamine receptor (D1) because, as described with reference to FIGURE 1, dopamine producing cells in the SNc are lost, leading to a deficit in dopaminergic signaling to the striatum. The reduced level of DA binds to and activates Dopamine D1 receptor to a lesser degree in the PD subject, which leads to minimal adenylyl cyclase activation and an attenuated increase in cAMP levels. However, as further shown in FIGURE 2C, the presence of a PDE7 inhibitory agent that is effective to inhibit PDE7 enzymatic activity blocks the hydrolysis of cAMP, thereby increasing the intracellular cAMP levels, allowing a more normal degree of activation of protein kinase A ("PKA"), which modulates phosphorylation of downstream elements in intracellular signaling pathways, leading to an increase in neuronal activation.

In support of the dopamine signaling model shown in FIGURES 2A-2C, the present inventors have discovered that administration of a PDE7 inhibitory agent that inhibits the enzymatic activity of PDE7 results in improvement of a movement abnormality associated with the pathology of a movement disorder, such as Parkinson's disease. The data presented herein demonstrate that PDE7 inhibitors are effective to restore limb movement, as measured by paw stride length, in an MPTP-treated mouse, and that synergistic effects are observed when PDE7 inhibitors are combined with L-dopa in the MPTP mouse model. Based on the surprising discovery made by the present inventors, it is believed that PDE7 has a role in post-synaptic dopamine signaling in the brain, specifically in areas known to be associated with locomotion.

In addition to Parkinson's disease, abnormal function of the basal ganglia has also been implicated in a variety of neurological disorders with movement abnormalities. Such neurological disorders include restless leg(s) syndrome (Hening, W., et al., Sleep 22:970-999, 1999). Therefore, based on the studies described herein, it is believed that a PDE7 inhibitory agent will have a therapeutic effect on such neurological movement disorders.

Therefore, while not wishing to be bound by theory, it is believed that PDE7 inhibitory agents may be useful to treat neurological disorders characterized by abnormal function of the basal ganglia, such as a deficiency in dopamine receptor signaling, for example, Parkinson's disease, Post-encephalitic Parkinsonism, Drug-induced Parkinsonism, Dopamine-Responsive Dystonia, Shy-Drager Syndrome, Periodic Limb Movement Disorder (PLMD), Periodic Limb Movements in Sleep (PLMS), and Restless Leg(s) Syndrome (RLS) by inhibiting PDE7 activity, and thereby preventing degradation of cAMP in the basal ganglia. It is therefore believed that PDE7 inhibitory agents may be useful to treat these and other neurological movement disorders and neurological disorders characterized by movement abnormalities that are currently treated with L-dopa, other dopamine agonists or precursors or other dopaminergic agents.

In some aspects of this disclosure, PDE7 inhibitors are used to treat a movement abnormality associated with the pathology of a neurological disorder, whether or not such disorder is associated with dopamine signaling defect of deficiency, wherein such inhibition of PDE7 enzymatic activity is the principal therapeutic mode of action of the PDE7 inhibitor in the treatment of the movement abnormality.

In some cases, described herein are methods of treating a movement abnormality associated with the pathology of a neurological movement disorder comprising administering to a patient in need thereof an amount of a PDE7 inhibitory agent effective to inhibit the enzymatic activity of PDE7, wherein such inhibition of PDE7 enzymatic activity is the principal therapeutic mode of action of the PDE7 inhibitor in the treatment of the movement abnormality. In some cases, described herein are provided methods of ameliorating the symptoms of a movement disorder, including but not limited to a dopamine receptor intracellular signaling pathway disorder, comprising administering a PDE7 inhibitory agent that inhibits the enzymatic activity of PDE7. In some cases, the neurological movement disorder is treatable with a dopamine receptor agonist or a precursor of a dopamine receptor agonist.

### Parkinson's Disease

Parkinsonism is a clinical syndrome consisting of four cardinal signs: (1) tremor at rest; (2) rigidity, (3) bradykinesia, and (4) deficiency of postural reflexes. Bradykinesia accounts for the majority of Parkinsonian symptoms and signs. Parkinsonism can be categorized into the following etiologic groups: the primary disorder referred to as Parkinson's disease, secondary, acquired Parkinsonism (due to exposure to drugs or toxins, previous strokes, or encephalitis), and "Parkinsonism-plus" syndrome (impaired ocular movements, orthostatic hypotension, cerebellar ataxia or dementia in a Parkinsonian patient).

Lesions of the substantia nigra with resulting loss of dopamine in the striatum result in the bradykinetic syndrome of Parkinsonism. In Parkinson's disease, there is a loss of pigmented neurons in the substantia nigra and locus ceruleus with subsequent loss of their dopamine and norepinephrine neurotransmitters.

Animal models of PD rely heavily on the fortuitous discovery that systemically administered MPTP (1-methyl-4-phenyl-1,2,3,6-tetrahydropyridine) causes specific neuronal cell death in the substantia nigra of humans, monkeys, and rodents (Jakowec, M.W., et al., Comp. Med. 54(5):497-513, 2004). The pattern of cell death is reminiscent of that seen in PD patients at the time of autopsy. Commonly used animal models for Parkinson's disease include a monkey MPTP model, a rat 6-OHDA model, and a mouse MPTP model. As described in Examples 5-7 herein, the MPTP lesioned mouse model of PD can be used to evaluate the efficacy of PDE7 inhibitory agents useful in the method of the invention to reduce or diminish the alterations induced by MPTP on their stride length, grid step length, and grid foot faults (Tillerson, J.L., et al., Exp. Neurol. 178(1):80-90, 2002).

As demonstrated in Examples 5-7, PDE7 inhibitory agents are effective to restore limb movement in an MPTP-treated mouse. Although current approaches to treating Parkinson's disease generally involve treatment with dopamine receptor agonists, the methods of the present invention are directed to inhibition of PDE7 phosphodiesterase activity in a subject with diminished dopamine signaling in order to increase cAMP levels, thereby leading to increased PKA activity. It is theorized that inhibitors of PDE7 may have advantages over current PD drugs or reduce the required levels of such drugs. For example, chronic use of L-dopa, the most common PD drug, causes severe dyskinesia (Bezard, E., et al., Nat. Rev. Neurosci. 2(8):577-88, 2001). Any PD drug alternative to L-dopa may avoid this serious side effect.

As further demonstrated in Examples 5-7, the combination of PDE7 inhibitory agent(s) and L-dopa, a dopamine receptor agonist, provides a synergistic effect, leading to even greater improvement in limb movement in an MPTP-treated mouse. A drug used in conjunction with L-dopa that allows for the lowering of the dose of L-dopa, such as a PDE7 inhibitory agent, may delay the onset of dyskinesia. Furthermore, because the increased levels of dopamine resulting from L-dopa therapy may increase oxidative damage to substantia nigra pars compacta neurons, an agent such as a PDE7 inhibitor that allows for the lowering of the dose of L-dopa may delay the progression of the disease. Accordingly, the PDE7 inhibitor(s) described herein may be administered in conjunction with L-dopa, other dopamine receptor agonist(s), dopamine receptor agonist precursor(s) or other dopaminergic agent(s), given in a combination dosage form, given concurrently (i.e., at the same time), or given sequentially (e.g., in rotation).

### Restless Leg(s) Syndrome (RLS)

Restless leg(s) syndrome (RLS) is a common neurologic condition that also involves dopamine systems. RLS is a sensory-motor disorder for which the major mandatory criteria for diagnosis are: (1) an urge to move the legs, usually associated by an uncomfortable sensation in the limbs, (2) a worsening of symptoms during rest or inactivity periods, (3) an improvement of symptoms by movement; and (4) an appearance or worsening of symptoms during evening or night. Allen, R.P., et al., Sleep Med 4:101-119, 2003. Supportive criteria, which are common but not essential for RLS diagnosis, include the presence of periodic limb movements in sleep (PLMS), which are involuntary movements of the lower limbs during sleep, often occurring in sequences of at least 4, with an inter-movement interval of 5-90 seconds. (Baier, et al., J. Neurological Sciences 198:71-77, 2002). Other supportive criteria for diagnosis of RLS are responsiveness to low doses of dopaminergic treatments. Allen, R.P., et al., *supra.* RLS and PLMS are highly represented in patients affected by Parkinson's disease and other forms of Parkinsonism. Poewe, W., et al., Neurology 63:S12-S16, 2004.

It has been determined that the pathogenic mechanism of RLS is characterized by a neurological dysfunction of the dopaminergic system. The dopaminergic system has been implicated in RLS by functional imaging studies (Turjanski, N., et al., Neurology 52:932-37, 1999), and by the strong efficacy of dopamine-agonist treatment for human RLS and PLMS (Montplaisier, J., et al., Neurology 52:938-43, 1999; Trenkwalder, C., et al., Neurology 62:1391-97, 2004; and Walters, A.S., et al., Mov. Disord. 19:1414-23, 2004). For example, clinical studies with the following drugs used to treat Parkinson's disease have also shown efficacy for RLS: (1) DA agonists: Sinemet™ (L-dopa, carbidopa), Stalevo™ (L-dopa, carbidopa, entacapone), Permax™ (pergolide), Parlodel™ (bromocryptine); (2) D2,D3,D4 agonists: Mirapex™ (pramipexole), Requip™ (ropinirole); (3) mACh antagonists: Cogentin™ (benztropine), Artane™ (trihexyphenidyl); (4) MAO inhibitors: Eldepryl™ (selegiline), and (5) COMT inhibitor Tasmar™ (tolcapone). See .e.g., Hentz J.G. et al., Mov Disord. 15(2): 324-7 (2000); Walters A.S. et al., Ann Neurol 24(3):455-8 (1988); Trenkwalder C. et al., Neurology 62(8): 1391-7 (2004); Polo O. et al., Clin Neuropharmacol 31(1):61 (2007); Kohnen R. Sleep 22(8):1073-81 (1999); and Shapiro C. Mov Disord 17(2): 398-401 (2002).

The MPTP mouse model described herein is widely known as a model of Parkinson's disease, but it can also represent disorders that are characterized by dopamine insufficiency or those that respond to dopamine receptor agonists (e.g., restless leg(s) syndrome). Therefore, the response observed in the MPTP-treated animals, as demonstrated in Examples 5-7 *supra,* can be reasonably considered to be transferable to Restless Leg(s) Syndrome, and other movement disorders characterized by dopamine insufficiency, such as Dopamine-Responsive Dystonia, Shy-Drager Syndrome, Periodic Limb Movements in Sleep (PLMS), and Tourette's syndrome.

### Periodic Limb Movement Disorder (PLMD)/Periodic Limb Movements in Sleep (PLMS)

Periodic Limb Movement Disorder (PLMD) is a syndrome characterized by sleep disturbance secondary to periodic limb movement during sleep (PLMS). While commonly associated with RLS (Manconi M. et al., Sleep Med. 8(5):491-7 (2007); Haba-Rubio J. et al., Neurophysiol Clin. 33(4):180-4 (2003)), PLMD can also be observed in the setting of spinal cord injury (De Mello M.T. et al., Spinal Cord. 42(4):218-21 (2004)), narcolepsy (Hornyak M. et al., Sleep Med Rev. 10(3):169-77 (2006)), other sleep disorders (Horyak, 2006 *supra,* Saletu M. et al., Hum Psychopharmacol. 16(2):177-187 (2001)), or uremia (Walker S.L., et al., Sleep 19(3):214-8 (1996)).

PLMD can occur in the absence of an identifiable primary pathology (Vetrugno R. et al., Neurol Sci. 28 Suppl 1:S9-S14 (2007), Horyak, 2006 *supra*). In all these settings, an underlying dysfunction in dopamine signaling is indicated by the clinical improvement observed with L-dopa (Wolkove N. et al., CMAJ. 176(10):1449-54 (2007), De Mello M.T. et al. 2004, supra) or dopaminergic agonists (Manconi M. et al., Sleep Med. 8(5):491-7 (2007); Haba-Rubio J. et al., Neurophysiol Clin. 33(4):180-4 (2003), Saletu M. et al., Hum Psychopharmacol. 16(2):177-187 (2001)). Therefore, because PLMD and PLMS are characterized by a dysfunction in dopamine signaling and are treatable with L-dopa, it is believed that the use of PDE7 inhibitory agents may be useful to treat PLMD and/or PLMS when administered to a subject in need thereof either alone, or in conjunction with L-dopa or other dopamine receptor agonist(s), either concurrently or sequentially. The aged rat animal model, described by Baier P.C. et al., J Neurol Sci. 15;198(1-2):71-7 (2002), may be used to assess the efficicacy of PDE7 inhibitory agents for treatment of PLMS.

### Multiple system atrophy including Shy-Drager syndrome

Multiple System Atrophy is a group of progressive neurodegenerative disorders that include Shy-Drager syndrome, olivopontocerebellar atrophy, and striatonigral degeneration. Characteristic symptoms include Parkinson's-like motor abnormalities, orthostatic hypotension, bladder dysfunction, and cerebellar dysfunction (Vanacore N., J Neural Transm. 112(12):1605-12 (2005). A pathological similarity with Parkinson's disease is suggested by the finding of alpha synuclein deposits in autopsy specimens from both diseases (Yoshida M., Neuropathology 27(5):484-93 (2007); Wenning G.K. et al., Acta Neuropathol. 109(2):129-40 (2005); Moore D.J. et al., Annu Rev Neurosci. 28:57-87 (2005). L-dopa is used commonly in therapy to alleviate parkinsonian symptoms with a response rate estimated between 33% and 60% (Gilman S. et al., J Neural Transm. 112(12):1687-94 (2005); Colosimo C. et al., J Neural Transm. 112(12):1695-704 (2005)). Therefore, because some multiple system atrophy disorders (including Shy-Drager syndrome) are treatable with L-dopa, it is believed that the use of PDE7 inhibitory agents may be useful to treat those types of multiple system atrophy disorders, such as Shy-Drager syndrome, that are therapeutically responsive to treatment with dopaminergic agents, when administered to a subject in need thereof either alone, or in conjunction with L-dopa, dopamine receptor agonist(s) or other dopaminergic agents, either concurrently or sequentially. It is known that the MPTP model is a model that is predictive for Multiple System Atrophy, including Shy-Drager syndrome. Stefanova N. et al., Trends Neurosci. 28(9):501-6 (2005). The animal model of multiple system atrophy, as described by Stefanova N. et al., Trends Neurosci. 28(9):501-6 (2005) may be also be used to assess the efficicacy of PDE7 inhibitory agents for treatment of multiple system atrophy disorders, such as Shy-Drager syndrome.

Therefore, based on the studies described herein, it is believed that the use of PDE7 inhibitory agents may be useful to treat multiple system atrophy disorders that are therapeutically responsive to treatment with dopaminergic agent(s), including Shy-Drager syndrome, when administered to a subject in need thereof either alone, or in conjunction with a dopamine receptor agonist(s), either concurrently or sequentially.

### Tourette's Syndrome

Tourette's syndrome is a neurodevelopmental disorder in which the prominent symptoms are stereotyped movements and vocalizations or "tics" (Müller N. Dialogues Clin Neurosci. 9(2):161-71 (2007); Leckman JF, et al J Child Neurol. 21(8):642-9 (2006)). There is anatomical and neuroimaging evidence for the involvement of the dopaminergic system in the basal ganglia in this disease (Müller N. Dialogues Clin Neurosci. 9(2):161-71 (2007)). While anti-psychotics, which block D2 dopamine receptors, are one of the drug classes used to treat disabling tics in Tourette's syndrome, a double-blind crossover clinical study with the dopamine receptor agonist pergolide demonstrated that this drug significantly improved tics (Gilbert DL, et al Neurology. 28;54(6):1310-5 (2000)).

Therefore, because Tourette's syndrome is characterized by a dysfunction in dopamine signaling and are treatable with the dopamine agonist pergolide, it is believed that the use of PDE7 inhibitory agents may be useful to treat Tourette's syndrome when administered to a subject in need thereof either alone, or in conjunction with dopamine receptor agonist(s) or other dopaminergic agent(s), either concurrently or sequentially.

### Huntington's Disease

Huntington's disease is a progressive, genetically determined, and fatal neurological disease that is characterized by jerking movements (chorea) that increase in severity and, in combination with cognitive impairments, eventually lead to complete immobility and loss of function in activities of daily living. The selective loss of medium spiny neurons in the striatum is a prominent pathological feature and is believed to be a primary cause of choreic movements (Standaert DG and Young AB in Goodman and Gilman's Pharmacological Basis of Therapeutics 10th ed McGraw-Hill New York 2001; Chapter 22, pp 562-564). There are no drugs that are useful in slowing the rate of progression of Huntington's and very few that are consistently useful in the amelioration of symptoms. A recent review cited anti-psychotic agents such as haloperidol as "possibly useful" in the treatment of choreic movements. The same review stated that L-dopa and the dopamine agonist pramipexole were "possibly useful" for the treatment of rigidity (Bonelli RM et al. Curr Pharm Des. 12(21):2701-20.(2006)). A few reports suggest that L-dopa or pramipexole may be useful in a specific variant (Westphal variant) of Huntington's in which parkinsonian symptoms are prominent (Bonelli RM et al Clin Neuropharmacol. 25(1):58-60 (2002); Reuter I, J Neurol Neurosurg Psychiatry. 68(2):238-41(2000)). However, controlled trials have not been performed. Therefore, it is possible that a PDE7 inhibitory compound could be useful in Huntington's patients who are responsive to L-dopa, other dopamine agonists or precursors, or other dopaminergic agents.

### Dopamine-responsive dystonia:

Dopamine-responsive dystonia (DRD) is an early onset, progressive, and largely genetically determined neurological disease characterized by diffuse rigidity and other Parkinson's-like symptoms. Segawa M et al., Adv Neurol. 14:215-33 (1976). Depletion of dopamine in the striatum is observed but nerve terminals are intact. A major cause of DRD is an inherited deficiency in the enzyme GTP cyclohydrolase, the rate-limiting enzyme in the synthesis of tetrahydrobiopterin (Segawa disease), which is in turn an essential co-factor for tyrosine hydroxylase. Ichinose H et al., J Biol Chem. 380(12):1355-64 (1999). This deficiency leads to the depletion of dopa and dopamine in nigro-striatal terminals. Treatment with L-dopa/carbidopa combinations (e.g., Sinemet) is highly successful and is the standard of care in this disease. Jeon B, J Korean Med Sci. 12(4):269-79 (1997). Because of the responsiveness of this disease to L-dopa and the intactness of the dopamine signaling pathway in medium spiny neurons, it is believed that PDE7 inhibitory compounds of the present invention may also prove to be effective treatments for DRD.

### III. PDE7 Inhibitory Agents

Cyclic nucleotide phosphodiesterase type 7 (PDE7) is identified as a unique family based on its primary amino acid sequence and distinct enzymatic activity. The PDE genes identified as PDE7 (PDE7A and PDE7B), code for cAMP-specific PDEs. The biochemical and pharmacological characterization of PDE7 shows a high-affinity cAMP-specific PDE (Km=0.2 µM) that is not affected by cGMP nor by selective inhibitors of other PDEs. The PDE7 enzyme selectively decomposes cAMP and is characterized as an enzyme that is not inhibited by rolipram, a selective inhibitor of PDE4, which is a distinct, cAMP-specific PDE family. Two sub-types have been identified within the PDE7 family, PDE7A (Michael, T., et al., J Biol. Chem. 268(17):12925-12932, 1993; Han, P., et al., J Biol. Chem. 272(26):16152-16157, 1997) and PDE7B (U.S. Patent No. 6,146,876; Gardner, C., et al., Biochem. Biophys. Res. Commun. 272(1):186-192, 2000; and Saski, T., et al., Biochem. Biophys. Res. Commun. 271(3):575-583, 2000). The two gene products exhibit 70% identity in their C-terminal catalytic domains (Hetman J.M., et al., PNAS 97(1):472-476 (2000).

PDE7A has three splice variants (PDE7A1, PDE7A2 and PDE7A3); these variants are generated via alternative splicing at both the N- and C-termini (Bloom, T.J., and J.A. Beavo, Proc. Natl. Acad. Sci. USA. 93:14188-14192, 1996). The nucleotide sequence of PDE7A, transcript variant 1, is accessible in public databases by the accession number NM_002603. Human PDE7A1 protein (SEQ ID NO: 2, encoded by SEQ ID NO:1) has 456 amino acids and migrates at an apparent molecular weight of 53-55 kDa on reduced SDS-PAGE.

The nucleotide sequence of PDE7A, transcript variant 2, is accessible in public databases by the accession number NM_002604. Human PDE7A2 protein (SEQ ID NO:4, encoded by SEQ ID NO:3) has 424 amino acids.

The PDE7A protein has a region of about 270 amino acids at the carboxy terminal end that displays significant similarity (~23% homology) to the analogous regions of other cAMP-hydrolyzing PDEs. This region serves as the catalytic domain. The amino-terminal region of this protein is divergent from that of other PDEs and presumably mediates the distinctive and regulatory properties unique to this enzyme family.

The nucleotide sequence of human PDE7B is accessible in public databases by the accession number NM_018945, provided as SEQ ID NO:6, encoded by SEQ ID NO:7. Three splice variants of PDE7B have been reported: PDE7B1, PDE7B2 and PDE7B3. PDE7B is published in WO 01/62904, U.S. Patent No. 6,146,876.

Both PDE7B2 and PDE7B3 possess unique N-terminal sequences. Human PDE7B gene products have an apparent molecular weight of 53-55 kDa on reduced SDS-PAGE (Sasaki, T., Kotera, J., Omori, K., Biochemical J. 361:211-220, 2002). As in PDE7A, the PDE7B has a significantly conserved region of about 270 amino acids common to all PDEs at the carboxy terminal, which serves as the catalytic domain. Similar to the PDE7A protein, the amino-terminal region of PDE7B protein is divergent and presumably accounts for the distinctive and regulatory properties unique to the individual PDE families. The PDE7B protein shows homology to other cAMP-dependent PDEs (23%) within the catalytic domain. The PDE7B polypeptide is 61% homologous to PDE7A, according to WO 2004/044196.

PDE7 is also uniquely localized in mammalian subjects relative to other PDE families. PDE7A expression has been detected in the majority of tissues analyzed, including the brain, heart, kidney, skeletal muscle, spleen and uterus (Bloom, et al., PNAS 93:14188, 1996). Within the brain, PDE7A is widely distributed in both neuronal and non-neuronal cell populations (Miro, et al., Synapse 40:201, 2001). PDE7A's wide expression in the brain, including the basal ganglia and substantia nigra, provides a theoretical basis for a role for PDE7A in motor control as well as other brain functions.

Whereas PDE7A expression is widely distributed in brain tissue, PDE7B brain expression is more restricted and highly enriched in areas linked to motor control, such as the striatum (Reyes-Irisarri, et al, Neuroscience 132:1173, 2005). However, despite the presence of PDE7 in the brain tissue, prior to the data disclosed in the present application, there have been no data linking PDE7 with any specific CNS disease, such as Parkinson's disease. Rather, the use of PDE7 inhibitors has been focused on immunological applications based on work demonstrating that PDE7 inhibition with small interfering RNAs (siRNA) could regulate T-cell proliferation. *See* Rotella, D.P., Drug Discovery 2007, 22-23.

Consistent with the dopamine signaling model shown in FIGURES 2A-2C, the expression pattern of PDE7A and PDE7B overlaps that of the dopaminergic system, supporting the theory that PDE7 is involved in the regulation of motor function. Therefore, while not wishing to be bound by theory, it is believed that treating PD by inhibiting PDE7 functions to boost dopamine signaling, which may be an alternative mechanism for treating PD compared to dopamine receptor agonists. It is also believed that a PDE7 inhibitor may be useful as a therapeutic agent for administration in conjunction (i.e., in combination, concurrently or sequentially) with one or more dopamine receptor agonist(s) or other dopaminergic agent(s).

In the practice of the methods described herein, representative PDE7 inhibitory agents that inhibit the phosphodiesterase activity of PDE7 include: molecules that bind to PDE7 and inhibit the enzyme activity of PDE7 (such as small molecule inhibitors or blocking peptides that bind to PDE7 and reduce enzymatic activity), and molecules that decrease the expression of PDE7 at the transcriptional and/or translational level (such as PDE7 antisense nucleic acid molecules, PDE7 specific RNAi molecules and PDE7 ribozymes), thereby preventing PDE7 from cleaving cAMP. The PDE7 inhibitory agents can be used alone as a primary therapy or in combination with other therapeutics (such as dopamine receptor agonists) as an adjuvant therapy to enhance the therapeutic benefits, as discussed *supra.*

The inhibition of PDE7 is characterized by at least one of the following changes that occur as a result of administration of a PDE7 inhibitory agent in accordance with the methods of the invention: the inhibition of PDE7-dependent enzymatic cleavage of the 3'-phosphodiester bond in cAMP to form 5'-adenosine monophosphate (5'-AMP) (measured, for example, as described in Example 1), a reduction in the gene or protein expression level of PDE7, measured, for example, by gene expression analysis (e.g., RT-PCR analysis) or protein analysis (e.g., Western blot).

In some aspects, a PDE7 inhibitory agent is a molecule or composition that inhibits the expression of PDE7A, PDE7B, or both PDE7A and PDE7B, such as an antisense or small inhibitory nucleotide (e.g., siRNA) that specifically hybridizes with the cellular mRNA and/or genomic DNA corresponding to the gene(s) of the target PDE7 so as to inhibit their transcription and/or translation, or a ribozyme that specifically cleaves the mRNA of a target PDE7.

### Potency of PDE7Inhibitory Agents

In one case, a PDE7 inhibitory agent useful in the methods of the invention is a compound that is sufficiently potent to inhibit the enzymatic activity of PDE7 (PDE7A, PDE7B, or PDE7A and PDE7B) at an IC₅₀ ≤ 1 µM, preferably less than or about 0.1 µM. In one case, the PDE7 inhibitory agent is sufficiently potent to inhibit the enzymatic activity of PDE7 (PDE7A, PDE7B, or PDE7A and PDE7B) at an IC₅₀ of from about 0.1 to about 500 nM. In one case, the PDE7 inhibitory agent is potent to inhibit the enzymatic activity of PDE7 (PDE7A, PDE7B, or PDE7A and PDE7B) at an IC₅₀ of from about 1 to about 100 nM.

Representative methods for determining the IC₅₀ for a PDE7 (PDE7A or PDE7B) inhibitory agent are provided in Example 1 herein, and are well known in the art, such as the Scintillation Proximity Assay (SPA) disclosed in Bardelle et al., Anal Biochem 15:275(2):148-55 (1999).

### PDE7A or PDE7B Selective Inhibitory Agents

In one case, the PDE7 inhibitor useful in the method described herein is a PDE7A inhibitory agent. In one case, the PDE7A inhibitory agent is potent to inhibit the enzymatic activity of PDE7A at an IC₅₀ of from about 0.1 to about 500 nM. In one case, the PDE7A inhibitor has an IC₅₀ of from about 1 to about 100 nM. A suitable assay for determining the IC₅₀ for a PDE7A inhibitor uses recombinant human PDE7A2 enzymes expressed in a baculoviral system. This assay method is a modification of the SPA assay reported by Bardelle et al. supra. An exemplary assay for measuring PDE7A inhibition is provided in Example 1.

In some cases, the PDE7 inhibitory agent exhibits isozyme-selective activity against PDE7A. A PDE7A selective inhibitory agent reduces PDE7A activity at least two-fold more than PDE7B activity, more preferably at least 10-fold, at least 20-fold, at least 50-fold, or at least 100-fold. In some cases, the PDE7A inhibitory agent is an inhibitory agent that is at least 10-fold (such as at least 20-fold, or at least 50-fold or at least 100-fold) more selective for inhibiting PDE 7A activity than for the enzyme activity of any other PDE (PDE1-6, 7B, and 8-11).

In another case, the PDE7 inhibitor useful in the method described herein is a PDE7B inhibitor. Due to the potential for reduced side effects due to the restricted expression of PDE7B, and high levels of expression in areas of the brain linked to motor control (e.g., the striatum), inhibitors for PDE7B may be useful for treatment of neurological movement disorders such as Parkinson's disease.

In one case, the PDE7B inhibitor has an IC₅₀ of from about 0.1 to about 500 nM. In one case, the PDE7B inhibitory agent is sufficiently potent to inhibit the enzymatic activity of PDE7B at an IC₅₀ of from about 0.1 to about 500 nM. In one case, the PDE7B inhibitor has an IC₅₀ of from about 1 to about 100 nM. Methods for determining the IC₅₀ for a PDE7B inhibitor are well known in the art, such as the assays disclosed in Bardelle et al., supra. An exemplary assay for measuring PDE7AB inihibition is provided in Example 1.

In some cases, the PDE7 inhibitor exhibits isozyme-selective activity against PDE7B. A PDE7B selective inhibitory agent reduces PDE7B activity at least two-fold more than PDE7A activity, more preferably at least 10-fold, at least 20-fold, at least 50-fold, or at least 100-fold. In some cases, the PDE7B inhibitory agent is an inhibitory agent that is at least 10-fold (such as at least 20-fold, or at least 50-fold or at least 100-fold) more selective for inhibiting PDE7B activity than for the enzyme activity of any other PDE (PDE1-6, 7A, and 8-11).

### PDE7 Selectivity as Compared to Other PDEs

In some cases, the PDE7 inhibitory agent has an IC₅₀ for inhibiting PDE1B activity of greater than 5 times (such as at least 10-fold, at least 20-fold, or at least 50-fold or at least 100-fold) the lesser of the IC₅₀ for inhibiting PDE7A activity and the IC₅₀ for inhibiting PDE7B activity. Stated differently, the PDE7 inhibitor is more potent (by 5 times, 10 times, 20 times, 50 times or 100 times) at inhibiting the activity of PDE7A or PDE7B (whichever PDE7A or PDE7B isozyme upon which the PDE7 inhibitor has the most effect), than it is at inhibiting the activity of PDE1B. For purposes of the present specification, by way of example, this property may be still more simply stated as the PDE7 inhibitor is more potent (by 5 times, 10 times, 20 times, 50 times or 100 times) at inhibiting the activity of PDE7 than it is at inhibiting the activity of PDE1B.

Dual inhibition of both PDE7 and PDE1B may confer additional benefit in the treatment of movement disorders based on a report that deletion of the gene for PDE1B in mice stimulated the metabolism of dopamine and sensitized the animals to the effects of dopaminergic agonists (Siuciak, et al., Neuropharmacology 53(1): 113-23 (2007)).

In some cases, the PDE7 inhibitory agent has an IC₅₀ for inhibiting PDE10 activity of greater than 5 times (such as at least 10-fold, or at least 20-fold, or at least 50-fold or at least 100-fold) the lesser of the IC₅₀ for inhibiting PDE7A activity and the IC₅₀ for inhibiting PDE7B activity. Dual inhibition of both PDE7 and PDE10 may confer additional benefit in the treatment of movement disorders based on a report that selective inhibitors of PDE10 cause an increase in cAMP levels in the striatum (Siuciak J.A. et al., Neuropharmacology 51(2):386-96 (2006)).

In some cases, the PDE7 inhibitory agent has an IC₅₀ for inhibiting PDE3 activity of greater than 10 times (such as at least 20-fold, at least 50-fold or at least 100-fold) the lesser of the IC₅₀ for inhibiting PDE7A activity and the IC₅₀ for inhibiting PDE7B activity. This is because the administration of selective inhibitors of PDE3 to patients in heart failure was shown to increase their rate of mortality (Packer M. et al., N Engl J Med. 325(21):1468-75 (1991)).

In some cases, the PDE7 inhibitory agent has an IC₅₀ for inhibiting PDE4 activity of greater than 10 times (such as at least 20-fold, at least 50-fold or at least 100-fold) the lesser of the IC₅₀ for inhibiting PDE7A activity and the IC50 for inhibiting PDE7B activity. This is because deletion of one of the PDE4 genes in mice has been shown to lead to cardiac myopathy (Lehnart S.E. et al., Cell 123(1):25-35 (2005)).

In some cases, the PDE7 inhibitory agent has an IC₅₀ for inhibiting PDE3 activity and PDE4 activity of greater than 10 times (such as at least 20-fold, at least 50-fold or at least 100-fold) the lesser of the IC₅₀ for inhibiting PDE7A activity and the IC50 for inhibiting PDE7B activity.

In some cases, the PDE7 inhibitory agent has an IC₅₀ for inhibiting PDE8 activity of greater than 10 times (such as at least 20-fold, at least 50-fold or at least 100-fold) the lesser of the IC₅₀ for inhibiting PDE7A activity and the IC₅₀ for inhibiting PDE7B activity.

In this invention, the PDE7 inhibitory agent has an IC₅₀ for inhibiting PDE4 activity and PDE8 activity of greater than 10 times (such as at least 20-fold, at least 50-fold or at least 100-fold) the lesser of the IC₅₀ for inhibiting PDE7A activity and the IC₅₀ for inhibiting PDE7B activity. In accordance with this case, it is known that the PDE families that specifically/preferentially hydrolyze cAMP include PDE4, PDE7, and PDE8.

In some cases, the PDE7 inhibitory agent has an IC₅₀ for inhibiting the activity of PDE 1, PDE2, PDE3, PDE4, and PDE8, PDE10, and PDE11 of greater than 10 times the lesser of the IC₅₀ for inhibiting PDE7A activity and the IC50 for inhibiting PDE7B activity. In accordance with this case, it is known that the PDE families that specifically/preferentially hydrolyze cAMP include PDE4, PDE7, and PDE8 and the PDE 1, PDE2, PDE3, PDE10, and PDE11 families show substantial activity against both cAMP and cGMP.

In some cases, the PDE inhibitory agent is a selective PDE7 inhibitor for which the lesser of the IC₅₀ for inhibiting PDE7A activity and the IC50 for inhibiting PDE7B activity is less than one-tenth (such as one-twentieth, one-fiftieth, or one-hundredth) the IC₅₀ that the agent has for inhibiting any other PDE enzyme from the PDE1-6 and PDE8-11 enzyme families.

A selective PDE7 inhibitor can be identified, for example, by comparing the ability of an agent to inhibit PDE7 (PDE7A, PDE7B or PDE7A and PDE7B) enzyme activity to its ability to inhibit PDE enzymes from the other PDE families. For example, an agent may be assayed for its ability to inhibit PDE7 activity as well as PDE1, PDE2, PDE3, PDE4, PDE5, PDE6, PDE8, PDE9, PDE10, and PDE11. Exemplary methods for comparing the ability of an agent to inhibit PDE7 enzyme activity to its ability to inhibit PDE enzymes from the other PDE families are provided in Example 2 herein. The ratio of the IC₅₀ inhibition for each of the PDE(1-6 and 8-11) isozymes to the IC₅₀ inhibition of PDE7 (i.e., the more sensitive of PDE7A or PDE7B) may be determined by a standard *in vitro, in vivo, or ex vivo* assay, such as those described herein.

### PDE7 Selectivity as Compared to Other Non-PDE Molecular Targets Known to be Involved with a Neurological Movement Disorder

In some cases, the PDE7 inhibitory agent is selective for PDE7 and substantially inactive against non-PDE molecular targets known or believed to be involved with the pathology of a neurological movement disorder. In some cases, the PDE7 inhibitory agent is a PDE7 inhibitory agent for which the lesser of the IC₅₀ for inhibiting PDE7A activity and the IC50 for inhibiting PDE7B activity is less than one-half (such as less than one-fifth, less than one-tenth, such as less than one-twentieth, less than one-fiftieth, or less than one-hundredth) of the IC₅₀ that the agent has for inhibiting activity at other molecular targets (i) known to be involved with the pathology of a neurological movement disorder selected from the group consisting of Parkinson's disease, Post-Encephalitic Parkinsonism, Dopamine-Responsive Dystonia, Shy-Drager Syndrome, Period Limb Movement Disorder (PLMD), Periodic Limb Movements in Sleep (PLMS), and Restless Leg(s) Syndrome (RLS), or (ii) at which other drug(s) that are therapeutically effective to treat the disorder act.

In other cases, the PDE7 inhibitory agent is selective for PDE7 and substantially inactive against non-PDE molecular targets known to be involved with the pathology of Parkinson's disease. In some cases, the PDE7 inhibitory agent is a PDE7 inhibitory agent for which the lesser of the IC₅₀ for inhibiting PDE7A activity and the IC50 for inhibiting PDE7B activity is less than one-half (such as less than one-fifth, less than one-tenth, less than one-twentieth, less than one-fiftieth, or less than one-hundredth) of the IC₅₀ that the agent has for inhibiting activity at other molecular targets (i) known to be involved with the pathology of Parkinson's disease, such as catechol-O-methyltransferase (COMT), monamine oxidase B (MAO-B), dopamine transporters (DAT), tyrosine hydroxylase, dopamine receptors, adenosine A_{2A} receptors, muscarinic acetylcholine receptors, N-methyl D-aspartate (NMDA) receptors, gamma amino butyric acid (GABA) receptors and gabapentin receptors, or (ii) at which other drug(s) that are therapeutically effective to treat Parkinson's disease act. Exemplary methods for comparing the ability of an agent to inhibit PDE7 enzyme activity to its ability to inhibit other molecular targets known to be involved with the pathology of Parkinson's disease are provided in Example 4 herein.

In other cases, the PDE7 inhibitory agent is selective for PDE7 and substantially inactive against non-PDE molecular targets known to be associated with the dopamine signaling pathway. In some cases, the PDE7 inhibitory agent is a PDE7 inhibitory agent for which the lesser of the IC₅₀ for inhibiting PDE7A activity and the IC50 for inhibiting PDE7B activity is less than one-half (such as less than one-fifth, less than one-tenth, such as less than one-twentieth, less than one-fiftieth, or less than one-hundredth) of the IC₅₀ that the agent has for inhibiting activity at other molecular targets known to be associated with the dopamine signaling pathway, such as catechol-O-methyltransferase (COMT), monamine oxidase B (MAO-B), dopamine transporters (DAT), tyrosine hydroxylase, dopa decarboxylase, dopamine receptors, adenylyl cyclase, protein kinase A (PKA), dopamine and cyclic AMP- regulated phosphoprotein of molecular weight 32,000 (DARPP32), and protein phosphatase-1. Exemplary methods for comparing the ability of an agent to inhibit PDE7 enzyme activity to its ability to inhibit other molecular targets known to be associated with the dopamine signaling pathway are provided in Example 4 herein.

### Types of PDE7 Inhibitory Agents

The PDE7 inhibitory agent can be any type of agent including, but not limited to, a chemical compound, a protein or polypeptide, a peptidomimetic, a nucleic acid molecule, or ribozyme. In some cases, PDE7 inhibitory agents are small molecule inhibitors including natural and synthetic substances that have a low molecular weight (i.e., less than about 450 g/mole), such as, for example, peptides, peptidomimetics and nonpeptide inhibitors such as chemical compounds.

### Chemical Compounds:

The PDE7 inhibitors useful in the methods described herein include agents that are administered by a conventional route (e.g., oral, intramuscular, subcutaneous, transdermal, transbucal, intravenous, etc.) into the bloodstream and are ultimately transported through the vascular system across the blood brain barrier to inhibit PDE7 in the brain. Accordingly, for these methods of administration, the PDE7 inhibitors have the ability to cross the blood brain barrier. Those PDE inhibitors described below that have the ability to cross the blood brain barrier (e.g., those having a molecular weight less than about 450 g/mole and that are sufficiently lipophilic) are useful in the methods of the invention when the inhibitors are administered by a route that ultimately transports the inhibitors to the brain in the bloodstream.

The following is a description of exemplary PDE7 inhibitors useful in the methods described herein.

In one case, PDE7 inhibitors useful in the methods of the invention are selected from those compounds generally or specifically disclosed in EP 1 454 897, WO 2003/053975, and US 20050148604. In one case, PDE7 inhibitors useful in the methods described herein have the formulas:

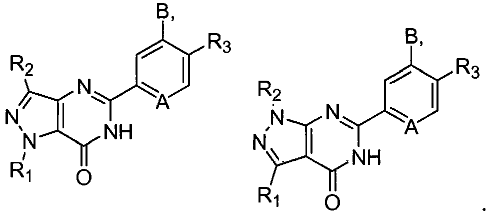

The substituents for the above compounds are defined as follows:
A represents N or CR₄,
B represents a hydrogen atom or a halogen atom,
R₁ represents optionally substituted C₃₋₇ cycloalkyl or tert-butyl,
R₂ represents hydrogen, methyl, or ethyl,
R₃ represents a hydrogen, nitro, cyano or halogen atom, NR₅R₆, C(=X)R₇, SO₂NR₅R₆, OR₈, NR₈CONR₅R₆, NR₈SO₂R₉, NR₈CO₂R₉, a heteroaryl group, optionally substituted C₁₋₃ alkyl, optionally substituted C₁₋₆ alkenyl, or optionally substituted saturated or unsaturated heterocycloalkyl,
R₄ represents hydrogen, or C₁₋₃ alkoxy substituted, if desired, by one or more fluorine atoms,
R₅ and R₆ are the same or different, and represent a hydrogen atom, optionally substituted C₁₋₆ alkyl, optionally substituted heterocycloalkyl, or optionally substituted acyl or, together with the nitrogen atom which they are bound to, form azetidinyl, pyrrolidinyl, piperidinyl, morpholino, thiomorpholino, piperazinyl, or homopiperazinyl, each of these groups being optionally substituted by optionally substituted C₁₋₄ alkyl, OH, C₁₋₃ alkoxy, CO₂H, NR₅R₆, an oxo group, NR₉COR₇, or C(=O)R₇,
R₇ represents optionally substituted C₁₋₆ alkyl, OH, OR₈, or NR₅R₆,
R₈ represents hydrogen, an optionally substituted C₁₋₆ alkyl group, or optionally substituted heterocycloalkyl,
R₉ represents an optionally substituted C₁₋₆ alkyl group, and
X represents O, S, or NH.

In regard to the above compounds, "optionally substituted" refers to optionally substituted linear, branched or cyclic alkyl group such as methyl, ethyl, propyl or cyclohexyl; a hydroxyl group; a cyano group; an alkoxy group such as methoxy or ethoxy; an optionally substituted amino group such as amino, methylamino or dimethylamino; an optionally substituted acyl group such as acetyl or propionyl; a carboxyl group; an optionally substituted aryl group such as phenyl or naphthyl; an optionally substituted heteroaryl group such as pyridinyl, thiazolyl, imidazolyl or pyrazyl; an optionally substituted saturated or unsaturated heterocycloalkyl group such as piperazinyl or morphonyl; an optionally substituted carbamoyl group; an optionally substituted amido group; a halogen atom such as chlorine, fluorine or bromine; a nitro group; an optionally substituted sulfone group; an optionally substituted sulfonylamido group; an oxo group; a urea group; and an optionally substituted linear, branched or cyclic alkenyl group such as ethenyl, propenyl or cyclohexenyl.

Examples of the heteroaryl group as R³ include a 5- to 7-membered monocyclic heteroaryl group having 2 to 8 carbon atoms and containing 1 to 4 hetero atoms consisting of oxygen atoms, nitrogen atoms or sulfur atoms, and a polycyclic heteroaryl group comprising two or more such identical or different monocyclic compounds fused together, examples of the monocyclic and polycyclic heteroaryl groups being pyrrole, furyl, thienyl, imidazolyl, thiazolyl, pyridyl, pyrazyl, indolyl, quinolyl, isoquinolyl, and tetrazolyl.

In one case, a PDE7 inhibitor useful in the invention has the formula:

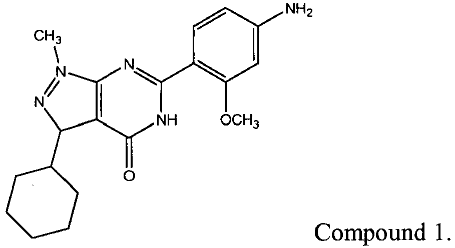

The activity of Compound 1 in inhibiting select PDEs is described in Examples 1 and 2. The effectiveness of Compound 1 in the MPTP Parkinson's model is described in Examples 5 and 6.

In others cases, PDE7 inhibitors useful in the methods described herein have the formulas:

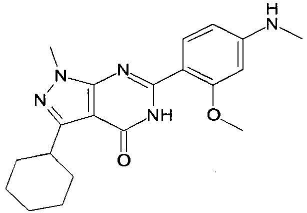

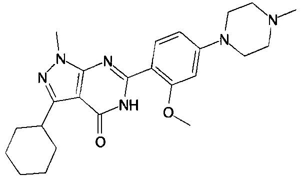

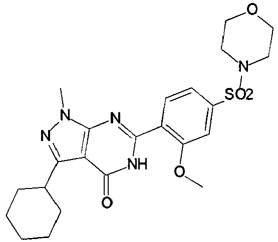

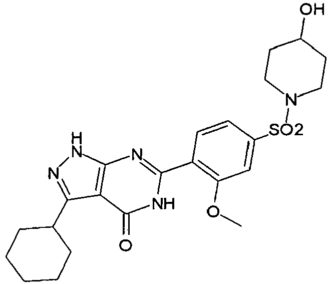

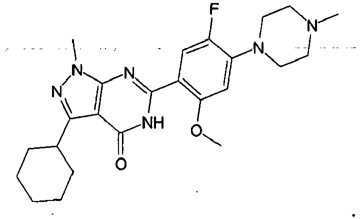

In another case, a PDE7 inhibitor useful in the methods described herein has the formula:

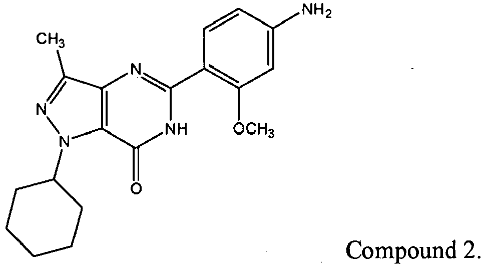

The activity of Compound 2 in inhibiting select PDEs is described in Examples 1 and 2. The effectiveness of Compound 2 in the MPTP Parkinson's model is described in Example 7.

In other cases, PDE7 inhibitors useful in the methods described herein have the formulas:

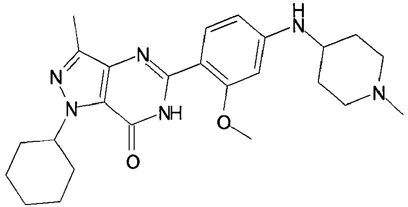

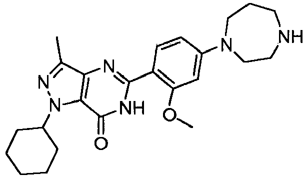

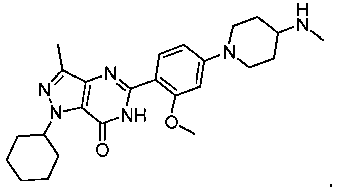

The preparation of the above compounds is described in EP 1 454 897, WO 2003/053975, and US 20050148604.

In another case, PDE7 inhibitors useful in the methods described herein are selected from those compounds generally or specifically disclosed in US 2002/0198198, WO 2002/076953, WO 2002/074754, WO 2006/092691, Bioorganic & Medicinal Chemistry Letters 14 (2004) 4623-4626, and Bioorganic & Medicinal Chemistry Letters 14 (2004) 4627-4631. In one case, PDE7 inhibitors useful in the methods described herein have the formulas:

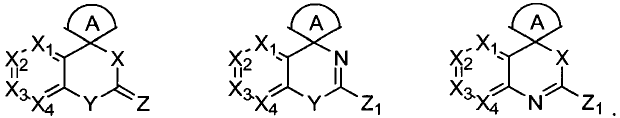

The substituents for the above compounds are defined as follows:
(a) X₁, X₂, X₃, and X₄ are the same or different and are selected from:
   N, provided that not more than two of the groups X₁, X₂, X₃, and X₄ simultaneously represent a nitrogen atom, or,
   C-R₁, in which R₁ is selected from:
      Q₁, or
      lower alkyl, lower alkenyl, or lower alkynyl, these groups being unsubstituted or substituted with one or several groups Q₂;
      the group X₅-R₅ in which,
         X₅ is selected from:
            a single bond,
            lower alkylene, lower alkenylene, or lower alkynylene; optionally interrupted with 1 or 2 heteroatoms chosen from O, S, S(=O), SO₂, or N, the carbon atoms of these groups being unsubstituted or substituted with one or several groups, identical or different, selected from SR₆, OR₆, NR₆R₇, =O, =S, or =NR₆ in which R₆ and R₇ are the same or different and are selected from hydrogen or lower alkyl, and,
            R₅ is selected from aryl, heteroaryl, cycloalkyl optionally interrupted with C(=O) or with 1, 2, or 3 heteroatoms chosen from O, S, S(=O), SO₂, or N, cycloalkenyl optionally interrupted with C(=O) or with 1, 2, or 3 heteroatoms chosen from O, S, S(=O), SO₂ or N, or a bicyclic group, these groups being unsubstituted or substituted with one or several groups selected from Q₃, heteroaryl, or lower alkyl optionally substituted with Q₃;
         in which Q₁, Q₂, and Q₃ are the same or different and are selected from:
            hydrogen, halogen, CN, NO₂, SO₃H, P(=O)(OH)₂, OR₂, OC(=O)R₂, C(=O)OR₂, SR₂, S(=O)R₂, NR₃R₄, Q-R₂, Q-NR₃R₄, NR₂-Q-NR₃R₄, or NR₃-Q-R₂ in which Q is selected from C(=NR), C(=O), C(=S), or SO₂, R is selected from hydrogen, or lower alkyl, and R₂, R₃, and R₄ are the same or different and are selected from:
            hydrogen, lower alkyl optionally interrupted with C(=O), (CH₂)ₙ-aryl, (CH₂)ₙ-heteroaryl, (CH₂)ₙ-cycloalkyl optionally interrupted with C(=O) or with 1 or 2 heteroatoms chosen from O, S, S(=O), SO₂, or N, in which n is an integer selected from 0, 1, 2, 3 or 4;
            these groups being unsubstituted or substituted with one or several groups selected from lower alkyl, halogen, CN, CH₃, SO₃H, SO₂CH₃, CF₃, C(=O)NHSO₂CH₃, OR₆, COOR₆, C(=O)R₆, NR₆R₇, C(=O)NR₆R₇, or SO₂NR₆R₇, in which R₆ and R₇ are the same or different and are selected from hydrogen or lower alkyl optionally substituted with one or two groups selected from OR, COOR or NRR₈ in which R and R₈ are hydrogen or lower alkyl, and,
            R₆ and R₇, and/or, R₃ and R₄, together with the nitrogen atom to which they are linked, can form a 4- to 8-membered heterocyclic ring, which may contain one or two heteroatoms selected from O, S, S(=O), SO₂, or N, and which may be substituted with,
            a 4- to 8-membered heterocyclic ring, which may contain one or two heteroatoms selected from O, S, or N, and which may be substituted with a lower alkyl, or,
            a lower alkyl optionally substituted with OR', NR'R", C(=O)NR'R" or COOR' in which R' and R" are the same or different and are selected from H, lower alkyl optionally substituted with OR or COOR in which R is hydrogen or lower alkyl, and R' and R" together with the nitrogen atom to which they are linked, can form a 4- to 8-membered heterocyclic ring, which may contain one or two heteroatoms selected from O, S, or N; or,
(b) X is O, S, or NR₉, in which R₉ is selected from hydrogen, CN, OH, NH₂, lower alkyl, lower alkenyl, or lower alkynyl, these groups being unsubstituted or substituted with cycloalkyl optionally interrupted with 1 or 2 heteroatoms chosen from O, S, S(=O), SO₂, or N, cycloalkenyl optionally interrupted with 1 or 2 heteroatoms chosen from O, S, S(=O), SO₂, or N, aryl, heteroaryl, OR₁₀, or NR₁₀R₁₁ in which R₁₀ and R₁₁ are the same or different and are selected from hydrogen or lower alkyl;
(c) Y is selected from O, S, or N-R₁₂, in which R₁₂ is selected from hydrogen, CN, OH, NH₂, lower alkyl, lower alkenyl, or lower alkynyl, these groups being unsubstituted or substituted with cycloalkyl optionally interrupted with 1 or 2 heteroatoms chosen from O, S, S(=O), SO₂, or N, cycloalkenyl optionally interrupted with 1 or 2 heteroatoms chosen from O, S, S(=O), SO₂, or N, aryl, heteroaryl, OR₁₀, or NR₁₀R₁₁ in which R₁₀ and R₁₁ are the same or different and are selected from hydrogen or lower alkyl;
(d) Z is chosen from CH-NO₂, O, S, or NR₁₃ in which R₁₃ is selected from hydrogen, CN, OH, NH₂, aryl, heteroaryl, cycloalkyl optionally interrupted with one or several heteroatoms chosen from O, S, S(=O), SO₂, or N, cycloalkenyl optionally interrupted with one or several heteroatoms chosen from O, S, S(=O), SO₂, or N, C(=O)R₁₄, C(=O)NR₁₄R₁₅, OR₁₄, or, lower alkyl, unsubstituted or substituted with one or several groups which are the same or different and which are selected OR₁₄ or NR₁₄R₁₅;
   R₁₄ and R₁₅ being independently selected from hydrogen or lower alkyl, or, R₁₄ and R₁₅, together with the nitrogen atom to which they are linked, can form a 4- to 8-membered heterocyclic ring which may contain one or two heteroatoms chosen from O, S, or N, and which may be substituted with a lower alkyl;
(e) Z₁ is chosen from H, CH₃, or NR₁₆R₁₇ in which R₁₆ and R₁₇ are the same or different and are selected from hydrogen, CN, aryl, heteroaryl, cycloalkyl optionally interrupted with one or several heteroatoms chosen from O, S, S(=O), SO₂, or N, cycloalkenyl optionally interrupted with one or several heteroatoms chosen from O, S, S(=O), SO₂, or N, C(=O)R₁₄, C(=O)NR₁₄R₁₅, OR₁₄, or, lower alkyl unsubstituted or substituted with one or several groups selected from OR₁₄ or NR₁₄R₁₅,
   R₁₄ and R₁₅ being chosen from hydrogen or lower alkyl, and, R₁₄ and R₁₅, and/or, R₁₆ and R₁₇, together with the nitrogen atom to which they are linked, can form a 4- to 8-membered heterocyclic ring which may contain one or two heteroatoms chosen from O, S, or N, and which may be substituted with a lower alkyl;
(f) A is a cycle selected from: 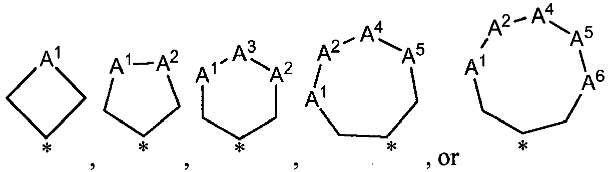 in which
   A₁, A₂, A₃, A₄, A₅, and A₆ are the same or different and are selected from O, S, C, C(=O), SO, SO₂, or NR₁₈ in which R₁₈ is selected from hydrogen, aryl, heteroaryl, cycloalkyl optionally interrupted with one or several heteroatoms chosen from O, S, S(=O), SO₂, or N, cycloalkenyl optionally interrupted with one or several heteroatoms chosen from O, S, S(=O), SO₂, or N, lower alkyl unsubstituted or substituted with aryl, heteroaryl, cycloalkyl optionally interrupted with one or several heteroatoms chosen from O, S, S(=O), SO₂, or N, cycloalkenyl optionally interrupted with one or several heteroatoms chosen from O, S, S(=O), SO₂, or N, CN, NR₁₉R₂₀, C(=O)NR₁₉R₂₀, OR₁₉, C(=O)R₁₉ or C(=O)OR₁₉ in which R₁₉ and R₂₀ are identical or different and are selected from hydrogen or lower alkyl;
   * represents the carbon atom which is shared between the cycle A and the backbone cycle containing X and/or Y;
   each carbon atom of the cycle A is unsubstituted or substituted with 1 or 2 groups, identical or different, selected from lower alkyl optionally substituted with OR₂₁, NR₂₁R₂₂, COOR₂₁, or CONR₂₁R₂₂, lower haloalkyl, CN, F, =O, SO₂NR₁₉R₂₀, OR₁₉, SR₁₉, C(=O)OR₁₉, C(=O)NR₁₉R₂₀, or NR₁₉R₂₀ in which R₁₉ and R₂₀ are identical or different and are selected from hydrogen or lower alkyl optionally substituted with OR₂₁, NR₂₁R₂₂, COOR₂₁, or CONR₂₁R₂₂, in which R₂₁ and R₂₂ are identical or different and are selected from hydrogen or lower alkyl, and, R₁₉ and R₂₀, and/or, R₂₁ and R₂₂, together with the nitrogen atom to which they are linked, can form a 4- to 8-membered heterocyclic ring;
   two atoms of the cycle A, which are not adjacent, may be linked by a 2, 3 or 4 carbon atom chain which may be interrupted with 1 heteroatom chosen from O, S or N; provided that not more than two of the groups A₁, A₂, A₃, A₄, A₅, and A₆ simultaneously represent a heteroatom; and
   their tautomeric forms, their racemic forms, their isomers, and their pharmaceutically acceptable derivatives.

In regard to the above compounds, halogen includes fluoro, chloro, bromo, and iodo. Preferred halogens are F and Cl. Lower alkyl includes straight and branched carbon chains having from 1 to 6 carbon atoms. Examples of such alkyl groups include methyl, ethyl, isopropyl, and tert-butyl. Lower alkenyl includes straight and branched hydrocarbon radicals having from 2 to 6 carbon atoms and at least one double bond. Examples of such alkenyl groups are ethenyl, 3-buten-1-yl, 2-ethenylbutyl, and 3-hexen-1-yl. Lower alkynyl includes straight and branched hydrocarbon radicals having from 2 to 6 carbon atoms and at least one triple bond. Examples of such alkynyl groups are ethynyl, 3-butyn-1-yl, propynyl, 2-butyn-1-yl, and 3-pentyn-1-yl. Lower haloalkyl includes a lower alkyl as defined above, substituted with one or several halogens. An example of haloalkyl is trifluoromethyl. Aryl is understood to refer to an aromatic carbocycle containing between 6 and 10 carbon atoms. An example of an aryl group is phenyl. Heteroaryl includes aromatic cycles which have from 5 to 10 ring atoms, from 1 to 4 of which are independently selected from the group consisting of O, S, and N. Representative heteroaryl groups have 1, 2, 3 or 4 heteroatoms in a 5- or 6-membered aromatic ring. Examples of such groups are tetrazole, pyridyl, and thienyl. Representative cycloalkyl contain from 3 to 8 carbon atoms. Examples of such groups are cyclopropyl, cyclobutyl, cyclopentyl, cyclohexyl, cycloheptyl and cyclooctyl. The term "interrupted" means that in a backbone chain, a carbon atom is replaced by an heteroatom or a group as defined herein. For example, in "cycloalkyl or cycloalkenyl optionally interrupted with C(==O) or with 1 heteroatom chosen from O, S, S(==O), SO₂ or N", the term "interrupted" means that C(==O) or a heteroatom can replace a carbon atom of the ring. Example of such groups are morpholine or piperazine. Cycloalkenyl includes 3- to 10- membered cycloalkyl containing at least one double bond. Heterocyclic rings include heteroaryl as defined above and cycloalkyl or cycloalkenyl, as defined above, interrupted with 1, 2 or 3 heteroatoms chosen from O, S, S(==O), SO₂, or N. Bicyclic substituents refer to two cycles, which are the same or different and which are chosen from aryl, heterocyclic ring, cycloalkyl or cycloalkenyl, fused together to form said bicyclic substituents. An example of a bicyclic substituent is indolyl.

In one case, a PDE7 inhibitor useful in the methods described herein has the formula:

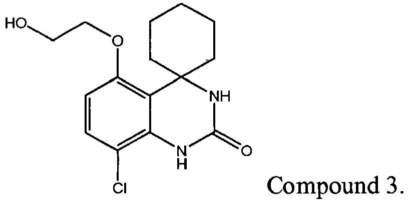

The activity of Compound 3 in inhibiting select PDEs is described in Examples 1 and 2. The effectiveness of Compound 3 in the MPTP Parkinson's model is described in Example 7.

In other cases, PDE7 inhibitors useful in the methods described herein have the formulas:

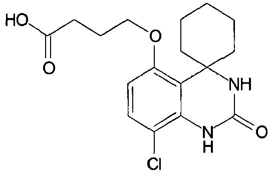

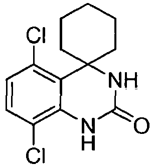

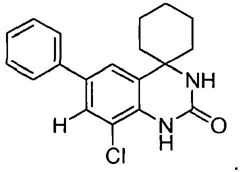

The preparation of the above compounds is described in US 2002/0198198, WO 2002/076953, WO 2002/074754, WO 2006/092691, Bioorganic & Medicinal Chemistry Letters 14 (2004) 4623-4626, and Bioorganic & Medicinal Chemistry Letters 14 (2004) 4627-4631.

In another case, PDE7 inhibitors useful in the methods described herein are selected from those compounds generally or specifically disclosed in EP 1 193 261, WO 2002/28847, US 20030045557, U.S. Patent No. 7,122,565, Bioorganic & Medicinal Chemistry Letters 14 (2004) 4607-4613, and Bioorganic & Medicinal Chemistry Letters 14 (2004) 4615-4621. In one case, PDE7 inhibitors useful in the methods described herein have the formula:

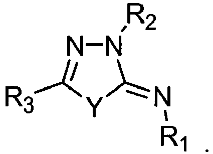

The substituents for the above compounds are defined as follows:
Y is S or O;
R₁ is C₁-C₁₀ alkyl, C₂-C₁₀ alkenyl, C₂-C₁₀ alkynyl, cycloalkyl, cycloalkenyl, heterocycle, aryl, or a polycyclic group; each optionally substituted with one or several groups X₁-R₄, identical or different, in which X₁ is a single bond, lower alkylene, C₂-C₆ alkenylene, cycloalkylene, arylene, or divalent heterocycle, and R₄ is:
   (1) H, =O, NO₂, CN, halogen, lower haloalkyl, lower alkyl, carboxylic acid bioisostere;
   (2) COOR₅, C(=O)R₅, C(=S)R₅, SO₂R₅, SOR₅, SO₃R₅, SR₅, OR₅;
   (3) C(=O)NR₇R₈, C(=S)NR₇R₈, C(=CH-NO₂)NR₇R₈, C(=N-CN)NR₇R₈, C(=N-SO₂NH₂)NR₇R₈, C(=NR₇)NHR₈, C(=NR₇)R₈, C(=NR₉)NHR₈, C(=NR₉)R₈, SO₂NR₇R₈, or NR₇R₈, wherein R₇ and R₈ are the same or different and are selected from OH, R₅, R₆, C(=O)NR₅R₆, C(=O)R₅, SO₂R₅, C(=NR₉)NHR₁₀, C(=NR₉)R₁₀, C(=CH-NO₂)NR₉R₁₀, C(=N-SO₂NH₂)NR₉R₁₀, C(=N-CN)NR₉R₁₀, or C(=S)NR₉R₁₀;
R₂ is lower alkyl, C₂-C₁₀ alkenyl, C₂-C₁₀ alkynyl, cycloalkyl, cycloalkenyl, heterocycle, aryl; each optionally substituted with one or several groups which are the same or different and which are selected from:
   (1) H, carboxylic acid bioisostere, lower haloalkyl, halogen,
   (2) COOR₅, OR₅, SO₂R₅,
   (3) SO₂NR₁₁R₁₂, C(=O)NR₁₁R₁₂, NR₁₁R₁₂, wherein R₁₁ and R₁₂ are the same or different and are selected from OH, R₅, R₆, C(=O)NR₅R₅, C(=O)R₅, SO₂R₅, C(=S)NR₉R₁₀, C(=CH-NO₂)NR₉R₁₀, C(=N-CN)NR₉R₁₀, C(=N-SO₂NH₂)NR₉R₁₀, C(=NR₉)NHR₁₀, or C(=NR₉)R₁₀;
R₃ is X₂-R'₃, wherein X₂ is a single bond or, a group selected from C₁-C₄ alkylene, C₂-C₆ alkenylene, C₂-C₆ alkynylene, each optionally substituted with one or several groups which are the same or different and which are selected from:
   (1) H, C₁-C₃ alkyl, C₃-C₄ cycloalkyl, aryl, heterocycle, =O, CN,
   (2) OR₅, =NR₅; or
   (3) NR₁₃R₁₄, wherein R₁₃ and R₁₄ are the same or different and are selected from R₅, R₆, C(=O)NR₅R₆, C(=O)R₅, SO₂R₅, C(=S)NR₉R₁₀, C(=CH-NO₂)NR₉R₁₀, C(=NR₉)NHR₁₀, or C(=NR₉)R₁₀;
R'₃ is cycloalkyl, cycloalkenyl, aryl, heterocycle, or a polycyclic group; each optionally substituted with one or several groups X₃-R₁₇ wherein X₃ is a single bond, lower alkylene, C₂-C₆ alkenylene, C₂-C₆ alkynylene, cycloalkylene, arylene, divalent heterocycle or a divalent polycyclic group, and, R₁₇ is:
   (1) H, =O, NO₂, CN, lower haloalkyl, halogen, carboxylic acid bioisostere, cycloalkyl,
   (2) COOR₅, C(=O)R₅, C(=S)R₅, SO₂R₅, SOR₅, SO₃R₅, SR₅, OR₅;
   (3) C(=O)NR₁₅R₁₆, C(=S)NR₁₅R₁₆, C(=N-CN)NR₁₅R₁₆, C(=N-SO₂NH₂)NR₁₅R₁₆, C(=CH-NO₂)NR₁₅R₁₆, SO₂NR₁₅R₁₆, C(=NR₁₅)NHR₁₆, C(=NR₁₅)R₁₆, C(=NR₉)NHR₁₆, C(=NR₉)R₁₆, or NR₁₅R₁₆ wherein R₁₅ and R₁₆ are the same or different and are selected from OH, R₅, R₆, C(=O)NR₅R₆, C(=O)R₅, SO₂R₅, C(=S)NR₉R₁₀, C(=CH-NO₂)NR₉R₁₀, C(=N-CN)NR₉R₁₀, C(=N-SO₂NH₂)NR₉R₁₀, C(=NR₉)NHR₁₀ or C(=NR₉)R₁₀,
   (4) heterocycle optionally substituted with one or several groups R₅;
wherein R₅ and R₆ are the same or different and are selected from H, lower alkyl, C₂-C₆ alkenyl, C₂-C₆ alkynyl, X₄-cycloalkyl, X₄-cycloalkenyl, X₄-aryl, X₄-heterocycle or X₄-polycyclic group, wherein X₄ is a single bond, lower alkylene, or C₂-C₆ alkenylene; each optionally substituted with one or several groups that are the same or different and selected from halogen, =O, COOR₂₀, CN, OR₂₀, O-lower alkyl optionally substituted with OR₂₀, C(=O)-lower alkyl, lower haloalkyl, 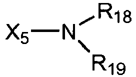 in which X₅ is a single bond or lower alkylene and R₁₈, R₁₉, and R₂₀, are the same or different and are selected from H or lower alkyl;
X₆-heterocycle, X₆-aryl, X₆-cycloalkyl, X₆-cycloalkenyl, or X₆-polycyclic group, wherein X₆ is a single bond or lower alkylene, these groups being optionally substituted with one or several groups, identical or different, selected from halogens, COOR₂₁, OR₂₁, or (CH₂)ₙNR₂₁R₂₂ in which n is 0, 1, or 2 and R₂₁ and R₂₂ are the same or different and are selected from H or lower alkyl;
R₉ is selected from H, CN, OH, lower alkyl, O-lower alkyl, aryl, heterocycle, SO₂NH₂, or 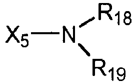 in which X₅ is a single bond or lower alkylene and R₁₈ and R₁₉ are the same or different and are selected from H or lower alkyl;
R₁₀ is selected from hydrogen, lower alkyl, cyclopropyl, or heterocycle;
or their pharmaceutically acceptable derivatives.

In regard to the above compounds, aryl refers to an unsaturated carbocycle, exclusively comprising carbon atoms in the cyclic structure, the number of which is between 5 and 10, including phenyl, naphthyl, or tetrahydronaphthyl. Heterocycle refers to a nonsaturated or saturated monocycle containing between 1 and 7 carbon atoms in the cyclic structure and at least one heteroatom in the cyclic structure, such as nitrogen, oxygen, or sulfur, preferably from 1 to 4 heteroatoms, identical or different, selected from nitrogen, sulfur and oxygen atoms. Suitable heterocycles include morpholinyl, piperazinyl, pyrrolidinyl, piperidinyl, pyrimidinyl, 2- and 3-furanyl, 2- and 3-thienyl, 2-pyridyl, 2- and 3-pyranyl, hydroxypyridyl, pyrazolyl, isoxazolyl, tetrazole, imidazole, triazole, and the like. Polycyclic groups include at least two cycles, identical or different, selected from aryl, heterocycle, cycloalkyl, cycloalkenyl groups fused together to form said polycyclic group such as 2- and 3-benzothienyl, 2- and 3-benzofuranyl, 2-indolyl, 2-and 3-quinolinyl, acridinyl, quinazolinyl, indolyl benzo[1,3]dioxolyl, and 9-thioxantanyl. Bicyclic groups refer to two cycles, which are the same or different and which are chosen from aryl, heterocycle, cycloalkyl or cycloalkenyl, fused together to form said bicyclic groups. Halogen refers to fluorine, chlorine, bromine, or iodine. Lower alkyl refers to an alkyl is linear or branched and contains 1 to 6 carbon atoms. Examples of lower alkyl groups include methyl, ethyl, propyl, butyl, isopropyl, tert-butyl, isobutyl, n-butyl, pentyl, hexyl and the like. Alkenyl refers to a linear or branched unsaturated carbon atom chain, comprising one or several double bonds, preferably one or two double bonds. Alkynyl refers to a linear or branched unsaturated carbon atom chain, comprising one or several triple bonds, preferably one or two triple bonds. Lower haloalkyl refers to a lower alkyl substituted with one or several halogens; preferred lower haloalkyl groups include perhaloalkyl groups such as CF₃. Cycloalkyl refers to saturated monocarbocyle containing from 3 to 10 carbon atoms; including cyclopropyl, cyclobutyl, cyclopentyl, cyclohexyl, and cycloheptyl. Cycloalkenyl refers to unsaturated monocarbocyle containing from 3 to 10 carbon atoms. Examples of suitable cycloalkenyl are 3-cyclohexene, and 3-cycloheptene. Carboxylic acid bioisostere has the classical meaning; common carboxylic acid bioisostere are tetrazole-5-yl, C(=O)N(H)OH, isoxazol-3-yl, hydroxythiadiazolyl, sulfonamido, sulfonylcarboxamido, phosphonic acid, phosphonamido, phosphinic acid, sulfonic acids, acyl sulfonamido, mercaptoazole, acyl cyanamides.

In one case, a PDE7 inhibitor useful in the methods described herein has the formula:

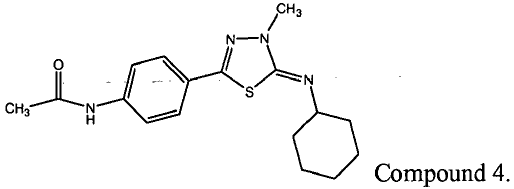

The activity of Compound 4 in inhibiting several PDEs is described in Examples 1 and 2. The effectiveness of Compound 4 in the MPTP Parkinson's model is described in Example 7.

In other cases, PDE7 inhibitors useful in the methods described herein have the formulas:

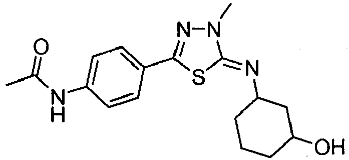

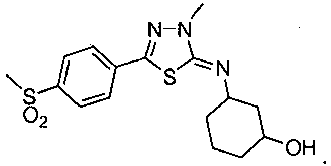

The preparation of the above compounds is described in EP 1 193 261, WO 02/28847, US 20030045557, U.S.Patent No. 7,122,565, Bioorganic & Medicinal Chemistry Letters 14 (2004) 4607-4613, and Bioorganic & Medicinal Chemistry Letters 14 (2004) 4615-4621.

In another case, PDE7 inhibitors useful in the methods described herein are selected from those compounds generally or specifically disclosed in WO 2004/111054, US 20060128728, and US 20070270419. In one case, PDE7 inhibitors useful in the methods described herein have the formulas:

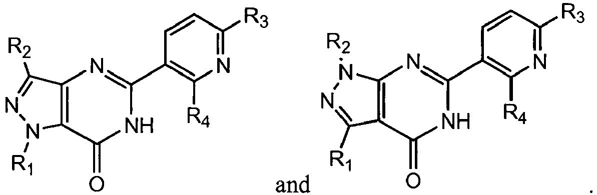

The substituents for the above compounds are defined as follows:
R₁ is a substituted or unsubstituted C₃₋₈ cycloalkyl group or tert-butyl group;
R₂ is a hydrogen atom or C₁₋₃ alkyl group;
R₃ is a group: NR₅R₆, C(=O)R₇, or S(O)₀₋₂R₈;
R₄ is a hydrogen atom or C₁₋₃ alkoxyl group which is unsubstituted or substituted by one or more fluorine atom(s);
R₅ and R₆ are, same or different from each other, a hydrogen atom, substituted or unsubstituted C₁₋₆ alkyl group, substituted or unsubstituted acyl group, substituted or unsubstituted heterocycloalkyl group, and substituted or unsubstituted heterocycloalkyl ring formed with a nitrogen atom which is binding R₅ and R₆;
R₇ is a group: OR₉ or NR₅R₆;
R₈ is a hydrogen atom, a halogen atom, a group: NR₅R₆, substituted or unsubstituted C₁₋₆ alkyl group, or substituted or unsubstituted aryl group;
R₉ is a hydrogen atom or substituted or unsubstituted C₁₋₆ alkyl group;
or pharmaceutically acceptable salts or solvates thereof.

In regard to the above compounds, the term "C₁-C₃ alkyl group" includes a straight or branched-chained alkyl group having 1 to 3 carbon atoms. The term "C₃-C₈ cycloalkyl group" includes a cycloalkyl group having 3 to 8 carbon atoms such as cyclopropyl, cyclobutyl, cyclopentyl, cyclohexyl and cyclooctyl. The term "heterocycloalkyl group" is 3 to 7 membered heterocyclic group containing the same or different 1 to 4 hetero atom(s) such as oxygen, nitrogen or sulfur atom(s), and examples may include pyrrolidinyl, piperidinyl, piperazinyl, homopiperazinyl, tetrahydrofuryl, tetrahydrophyranyl, morpholinyl and azetidinyl. The term "C₁-C₃ alkoxy group" means alkoxy group having 1 to 3 carbon atoms. The term "acyl group" means acyl group having 1 to 8 carbon atoms. The term "aryl group" is phenyl, naphthyl, biphenyl group, having 6 to 12 carbon atoms, and the term "heteroaryl group" is 5 to 7 membered monocyclic or polycyclic group thereof containing 2 to 8 carbon atoms and the same or different 1 to 4 hetero atom(s) such as oxygen, nitrogen, sulfur atom(s). The examples include pyrrole, furyl, thienyl, imidazolyl, thiazolyl, pyrazinyl, indolyl, quinolinyl, isoquinolinyl, tetrazolyl, pyridinyl, pyrazolyl pyridazinyl and pyrimidinyl. Examples of suitable substituent of "substituted or unsubstituted C₁-C₆ alkyl group" include hydroxyl group and halogen atom, and examples of suitable substituent of "substituted or unsubstituted acyl group" include halogen atom and nitro group. Further, examples of suitable substituent of "substituted or unsubstituted aryl group" include C₁-C₃ alkyl, halogen atom, amino group, acyl group, amide group, hydroxyl group, acylamino group, carboxyl group and sulfonyl group. Examples of suitable substituent of "substituted or unsubstituted C₃-C₈ cycloalkyl group" is C₁-C₃ alkyl, hydroxyl group and oxo group, and examples of suitable substituent of "substituted or unsubstituted heterocycloalkyl group" may include carboxy group, acyl group, alkoxy group, amino group, alkylamino group, acylamino group, hydroxyl group, oxo group, ethylenedioxy group, methyl group, ethyl group and hydroxyethyl group.

In other cases, PDE7 inhibitors useful in the methods described herein have the formulas:

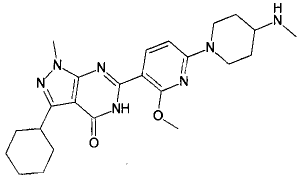

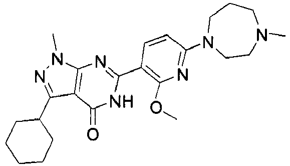

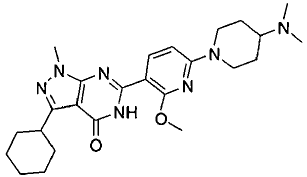

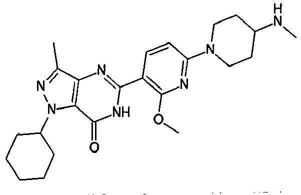

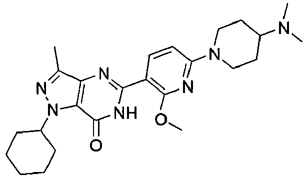

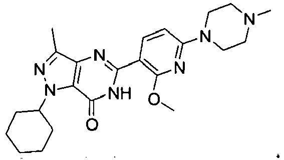

The preparation of the above compounds is described in WO 2004/111054, US 20060128728, and US 20070270419.

In another case, PDE7 inhibitors useful in the methods described herein are selected from those compounds generally or specifically disclosed in U.S. Patent No. 6,903,109, US 20040082578, WO 2003/088963, and US 20060154949. In one case, PDE7 inhibitors useful in the methods described herein have the formula:

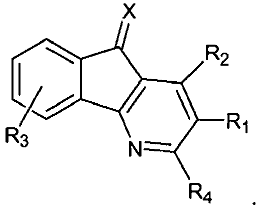

The substituents for the above compounds are defined as follows:
(a) R₁ is selected from the group consisting of:
   (i) COR₅, wherein R₅ is selected from H, optionally substituted C₁₋₈ straight or branched chain alkyl, optionally substituted aryl and optionally substituted arylalkyl; wherein the substituents on the alkyl, aryl and arylalkyl group are selected from C₁₋₈ alkoxy, phenylacetyloxy, hydroxy, halogen, p-tosyloxy, mesyloxy, amino, cyano, carboalkoxy, or NR₂₀R₂₁ wherein R₂₀ and R₂₁ are independently selected from the group consisting of hydrogen, C₁₋₈ straight or branched chain alkyl, C₃₋₇ cycloalkyl, benzyl, or aryl;
   (ii) COOR₆, wherein R₆ is selected from H, optionally substituted C₁₋₈ straight or branched chain alkyl, optionally substituted aryl and optionally substituted arylalkyl; wherein the substituents on the alkyl, aryl and arylalkyl group are selected from C1-8 alkoxy, phenylacetyloxy, hydroxy, halogen, p-tosyloxy, mesyloxy, amino, cyano, carboalkoxy, or NR₂₀R₂₁ wherein R₂₀ and R₂₁ are independently selected from the group consisting of hydrogen, C₁₋₈ straight or branched chain alkyl, C₃₋₇ cycloalkyl, benzyl, or aryl;
   (iii) cyano;
   (iv) a lactone or lactam formed with R₄;
   (v) CONR₇R₈ wherein R₇ and R₈ are independently selected from H, C₁₋₈ straight or branched chain alkyl, C₃₋₇ cycloalkyl, trifluoromethyl, hydroxy, alkoxy, acyl, alkylcarbonyl, carboxyl, arylalkyl, aryl, heteroaryl, and heterocyclyl; wherein the alkyl, cycloalkyl, alkoxy, acyl, alkylcarbonyl, carboxyl, arylalkyl, aryl, heteroaryl, and heterocyclyl groups may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, alkoxy, or arylalkyl;
      or R₇ and R₈ taken together with the nitrogen to which they are attached form a heterocyclyl or heteroaryl group;
   (vi) a carboxylic ester or carboxylic acid bioisostere including optionally substituted heteroaryl groups;
(b) R₂ is selected from the group consisting of optionally substituted alkyl, optionally substituted aryl, optionally substituted heteroaryl, optionally substituted C₃₋₇ cycloalkyl, optionally substituted heterocyclyl, wherein the heterocyclyl is 1,3-dioxolane or furan, or R₂ is 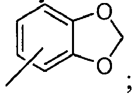
(c) R₃ is from one to four groups independently selected from the group consisting of:
   (i) hydrogen, halo, C₁₋₈ straight or branched chain alkyl, arylalkyl, C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, aryl, heteroaryl, and heterocyclyl;
   (ii) NR₁₀R₁₁ wherein R₁₀ and R₁₁ are independently selected from H, C₁₋₈ straight or branched chain alkyl, arylalkyl, C₃₋₇ cycloalkyl, carboxyalkyl, aryl, heteroaryl, or heterocyclyl, or R₁₀ and R₁₁ taken together with the nitrogen to which they are attached form a heterocyclyl or heteroaryl group;
   (iii) NR₁₂COR₁₃ wherein R₁₂ is selected from hydrogen or alkyl and R₁₃ is selected from hydrogen, alkyl, substituted alkyl, C₁₋₃ alkoxyl, carboxyalkyl, R₃₀R₃₁N(CH₂)ₚ, R₃₀R₃₁NCO(CH₂)ₚ, aryl, arylalkyl, heteroaryl, or heterocyclyl, or R₁₂ and R₁₃ taken together with the carbonyl group form a carbonyl containing heterocyclyl group, wherein R₃₀ and R₃₁ are independently selected from H, OH, alkyl, and alkoxy, and p is an integer from 1-6, wherein the alkyl group may be substituted with carboxyl, alkyl, aryl, substituted aryl, heterocyclyl, substituted heterocyclyl, heteroaryl, substituted heteroaryl, hydroxamic acid, sulfonamide, sulfonyl, hydroxy, thiol, alkoxy, or arylalkyl;
(d) R₄ is selected from the group consisting of (i) hydrogen, (ii) C₁₋₃ straight or branched chain alkyl, (iii) benzyl, and (iv) NR₁₃R₁₄, wherein R₁₃ and R₁₄ are independently selected from hydrogen and C₁₋₆ alkyl; wherein the C₁₋₃ alkyl and benzyl groups are optionally substituted with one or more groups selected from C₃₋₇ cycloalkyl, C₁₋₈ alkoxy, cyano, C₁₋₄ carboalkoxy, trifluoromethyl, C₁₋₈ alkylsulfonyl, halogen, nitro, hydroxy, trifluoromethoxy, C₁₋₈ carboxylate, amino, NR₁₃R₁₄, aryl, and heteroaryl; and
(e) X is selected from S and O;
and the pharmaceutically acceptable salts, esters and pro-drug forms thereof.

In an alternative case, R₁, R₃, and R₄ are as above and R₂ is NR₁₅R₁₆, where R₁₅ and R₁₆ are independently selected from hydrogen, C₁₋₈ straight or branched chain alkyl, arylalkyl, C₃₋₇ cycloalkyl, aryl, heteroaryl, and heterocyclyl, or R₁₅ and R₁₆ taken together with the nitrogen to which they are attached form a heterocyclyl or heteroaryl group.

In regard to the above compounds, "alkyl" refers to straight, cyclic and branched-chain alkyl. The alkyl group may be optionally substituted with one or more groups such as halogen, OH, CN, mercapto, nitro, amino, C₁-C₈-alkyl, C₁-C₈-alkoxyl, C₁-C₈-alkylthio, C₁-C₈-alkyl-amino, di(C₁-C₈-alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, carboxamide, hydroxamic acid, sulfonamide, sulfonyl, thiol, aryl, aryl(c₁-c₈)alkyl, heterocyclyl, and heteroaryl. The term "bioisostere" is defined as "groups or molecules which have chemical and physical properties producing broadly similar biological properties." (Burger's Medicinal Chemistry and Drug Discovery, M. E. Wolff, ed. Fifth Edition, Vol. 1, 1995, Pg. 785). The term "acyl" as used herein, whether used alone or as part of a substituent group, means an organic radical having 2 to 6 carbon atoms (branched or straight chain) derived from an organic acid by removal of the hydroxyl group. "Aryl" or "Ar," whether used alone or as part of a substituent group, is a carbocyclic aromatic radical including, but not limited to, phenyl, 1- or 2-naphthyl and the like. The carbocyclic aromatic radical may be substituted by independent replacement of 1 to 5 of the hydrogen atoms thereon with halogen, OH, CN, mercapto, nitro, amino, C₁-C₈-alkyl, C₁-C₈-alkoxyl, C₁-C₈-alkylthio, C₁-C₈-alkyl-amino, di(C₁-C₈ -alkyl)amino, (mono-, di-, tri-, and per-) halo-alkyl, formyl, carboxy, alkoxycarbonyl, C₁-C₈-alkyl-CO-O-, C₁-C₈-alkyl-CO-NH-, or carboxamide. Illustrative aryl radicals include, for example, phenyl, naphthyl, biphenyl, fluorophenyl, difluorophenyl, benzyl, benzoyloxyphenyl, carboethoxyphenyl, acetylphenyl, ethoxyphenyl, phenoxyphenyl, hydroxyphenyl, carboxyphenyl, trifluoromethylphenyl, methoxyethylphenyl, acetamidophenyl, tolyl, xylyl, dimethylcarbamylphenyl and the like. The term "heteroaryl" refers to a cyclic, fully unsaturated radical having from five to ten ring atoms of which one ring atom is selected from S, O, and N; 0-2 ring atoms are additional heteroatoms independently selected from S, O, and N; and the remaining ring atoms are carbon. The radical may be joined to the rest of the molecule via any of the ring atoms. The terms "heterocycle," "heterocyclic," and "heterocycle" refer to an optionally substituted, fully or partially saturated cyclic group which is, for example, a 4-to 7-membered monocyclic, 7- to 11-membered bicyclic, or 10- to 15-membered tricyclic ring system, which has at least one heteroatom in at least one carbon atom containing ring. Each ring of the heterocyclic group containing a heteroatom may have 1, 2, or 3 heteroatoms selected from nitrogen atoms, oxygen atoms, and sulfur atoms, where the nitrogen and sulfur heteroatoms may also optionally be oxidized. The nitrogen atoms may optionally be quaternized. The heterocyclic group may be attached at any heteroatom or carbon atom.

In other cases, PDE7 inhibitors useful in the methods described herein have the formulas:

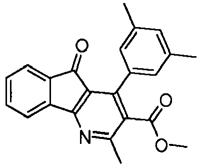

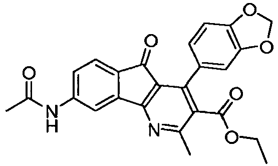

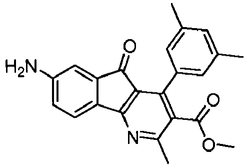

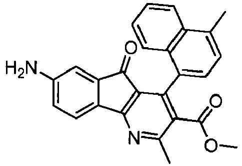

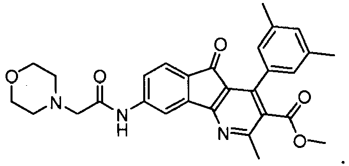

The preparation of the above compounds is described in U.S. Patent No. 6,903,109, US 20040082578, WO 2003/088963, and US 20060154949.

In another case, PDE7 inhibitors useful in the methods described herein are selected from those compounds generally or specifically disclosed in U.S. Patent No. 6,958,328, WO 2002/085894, and US 20030212089. These PDE7 inhibitors have the same formula as those described above (e.g., U.S. Patent No. 6,903,109), except that R₁ is not a carboxylic ester or carboxylic acid bioisostere. The preparation of these compounds is described in U.S. Patent No. 6,958,328, US 20030212089, and WO 2002/085894.

In another case, PDE7 inhibitors useful in the methods described herein are selected from those compounds generally or specifically disclosed in WO 2006/004040 and EP 1 775 298. In one case, PDE7 inhibitors useful in the methods described herein have the formula:

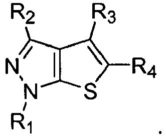

The substituents for the above compounds are defined as follows:
R₁ is substituted or unsubstituted C₃₋₈ alkyl group, substituted or unsubstituted cycloalkyl group, or substituted or unsubstituted heterocycloalkyl group (e.g., cyclohexyl, cycloheptyl, or tetrahydropyranyl);
R₂ is a hydrogen atom or substituted or unsubstituted C₁₋₃ alkyl group (e.g., methyl);
R₃ is a hydrogen atom, substituted or unsubstituted C₁₋₃ alkyl group, or a halogen atom; and
R₄ is substituted or unsubstituted aryl group, substituted or unsubstituted heteroaryl group, or a group CONR₅R₆, or CO₂R₇,
wherein R₅ and R₆ are, same or different from each other, a hydrogen atom; substituted or unsubstituted C₁₋₆ alkyl group which may be substituted by a halogen atom, substituted or unsubstituted aryl group, substituted or unsubstituted heteroaryl group, substituted or unsubstituted heterocycloalkyl group, substituted or unsubstituted cycloalkyl group, a group NR₇COR₈, COR₈, NR₉R₁₀; substituted or unsubstituted cycloalkyl group; substituted or unsubstituted heterocycloalkyl group; substituted or unsubstituted aryl group; substituted or unsubstituted heteroaryl group;or substituted or unsubstituted heterocycloalkyl group in which the ring is formed together with the nitrogen atom binding R₅ and R₆;
wherein R₇ is a hydrogen atom or substituted or unsubstituted C₁₋₃ alkyl group;
wherein R₈ is substituted or unsubstituted heterocycloalkyl group, or a group OH, OR₇, or NR₉R₁₀;
wherein R₉ and R₁₀ are, same or different from each other, a hydrogen atom; substituted or unsubstituted C₁₋₃ alkyl group, substituted or unsubstituted heterocycloalkyl group; substituted or unsubstituted acyl; a group SO₂R₇, or substituted or unsubstituted heterocycloalkyl group in which the ring is formed together with the nitrogen atom binding R₅ and R₆;
or pharmaceutically acceptable salts or solvates thereof.

In regard to the above compounds, the term "cycloalkyl group" means cycloalkyl group having 3 to 8 carbon atoms. The term "heterocycloalkyl group" may be 3 to 7 membered monocyclic or polycyclic heterocyclic group containing the same or different 1 to 4 hetero atom(s) such as oxygen, nitrogen or sulfur atom(s). The term "aryl group" may be aromatic hydrocarbon group, which consists of mono-benzene ring, or binding or condensed benzene ring, such as phenyl, naphthyl, biphenyl and the like; and dicyclic or tricyclic group, which consists of benzene ring condensed with cycloalkyl or heterocyclic ring, such as 1,2,3,4-tetrahydronaphthalene, 2,3-dihydroindene, indoline, coumarone and the like. The term "heteroaryl group" may be 5 to 7 membered monocyclic heteroaryl group or polycyclic heteroaryl group, and having 2 to 8 carbon atoms with 1 to 4 hetero atom(s) such as oxygen, nitrogen, sulfur atom(s), in which the polycyclic heteroaryl group has condensed ring system by the same or different monocyclic heteroaryl or benzene ring each other; or polycyclic group which is consisted of heteroaryl group condensed with cycloalkyl or heterocycloalkyl ring. Examples of suitable substituent of the present invention may include straight, branched-chained or cyclic C₁-C₈ alkyl group, which may be substituted by one or more methyl, ethyl, propyl, isopropyl, n-butyl, t-butyl, cyclohexyl, cycloheptyl, methoxymethyl, hydroxymethyl, trifluoromethyl, C₁-C₃ alkoxy group, halogen atom, and hydroxyl group; hydroxyl group; cyano group; substituted or unsubstituted alkoxy group such as methoxy, ethoxy group; amino group which may be substituted by C₁-C₆ alkyl group or acyl group such as amino, methylamino, ethylamino, dimethylamino, acylamino and the like; carboxylic group; substituted or unsubstituted ester group; phosphate group; sulfonic group; substituted or unsubstituted aryl group; substituted or unsubstituted heteroaryl group; saturated or unsaturated heterocycloalkyl group which may be substituted; substituted or unsubstituted carbamoyl group; substituted or unsubstituted amide group; substituted or unsubstituted thioamide group; halogen atom; nitro group; substituted or unsubstituted sulfone group; substituted or unsubstituted sulfonylamide group; oxo group; substituted or unsubstituted urea group; straight, branched-chained or cyclic alkenyl group such as ethenyl, propenyl, cyclohexenyl and the like.

In other cases, PDE7 inhibitors useful in the methods described herein have the formulas:

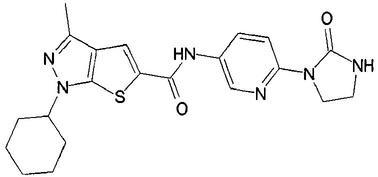

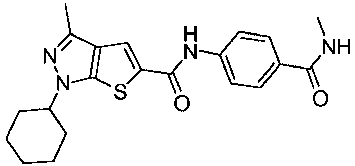

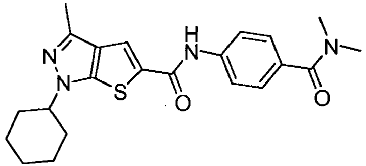

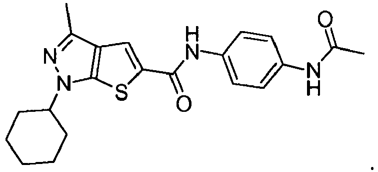

The preparation of the above compounds is described in EP 1 775 298 and WO 2006/004040.

In another case, PDE7 inhibitors useful in the methods described herein are selected from those compounds generally or specifically disclosed in WO 2004/111053 and US 20060128707. In one case, PDE7 inhibitors useful in the methods described herein have the formulas:

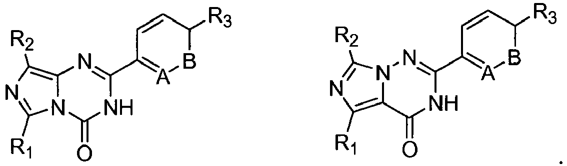

The substituents for the above compounds are defined as follows:
A is N or CR₄;
B is N or CH;
R₁ is substituted or unsubstituted C₃₋₈ cycloalkyl group or tert-butyl group;
R₂ is a hydrogen atom or C₁₋₆ alkyl group;
R₃ is a hydrogen atom; nitro group; cyano group; a halogen atom; heteroaryl group; substituted or unsubstituted C₁₋₆ alkyl group; substituted or unsubstituted C₂₋₆ alkenyl group; saturated or unsaturated heterocycloalkyl group which is substituted or unsubstituted; a group: NR₅R₆, C(O)R₇, SO₂R₇, OR₈, NR₈COR₇, NR₈SO₂R₇;
R₄ is a hydrogen atom or C₁₋₃ alkoxy group which is unsubstituted or substituted by one or more fluorine atom(s);
R₅ and R₆ are, same or different from each other, a hydrogen atom; substituted or unsubstituted C₁₋₆ alkyl group; substituted or unsubstituted acyl group; or substituted or unsubstituted heterocycloalkyl group;
R₇ is a hydrogen atom; substituted or unsubstituted C₁₋₆ alkyl group; substituted or unsubstituted heterocycloalkyl group; OH; OR₈ or NR₅R₆;
R₈ is a hydrogen atom, substituted or unsubstituted C₁₋₆ alkyl group; or substituted or unsubstituted heterocycloalkyl group;
or pharmaceutically acceptable salts or solvates thereof.

In regard to the above compounds, the term "C₁-C₆ alkyl group" refers to a straight or branched-chained alkyl group having 1 to 6 carbon atoms, and the term "C₂-C₆ alkenyl group" refers to a straight or branched-chained alkenyl group having 2 to 6 carbon atoms. The term "cycloalkyl group" refers to a cycloalkyl group having 3 to 8 carbon atoms such as cyclopropyl, cyclobutyl, cyclopentyl, cyclohexyl, cycloheptyl, and cyclooctyl. The term "heterocycloalkyl group" is 3 to 7 membered heterocyclic group containing the same or different 1 to 4 hetero atom(s) such as oxygen, nitrogen or sulfur atom(s), and examples may include piperidinyl, pyrrolidinyl, piperazinyl, tetrahydrofuryl, tetrahydropyranyl, morpholinyl, azetidinyl, and homopiperazinyl. The term "heteroaryl group" is 5 to 7 membered monocyclic or polycyclic group thereof containing 2 to 8 carbon atoms and the same or different 1 to 4 hetero atom(s) such as oxygen, nitrogen or sulfur atom(s). The examples include pyrrole, furyl, thienyl, imidazolyl, thiazolyl, pyrazinyl, indolyl, quinolinyl, isoquinolinyl, tetrazolyl, pyridinyl, pyrazolyl, pyridazinyl, and pyrimidinyl. The "halogen atom" includes fluorine, chlorine, bromine and iodine. Examples of the suitable substituent of "substituted or unsubstituted C₁-C₆ alkyl group", "substituted or unsubstituted C₃-C₈ cycloalkyl group", "substituted or unsubstituted alkenyl group", "substituted or unsubstituted heterocycloalkyl group" and "substituted or unsubstituted acyl group" include a straight or branched-chained, or substituted or unsubstituted alkyl group such as methyl, ethyl, propyl, isopropyl, n-butyl, tert-butyl, substituted or unsubstituted cycloalkyl group such as cyclopropyl, cyclobutyl, cyclopentyl, cyclohexyl, and cycloheptyl; hydroxyl group; cyano group; alkoxy group such as methoxy and ethoxy; substituted or unsubstituted amino group such as amino, methylamino, ethylamino, and dimethylamino; substituted or unsubstituted acyl group such as acetyl, and propionyl; substituted or unsubstituted aryl group; substituted or unsubstituted heteroaryl group; saturated or unsaturated heterocycloalkyl group which is substituted or unsubstituted; substituted or unsubstituted carbamoyl group; substituted or unsubstituted amide group; halogen atom; nitro group; substituted or unsubstituted sulfone group; oxo group; urea group; a straight or branched-chained, or cyclic alkenyl group which is substituted or unsubstituted such as ethenyl, propenyl, and cyclohexenyl.

In other cases, PDE7 inhibitors useful in the methods described herein have the formulas:

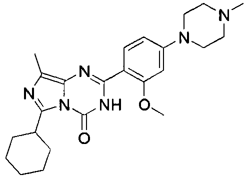

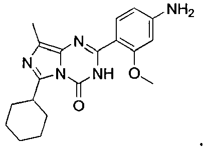

The preparation of the above compounds is described in US 20060128707 and WO 2004/111053.

In another case, PDE7 inhibitors useful in the methods described herein are selected from those compounds generally or specifically disclosed in U.S. Patent No. 6,617,357, US 20020156064, and Molecular Pharmacology, 66:1679-1689, 2004. In one case, PDE7 inhibitors useful in the methods described herein have the formula:

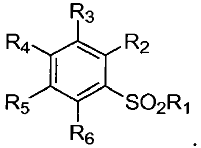

The substituents for the above compounds are defined as follows:
R₁ is NRₐR_{b} where Rₐ and R_{b} are independently H or C₁₋₆ alkyl, or represents a 5 to 7 member ring comprised of carbon or carbon and one or more additional heteroatoms selected from O, N, or S;
R₂ is H, C₁₋₈ alkyl, C₁₋₃ alkyl-Ar, C₁₋₃ alkyl-C₃₋₆ cycloalkyl, C₂₋₈ alkenyl, C₂₋₄ alkenyl-Ar, or C₂₋₄ alkenyl-C₃₋₆ cycloalkyl, wherein Ar is substituted or unsubstituted phenyl;
R₃ is NO₂, halo, CN, C(O)OR₇, COR₁, or NRₐR_{b} where Rₐ and R_{b} are independently H or C₁₋₆ alkyl;
R₄ is H, OC₁₋₆ alkyl, halo, C(O)NRₐR_{b}, C(O)OR₇, C₁₋₈ alkyl, OCHF₂, CH₂OR₈, OC₁₋₃ alkyl-Ar, or CH₂NHC(O)CH₃;
R₅ is H, halo, or alkyl;
R₆ is C₁₋₈ alkyl, OC₁₋₄ alkyl, or halo;
R₇ is hydrogen or an ester or amide-forming group;
R₈ is hydrogen or C₁₋₆ alkyl;
or a pharmaceutically acceptable salt or solvate thereof.

In one case, a PDE7 inhibitor useful in the methods of the invention has the formula:

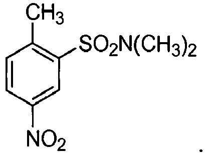

The preparation of the above compounds is described in U.S. Patent No. 6,617,357, US 20020156064, and Molecular Pharmacology, 66:1679-1689, 2004.

In another case, PDE7 inhibitors useful in the methods described herein are selected from those compounds generally or specifically disclosed in U.S. Patent No. 6,852,720, EP 1 348 433, and WO 2003/082277. In one case, PDE7 inhibitors useful in the methods described herein have the formula:

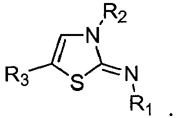

The substituents for the above compounds are defined as follows:
R₁ is a group selected from cycloalkyl, heterocycloalkyl, aryl and heteroaryl, those groups being optionally substituted by one or more groups, identical or different, selected independently of each other from halogen, trifluoromethyl, nitro, cyano, oxo, NR₄R₅, CO₂R₄, CONR₄R₅, OR₄, S(O)ₙR₄, S(O)ₙNR₄R₅, tetrazolyl and (C₁-C₆) alkyl which is optionally substituted by 1 to 3 groups, identical or different, selected independently of each other from OR₄, NR₄ R₅, and CO₂ R₄; wherein n is an integer from 0 to 2 inclusive, R₄ and R₅ are identical or different and independently of each other are a hydrogen atom or a group of formula X₁-Rₐ, wherein X₁ is a single bond or a (C₁-C₆) alkylene group, and Rₐ is a group selected from (C₁-C₆) alkyl, cycloalkyl, heterocycloalkyl, aryl, and heteroaryl,
R₂ is a group selected from (C₁-C₆) alkyl, (C₂-C₆) alkenyl, (C₂-C₆) alkynyl, aryl, and cycloalkyl,
R₃ is a group selected from cycloalkyl, heterocycloalkyl, aryl and heteroaryl, these groups being optionally substituted by one or more groups, identical or different, selected independently of each other from halogen, nitro, cyano, trifluoromethyl, oxo, (C₁-C₆) alkyl, OR₆, NR₆R₇, COR₆, CO₂R₆, CONHOH, CONR₆R₇, S(O)ₘR₆, S(O)ₘNR₆R₇, NR₆COR₇, NR₆SO₂R₇, N(SO₂R₇)₂, NR₆CONR₇R₈, C(=NCN)NR₆R₇, NR₈C(=NCN)NR₅R₇, and tetrazolyl optionally substituted with a (C₁-C₄) alkyl, wherein m is an integer from 0 to 2 inclusive, R₆ and R₇ are identical or different and independently of each other are a hydrogen atom or a group of formula X₂R_{b}, wherein X₂ is a single bond or a (C₁-C₆) alkylene group, R_{b} is a group selected from (C₁-C₆) alkyl, cycloalkyl, heterocycloalkyl, aryl and heteroaryl, these groups being optionally substituted by 1 to 3 groups, identical or different, selected independently of each other from hydroxy, (C₁-C₆) alkoxy, (C₁-C₆) alkyl, amino, mono(C₁-C₆) alkylamino, di(C₁-C₆) alkylamino (each alkyl amino being identical or different, independently of each other), carboxy, (C₁-C₆) alkoxycarbonyl, and benzyl, and R₈ represents a hydrogen atom or a (C₁-C₆) alkyl group;
a racemic form thereof, an isomer thereof, an N-oxide thereof, or a pharmaceutically acceptable acid or base salt thereof.

The preparation of the above compounds is described in U.S. Patent No. 6,852,720, EP 1 348 433, and WO 2003/082277.

In another case, PDE7 inhibitors useful in the methods described herein are selected from those compounds generally or specifically disclosed in U.S. Patent No. 6,753,340, US 20030191167, EP 1 348 701, and WO 2003/082839. In one case, PDE7 inhibitors useful in the methods described herein have the formula:

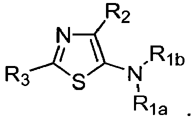

The substituents for the above compounds are defined as follows:
R₁ₐ is a group selected from hydrogen, (C₁-C₆) alkyl and aryl(C₁-C₆) alkyl,
R_{1b} is a group selected from cycloalkyl, heterocycloalkyl, aryl and heteroaryl, those groups being optionally substituted by one or more groups, identical or different, selected independently of each other from halogen, trifluoromethyl, nitro, cyano, oxo, NR₄R₅, CO₂R₄, CONR₄R₅, OR₄, S(O)ₙR₄, S(O)ₙNR₄R₅, tetrazolyl, and (C₁-C₆) alkyl which is optionally substituted by 1 to 3 groups, identical or different, selected independently of each other from OR₄, NR₄ R₅, and CO₂R₄, wherein n is an integer from 0 to 2 inclusive, R₄ and R₅ are identical or different and independently of each other are a hydrogen atom or a group of formula X₁-Rₐ, wherein X₁ is a single bond or a (C₁-C₆) alkylene group, and Rₐ is a group selected from (C₁-C₆) alkyl, cycloalkyl, heterocycloalkyl, aryl and heteroaryl,
R₂ is a group selected from (C₁-C₆) alkyl, (C₂-C₆) alkenyl, (C₂-C₆) alkynyl, aryl and cycloalkyl,
R₃ is a group selected from cycloalkyl, heterocycloalkyl, aryl and heteroaryl, these groups being optionally substituted by one or more groups, identical or different, selected independently of each other from halogen, nitro, cyano, trifluoromethyl, oxo, (C₁-C₆) alkyl, OR₆, NR₆R₇, COR₆, CO₂R₆, CONHOH, CONR₆R₇, S(O)ₘR₆, S(O)ₘNR₆R₇, NR₆COR₇, NR₆SO₂R₇, N(SO₂R₇)₂, NR₆CONR₇R₈, C(=N-CN)NR₆R₇, NR₈C(=N-CN)NR₆R₇, and tetrazolyl optionally substituted with a (C₁-C₄) alkyl, wherein m is an integer from 0 to 2 inclusive, R₆ and R₇ are identical or different and independently of each other are a hydrogen atom or a group of formula X₂-R_{b}, wherein X₂ is a single bond or a (C₁-C₆) alkylene group, R_{b} is a group selected from (C₁-C₆) alkyl, cycloalkyl, heterocycloalkyl, aryl and heteroaryl, these groups being optionally substituted by 1 to 3 groups, identical or different, selected independently of each other from hydroxy, (C₁-C₆) alkoxy, (C₁-C₆) alkyl, amino, mono(C₁-C₆) alkylamino, di(C₁-C₆) alkylamino (each alkyl amino being identical or different, independently of each other), carboxy, (C₁-C₆) alkoxycarbonyl, and benzyl, and R₈ is a hydrogen atom or a (C₁-C₆) alkyl group, or
a racemic form thereof, an isomer thereof, an N-oxide thereof or a pharmaceutically acceptable acid or base salt thereof.

The preparation of these compounds is described in U.S. Patent No. 6,753,340, US 20030191167, EP 1 348 701, and WO 2003/082839.

In another case, PDE7 inhibitors useful in the methods described herein are selected from those compounds generally or specifically disclosed in U.S. Patent No. 6,849,638, US 20030119829, and WO 2002/088138.

In one case, PDE7 inhibitors useful in the methods described herein have the formula:

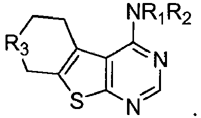

The substituents for the above compounds are defined as follows:
R₁ and R₂ are independently selected from the group consisting of hydrogen, alkyl of 1-8 carbon atoms, alkenyl of 2-8 carbon atoms, alkynyl of 2-8 carbon atoms, cycloalkyl of 3-7 carbon atoms, fully saturated heterocycle of 2-6 carbon atoms and 1-2 heteroatoms selected from NH, S and O, aryl of 6-12 carbon atoms, that may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1, 2 heteroatoms selected from N, S, and O, heteroaryl of 4-11 carbon atoms and 1-2 heteroatoms selected from N, S and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S and O, and R₄-R₅, or R₁ and R₂ combine to form, together with the nitrogen atom to which they are attached, a 5-7 membered saturated ring which may contain 1-2 additional heteroatoms selected from the group consisting of NH, NR₈, S and O, or combine to form, together with the nitrogen atom to which they are attached, a 5-7 membered unsaturated ring that may contain 1-2 additional heteroatoms selected from the group consisting of N, S and O,
wherein said saturated or unsaturated ring may be substituted with 1-2 substituents selected from the group consisting of OH, alkyl of 1-6 carbon atoms, alkenyl of 2-6 carbon atoms, alkynyl of 2-6 carbon atoms, cycloalkyl of 3-7 carbon atoms, fully saturated heterocycle of 2-6 carbon atoms and 1-2 heteroatoms selected from NH, S, and O, halogen, haloalkyl of 1-2 carbon atoms and a number of halogen atoms up to the perhalo level, alkoxy of 1-6 carbon atoms, haloalkoxy of 1-6 carbon atoms and a number of halogen atoms up to the perhalo level, and R₉-R₁₀; or
R₁ and R₂ combine to form, together with the nitrogen atom to which they are attached, an 8-10 membered bicyclic saturated ring;
R₃ is selected from the group consisting of NH, S, S(=O)₂, and O;
R₄ is selected from alkyl of 1-8 carbon atoms, alkenyl of 2-8 carbon atoms, alkynyl of 2-8 carbon atoms, C(=C), S(=O)₂, and C(=O)O;
R₅ is selected from hydrogen, OH, alkyl of 1-8 carbon atoms, alkenyl of 2-8 carbon atom, alkynyl of 2-8 carbon atoms, alkoxy of 1-8 carbon atoms, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkyl of 1-6 carbon atoms and a number of halogen atoms up to the perhalo level, aryl of 6-12 carbon atoms and heteroaryl of 4-11 carbon atoms and 1-2 heteroatoms selected from N, S, and O, heteroaryl of 4-11 carbon atoms and 1-2 heteroatoms selected from N, S, and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms and heteroaryl of 4-11 carbon atoms and 1-2 heteroatoms selected from N, S, and O, cycloalkyl of 3-7 carbon atoms, fully saturated heterocycle of 2-6 carbon atoms and 1-2 heteroatoms selected from NH, S and O, and NR₆R₇,
R₆ and R₇ are independently selected from hydrogen, alkyl of 1-8 carbon atoms, alkenyl of 2-8 carbon atoms, and alkynyl of 2-8 carbon atoms, or R₆ and R₇ combine together with the nitrogen atom to which they are attached to form a 5-7 membered, unsaturated ring which may contain 1-2 additional heteroatoms selected from N, S and O or to form a 5-7 membered, saturated ring which may contain 1-2 additional heteroatoms selected from NH, S, and O;
R₈ is selected from alkyl of 1-8 carbon atoms, alkenyl of 2-8 carbon atoms, alkynyl of 2-8 carbon atoms, R₁₁-R₁₂, cycloalkyl of 3-7 carbon atoms, fully saturated heterocycle of 2-6 carbon atoms and 1-2 heteroatoms selected from NH, S, and O, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S, and O, heteroaryl of 4-11 carbon atoms and 1-2 heteroatoms selected from N, S, and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S, and O;
R₉ is selected from alkyl of 1-8 carbon atoms, alkenyl of 2-8 carbon atoms, and alkynyl of 2-8 carbon atoms,
R₁₀ is selected from OH, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S, and O, and heteroaryl of 4-11 carbon atoms and 1-2 heteroatoms selected from N, S, and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S, and O;
R₁₁ is selected from alkyl of 1-8 carbon atoms, alkenyl of 2-8 carbon atoms, and alkynyl of 2-8 carbon atoms; and
R₁₂ is selected from cycloalkyl of 3-7 carbon atoms, fully saturated heterocycle of 2-6 carbon atoms and 1-2 heteroatoms selected from NH, S, and O, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S, and O, and heteroaryl of 4-11 carbon atoms and 1-2 heteroatoms selected from N, S and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S and O;
and pharmaceutically acceptable salts thereof.

The preparation of these compounds is described in U.S. Patent No. 6,849,638, US 20030119829, and WO 2002/088138.

In another case, PDE7 inhibitors useful in the methods described herein are selected from those compounds generally or specifically disclosed in US 2005222138 and WO 2003/064389. In one case, PDE7 inhibitors useful in the methods described herein have the formula:

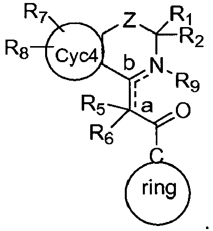

The substituents for the above compounds are defined as follows:
R₁ and R₂ are each independently, (1) hydrogen atom, or (2) C₁₋₈ alkyl, or
R₁ and R₂ may be taken together with the carbon atom to which they are attached to form Cyc1,
wherein R₁ and R₂ do not represent hydrogen atom at the same time;
Z is (1) CR₃R₄, (2) O, (3) S, or (4) a bond;
R₃ and R₄ are each independently, (1) hydrogen atom, (2) C₁₋₈ alkyl, (3) C₁₋₈ alkoxy, or (4) hydroxy, or
R₃ and R₄ may be taken together with the carbon atom to which they are attached to form Cyc1 or C(O);
R₅ and R₆ are each independently, (1) hydrogen atom, or (2) C₁₋₈ alkyl, or
R₅ and R₆ may be taken together with the carbon atom to which they are attached to form Cyc1;
Cyc1, which is represented by R₁ and R₂, R₃ and R₄, R₅ and R₆ is, each independently, (1) C₃₋₁₀ cycloalkyl, or (2) 3-10 membered monocyclic hetero-ring comprising 1-2 of heteroatom selected from oxygen, nitrogen and sulfur, and Cyc1 may be substituted with R₁₀;
R₁₀ is (1) C₁₋₈ alkyl, (2) C₁₋₈ alkoxy, (3) hydroxy, (4) COOR₁₁, (5) oxo, (6)SO₂R₁₂, or (7) COR₁₃;
R₁₁ is hydrogen atom, or C₁₋₈ alkyl;
R₁₂ and R₁₃ are (1) C₁₋₈ alkyl, or (2) phenyl which may be substituted with C₁₋₈ alkyl;
R₇ and R₈ are each independently, (1) hydrogen atom, (2) C₁₋₈ alkyl, (3) C₁₋₈ alkoxy, (4) hydroxy, (5) cyano, (6) halogen atom, (7) COOR₁₄, (8) CONR₁₅R₁₆, (9) Cyc2, (10) C₂₋₈ alkenyl, (11) C₂₋₈ alkynyl, (12) NR₅₁R₅₂, (13) nitro, (14) formyl, (15) C₂₋₈ acyl, (16) C₁₋₈ alkyl substituted with hydroxy, C₁₋₈ alkoxy, Cyc2, NR₅₁R₅₂, or NR₅₃-Cyc2, (17) NR₅₄COR₅₅, (18) NR₅₆SO₂R₅₇, (19) SO₂NR₅₈R₅₉, (20) C₂₋₈ alkenyl substituted with COOR₁₄, (21) CH=N-OH, (22) C₁₋₈ alkylene-NR₆₀-(C₁₋₈ alkylene)-R₆₁, (23) C₁₋₈ alkylthio, (24) C₁₋₈ alkyl substituted with 1-3 of halogen atom, (25) C₁₋₈ alkoxy substituted with 1-3 of halogen atom, (26) C₁₋₈ alkoxy substituted with Cyc2, (27) O-Cyc2, (28) OSO₂R₆₅, or (29) CH=N-OR₁₃₇;
R₁₄ is hydrogen atom, or C₁₋₈ alkyl;
R₁₅ and R₁₆ are each independently hydrogen atom or C₁₋₈ alkyl;
R₅₁ and R₅₂, R₅₈ and R₅₉ are each independently, hydrogen atom, or C₁₋₈ alkyl;
R₅₃, R₅₄, R₅₆, and R₆₀ are each independently, hydrogen atom, or C₁₋₈ alkyl;
R₅₅ is hydrogen atom, C₁₋₈ alkyl, or C₁₋₈ alkoxy; R₅₇ is C₁₋₈ alkyl;
R₆₁ is NR₆₂R₆₃ or hydroxy;
R₆₂ and R₆₃ are each independently, hydrogen atom, or C₁₋₈ alkyl;
R₆₅ is C₁₋₈ alkyl;
R₁₃₇ is C₁₋₈ alkyl; 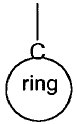 (hereinafter it is abbreviated as ring) is Cyc2 wherein the group which attaches to carbonyl is carbon;
R₇, R₈, and Cyc2 represented by ring are each independently, (1) C₃₋₁₅ mono-, bi-or tri-cyclic (fused or spiro)carboring, or (2) 3-15 membered mono-, bi- or tri-cyclic (fused or spiro)heteroring comprising 1-4 of heteroatom selected from oxygen, nitrogen and sulfur;
Cyc2 may be substituted with 1-5 of R₁₇ or R_{17'};
R₁₇ is (1) C₁₋₈ alkyl, (2) C₂₋₈ alkenyl, (3) C₂₋₈ alkynyl, (4) C₁₋₈ alkoxy, (5) C₁₋₈ alkylthio, (6) hydroxy, (7) halogen atom, (8) nitro, (9) oxo, (10) carboxy, (11) formyl, (12) cyano, (13) NR₁₈R₁₉, (14) phenyl, phenoxy or phenylthio, which may be substituted with 1-5 of R₂₀, (15) C₁₋₈ alkyl, C₂₋₈ alkenyl, C₁₋₈ alkoxy or C₁₋₈ alkylthio, which may be substituted with 1-5 of R₂₁ (16) OCOR₂₂, (17) CONR₂₃R₂₄, (18) SO₂NR₂₅R₂₆ (19) COOR₂₇, (20) COCOOR₂₈, (21) COR₂₉, (22) COCOR₃₀, (23) NR₃₁COR₃₂, (24) SO2R₃₃, (25) NR₃₄SO₂R₃₅, or (26) SOR₆₄;
R₁₈ and R₁₉, R₃₁ and R₃₄ are each independently, hydrogen atom, or C₁₋₈ alkyl;
R₂₀ and R₂₁ are C₁₋₈ alkyl, C₁₋₈ alkoxy, hydroxy, halogen atom, nitro, or COOR₃₆;
R₂₂ and R₆₄ are each independently C₁₋₈ alkyl;
R₂₃, R₂₄, R₂₅ and R₂₆ are each independently hydrogen atom, C₁₋₈ alkyl, or phenyl;
R₂₇, R₂₈, R₂₉, R₃₀, R₃₂, R₃₃ and R₃₅ are (1) C₁₋₈ alkyl. (2) C₂₋₈ alkenyl, (3) C₁₋₈ alkyl substituted with 1-5 of R₃₇, (4) diphenylmethyl, (5) triphenylmethyl, (6) Cyc3, (7) C₁₋₈ alkyl or C₂₋₈ alkenyl substituted with Cyc3, (8) C₁₋₈ alkyl substituted with O-Cyc3, S-Cyc3 or SO₂-Cyc3;
R₃₆ is hydrogen atom, or C₁₋₈ alkyl;
R₃₇ is C₁₋₈ alkoxy, C₁₋₈ alkylthio, benzyloxy, halogen atom, nitro or COOR₃₈;
R₃₈ is hydrogen atom, C₁₋₈ alkyl or C₂₋₈ alkenyl;
Cyc3 is (1) C₃₋₁₅ mono-, bi- or tri-cyclic (fused or spiro)carboring, or (2) 3-15 membered mono-, bi- or tri-cyclic (fused or spiro)heteroring comprising 1-4 of heteroatom selected from oxygen, nitrogen and sulfur;
Cyc3 may be substituted with 1-5 of R₃₉;
R₃₉ is (1) C₁₋₈ alkyl, (2) C₂₋₈ alkenyl, (3) C₂₋₈ alkynyl, (4) C₁₋₈ alkoxy, (5) C₁₋₈ alkylthio, (6) hydroxy, (7) halogen atom, (8) nitro, (9) oxo, (10) cyano, (11) benzyl, (12) benzyloxy, (13) C₁₋₈ alkyl, C₁₋₈ alkoxy or C₁₋₈ alkylthio substituted with 1-5 of R₄₀, (14) phenyl, phenoxy, phenylthio, phenylsulfonyl or benzoyl which may be substituted with 1-5 of R₄₁, (15) OCOR₄₂, (16) SO₂R₄₃, (17) NR₄₄COR₄₅, (18) SO₂NR₄₆R₄₇, (19) COOR₄₈, or (20) NR₄₉R₅₀;
R₄₀ is halogen atom;
R₄₁ is C₁₋₈ alkyl, C₁₋₈ alkoxy, halogen atom, or nitro;
R₄₂, R₄₃ and R₄₅ are C₁₋₈ alkyl;
R₄₄ and R₄₈ are hydrogen atom or C₁₋₈ alkyl;
R₄₆ and R₄₇, R₄₉ and R₅₀ are each independently, hydrogen atom or C₁₋₈ alkyl;
R_{17'} is (1) SH, (2) NR₆₆CHO, (3) Cyc5, (4) C₁₋₈ alkyl, C₂₋₈ alkenyl or C₂₋₈ alkynyl substituted with Cyc5, (5) CO-(NH-amino acid residue-CO)n-OH, (6) NR₆₇CONR₆₈R₆₉, (7) CONR₇₀NR₇₁R₇₂, (8) CONR₇₃OR₇₄, (9) CONR₇₅COR₇₆, (10) C(S)NR₇₇R₇₈, (11) CONR₇₉C(S)COOR₈₀, (12) NR₈₁COCOOR₈₂, (13) NR₈₃COOR₈₄, (14) CONR₈₅C(S)R₈₆, (15) OCOR₈₇, (16) SOR₈₈, (17) CONR₉₉R₉₀, (18) SO₂NR₉₁R₉₂, (19) COOR₉₃, (20) COCOOR₉₄, (21) COR₉₅, (22) COCOR₉₆, (23) NR₉₇COR₉₈, (24) SO₂R₉₉, (25) NR₁₀₀SO₂R₁₀₁, or (26) NR₁₀₂R₁₀₃;
n is an integer of 1 or 2;
R₆₆, R₇₃, R₇₅, R₇₇, R₇₉, R₈₁, R₈₃, R₈₅, R₉₇, R₁₀₀ and R₁₀₂ are hydrogen atom, or C₁₋₈ alkyl;
R₆₇ and R₆₈, R₇₀ and R₇₁ are each independently, hydrogen atom, or C₁₋₈ alkyl; R₈₉ and R₉₁ are (1) hydrogen atom, (2) C₁₋₈ alkyl, (3) phenyl, or (4) C₁₋₈ alkyl substituted with cyano or C₁₋₈ alkoxy;
R₁₀₃ is Cyc6;
R₆₉, R₇₂, R₇₄, R₇₆, R₇₈, R₈₀, R₈₂, R₈₄, R₈₆, R₈₇, R₈₈, R₉₀ and R₉₂ are (1) hydrogen atom, (2) C₁₋₈ alkyl, (3) C₂₋₈ alkenyl, (4) C₂₋₈ alkynyl, (5) C₁₋₈ alkyl substituted with 1-5 of R₁₀₄, (6) diphenylmethyl, (7) triphenylmethyl, (8) Cyc6, (9) C₁₋₈ alkyl or C₂₋₈ alkenyl substituted with Cyc6, or (10) C₁₋₈ alkyl substituted with O-Cyc6, S-Cyc6 or SO₂-Cyc6;
R₁₀₄ is (1) C₁₋₈ alkoxy, (2) C₁₋₈ alkylthio, (3)benzyloxy, (4) halogen atom, (5) nitro, (6) COOR₁₀₅, (7) cyano, (8) NR₁₀₆R₁₀₇, (9) N₁₀₈COR₁₀₉, (10) hydroxy, (11) SH, (12) SO₃H, (13) S(O)OH, (14) OSO₃H, (15) C₂₋₈ alkenyloxy, (16) C₂₋₈ alkynyloxy, (17) COR₁₁₀, (18) SO₂R₁₁₁, or (19) C₁₋₈ alkoxy or C₁₋₈ alkylthio substituted with hydroxy;
R₁₀₅ is hydrogen atom, C₁₋₈ alkyl, or C₂₋₈ alkenyl;
R₁₀₆ and R₁₀₇ are each independently, hydrogen atom, or C₁₋₈ alkyl;
R₁₀₈ is hydrogen atom, or C₁₋₈ alkyl;
R₁₀₉ and R₁₁₁ are C₁₋₈ alkyl;
R₁₁₀ is C₁₋₈ alkyl, or halogen atom;
R₉₃, R₉₄, R₉₅, R₉₆, R₉₈, R₉₉ and R₁₀₁ are (1) C₂₋₈ alkynyl, (2) C₁₋₈ alkyl substituted with R₁₂₈ which may be substituted with 1-4 of R₂₉, (3) Cyc8, (4) C₁₋₈ alkyl or C₂₋₈ alkenyl substituted with Cyc8, or (5) C₁₋₈ alkyl substituted with O-Cyc8, S-Cyc8 or SO₂-Cyc8; R₁₂₈ is (1) cyano, (2) NR₁₀₆R₁₀₇, (3) NR₁₀₈COR₁₀₉, (4) hydroxy, (5) SH, (6) SO₃H, (7) S(O)OH, (8) OSO₃H, (9) C₂₋₈ alkenyloxy, (10) C₂₋₈ alkynyloxy, (11) COR₁₁₀, (12) SO₂R₁₁₁, or (13) C₁₋₈ alkoxy or C₁₋₈ alkylthio substituted with hydroxy;
R₁₂₉ has the same meaning as R₁₀₄;
Cyc5 and Cyc6 may be substituted with 1-5 of R₁₁₂;
R₁₁₂ is (1) C₁₋₈ alkyl, (2) C₂₋₈ alkenyl, (3) C₂₋₈ alkynyl, (4) C₁₋₈ alkoxy, (5) C₁₋₈ alkylthio, (6) hydroxy, (7) halogen atom, (8) nitro, (9) oxo, (10) cyano, (11) benzyl, (12) benzyloxy, (13) C₁₋₈ alkyl, C₁₋₈ alkoxy or C₁₋₈ alkylthio substituted with 1-5 of R₁₁₃, (14) phenyl, phenoxy, phenylthio or benzoyl, which may be substituted with 1-5 of R₁₁₄, (15) COR₁₁₅, (16) SO₂R₁₁₆, (17) NR₁₁₇COR₁₁₈, (18) SO₂NR₁₁₉R₁₂₀, (19) COOR₁₂₁, (20) NR₁₂₂R₁₂₃, (21) COR₁₂₄, (22) CONR₁₂₅R₁₂₆, (23) SH, (24) C₁₋₈ alkyl substituted with hydroxy or NR₁₂₇-benzoyl, or (25) Cyc7;
R₁₁₃ is halogen atom;
R₁₁₄ is C₁₋₈ alkyl, C₁₋₈ alkoxy, halogen atom, or nitro;
R₁₁₅, R₁₁₆ and R₁₁₈ are C₁₋₈ alkyl;
R₁₁₇, R₁₂₁, R₁₂₄ and R₁₂₇ are hydrogen atom, or C₁₋₈ alkyl;
R₁₁₉ and R₁₂₀, R₁₂₂ and R₁₂₃, R₁₂₅ and R₁₂₆ are each independently, hydrogen atom or C₁₋₈ alkyl;
Cyc7 may be substituted with 1-5 group selected from (1) C₁₋₈ alkyl, (2) C₁₋₈ alkoxy, (3) halogen atom, or (4) nitro;
Cyc8 may be substituted with R₁₃₀, and it further may be substituted with 1-4 of R₁₃₁;
R₁₃₀ is (1) COR₁₂₄, (2) CONR₁₂₅R₁₂₆, (3) SH, (4) C₁₋₈ alkyl substituted with hydroxy or NR₁₂₇-benzoyl, or (5) Cyc7;
R₁₃₁ has the same meaning as R₁₁₂;
Cyc5, Cyc6, Cyc7 and Cyc8 are (1) C₃₋₁₅ mono-, bi- or tri-cyclic (fused or spiro)carboring, or (2) 3-15 membered mono-, bi- or tri-cyclic (fused or spiro)heteroring comprising 1-4 of heteroatom selected from 1-4 of oxygen, nitrogen or sulfur;
wherein when R_{17'} is Cyc5, Cyc5 is not phenyl which may be substituted with 1-5 selected from C₁₋₈ alkyl, C₁₋₈ alkoxy, hydroxy, halogen atom, nitro, COOH, or COO(C₁₋₈ alkyl);
wherein Cyc7 is not phenyl;
Cyc4 is (1) C₅₋₇ monocyclic carboring, or (2) 5-7 membered monocyclic heteroring comprising 1-2 of heteroatom selected from oxygen, nitrogen and sulfur; (abbreviated as dashed line a hereafter;) and (abbreviated as dashed line b hereafter;) are (1) a bond, or (2) a double bond;
R₉ (1) absent or (2) is hydrogen atom;
   wherein
   (1) when dashed line a is a bond, dashed line b is a double bond, and R₉ is absent,
   (2) when dashed line a is a double bond, dashed line b is a bond, and R₉ is hydrogen atom and R₆ is absent, and
   (3) 2-(3,3-dimethyl-3,4-dihydro-(2H)-isoquinolin-1-ylidene)-1-phenylethan-1-one is excluded, or a pharmacologically acceptable salt thereof.

The preparation of these compounds is described in US 2005222138 and WO 2003/064389.

In another case, PDE7 inhibitors useful in the methods described herein are selected from those compounds generally or specifically disclosed in WO 2003/057149. In one case, PDE7 inhibitors useful in the methods described herein have the formula:

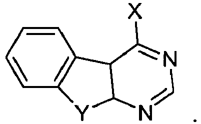

The substituents for the above compounds are defined as follows:
(1) X is selected from halogen and NR₁R₂,
(2) Y is selected from NR₃, S, and O, with the proviso that Y is not S when X is Cl,
(3) R₁ and R₂ are independently selected from hydrogen, alkyl of 1-8 carbon atoms, alkenyl of 2-8 carbon atoms, alkynyl of 2-8 carbon atoms, cycloalkyl of 3-7 carbon atoms, polycycloalkyl of 5-9 carbon atoms, heterocycloalkyl of 2-6 carbon atoms and 1-2 heteroatoms selected from NH, S, and O, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms, or heteroaryl of 4-11 carbon atoms and 1-2 heteroatoms selected from N, S, and O, heteroaryl of 4-11 carbon atoms and 1-2 heteroatoms selected from N, S and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms, or heteroaryl of 4-11 carbon atoms and 1-2 heteroatoms selected from N, S, and O, and R₄R₅, or R₁ and R₂ combine to form, together with the nitrogen atom to which they are attached, a 5-7 membered monocyclic saturated ring, which optionally contains 1-2 additional heteroatoms selected from the group consisting of NH, NR₆, S, and O, or combine to form, together with the nitrogen atom to which they are attached, a 6-10 membered fused polycyclic saturated ring, which optionally contains 1-2 additional heteroatoms selected from the group consisting of NH, NR₆, S, and O, or combine to form, together with the nitrogen atom to which they are attached, a 5-7 membered unsaturated ring, which optionally contains 1-2 additional heteroatoms selected from the group consisting of N, S, and O, wherein said monocyclic saturated ring, polycyclic saturated ring or unsaturated ring may be substituted with 1-2 substituents selected from the group consisting of OH, alkyl of 1-6 carbon atoms, alkenyl of 2-6 carbon atoms, alkynyl of 2-6 carbon atoms, cycloalkyl of 3-7 carbon atoms, heterocycloalkyl of 2-6 carbon atoms and 1-2 heteroatoms selected from NH, S, and O, halogen, haloalkyl of 1-2 carbon atoms and a number of halogen atoms up to the perhalo level, alkoxy of 1-6 carbon atoms, haloalkoxy of 1-6 carbon atoms and a number of halogen atoms up to the perhalo level, and R₇R₈,
(4) R₃ is selected from hydrogen, alkyl of 1-8 carbon atoms, alkenyl of 2-8 carbon atoms, alkynyl of 2-8 carbon atoms, cycloalkyl of 3-7 carbon atoms, and heteroaryl of 4-11 carbon atoms and 1-2 heteroatoms selected from N, S, and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atom sup to the perhalo level, haloalkoxy of 1-6 carbon atoms and a number of halogen atoms up to the perhalo level, aryl of 6-12 carbon atoms, or heteroaryl of 4-11 carbon atoms and 1-2 heteroatoms selected from N, S, and O,
(5) R₄ is selected from alkyl of 1-8 carbon atoms, alkenyl of 2-8 carbon atoms, alkynyl of 2-8 carbon atoms, C(=O), S(=O)₂, and C(=O)O,
(6) R₅ is selected from hydrogen, OH, alkyl of 1-8 carbon atoms, alkenyl of 2-8 carbon atoms, alkynyl of 2-8 carbon atoms, alkoxy of 1-8 carbon atoms, thioxy of 1-8 carbon atoms, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms, or heteroaryl of 4-11 carbon atoms and 1-2 heteroatoms selected from N, S, and O, heteroaryl of 4-11 carbon atoms and 1-2 heteroatoms selected from N, S, and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms, or heteroaryl of 4-11 carbon atoms and 1-2 heteroatoms selected from N, S, and O, cycloalkyl of 3-7 carbon atoms, heterocycloalkyl of 2-6 carbon atoms and 1-2 heteroatoms selected from NH, S, and O, and NR₉R₁₀,
(7) R₆ and R₇ are independently selected from alkyl of 1-8 carbon atoms, alkenyl of 2-8 carbon atoms, and alkynyl of 2-8 carbon atoms,
(8) R₈ is selected from OH, aryl of 6-12 carbon atoms, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, haloalkoxy of 1-6 carbon atoms and a number of halogen atoms up to the perhalo level, aryl of 6-12 carbon atoms or heteroaryl of 4-11 carbon atoms and 1-2 heteroatoms selected from N, S, and O, and heteroaryl of 4-11 carbon atoms and 1-2 heteroatoms selected from N, S, and O, which may be substituted with alkyl of 1-6 carbon atoms, alkenyl of 2-6 carbon atoms, alkynyl of 2-6 carbon atoms, alkoxy of 1-6 carbon atoms, halogen, haloalkyl of 1-6 carbon atoms and a number of halogen atoms up to the perhalo level, aryl of 6-12 carbon atoms or heteroaryl of 4-11 carbon atoms and 1-2 heteroatoms selected from N, S, and O;
(9) R₉ and R₁₀ are independently selected from hydrogen, alkyl of 1-8 carbon atoms, alkenyl of 2-8 carbon atoms, and alkynyl of 2-8 carbon atoms, or R₉ and R₁₀ combine together with the nitrogen atom to which they are attached to form a 5-7 membered, unsaturated ring which may contain 1-2 additional heteroatoms selected from N, S, and O, or to form a 5-7 membered, saturated ring which may contain 1-2 additional heteroatoms selected from NH, NR₁₁, S, and O;
(10) R₁ is selected from alkyl of 1-8 carbon atoms, alkenyl of 2-8 carbon atoms, and alkynyl of 2-8 carbon atoms, and pharmaceutically acceptable salts thereof.

The preparation of these compounds is described in WO 2003/057149.

In another case, PDE7 inhibitors useful in the methods described herein are selected from those compounds generally or specifically disclosed in US 20030092721, U.S. Patent No. 7,022,849, WO 2002/102315, and US 2006116516. In one case, PDE7 inhibitors useful in the methods described herein have the formula:

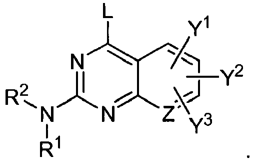

The substituents for the above compounds are defined as follows:
R₁ is H or alkyl;
R₂ is (a) heteroaryl or heterocyclo, either of which may be optionally substituted with one to three groups T1, T2, T3; or (b) aryl fused to a heteroaryl or heterocyclo ring wherein the combined ring system may be optionally substituted with one to three groups T1, T2, T3;
L is (a) OR₄, C(O)R₄, C(O)OR₄, SR₄, NR₃R₄, C(O)NR₃R₄, NR₃SO₂R_{4b}, halogen, nitro, or haloalkyl; or (b) alkyl, aryl, heteroaryl, heterocyclo, or cycloalkyl any of which may be optionally substituted with one to three groups T1a, T2a and/or T3a;
Y₁, Y₂ and Y₃ are independently (a) hydrogen, halo, or -OR₄ₐ; or (b) alkyl, alkenyl, or alkynyl, any of which may be optionally substituted with one to three groups T1b, T2b and/or T3b;
R₃ and R₄ are independently H, alkyl, alkenyl, aryl, (aryl) alkyl, heteroaryl, (heteroaryl) alkyl, cycloalkyl, (cycloalkyl) alkyl, heterocyclo, or (heterocyclo) alkyl, any of which may be optionally substituted with one to three groups T1a, T2a and/or T3a; or
R₃ and R₄ together with the nitrogen atom to which they are attached may combine to form a 4- to 8-membered heterocyclo ring optionally substituted with one to three groups T1a, T2a and/or T3a;
R₄ₐ is hydrogen, alkyl, alkenyl, aryl, heteroaryl, (aryl) alkyl, (heteroaryl) alkyl, heterocyclo, (heterocyclo) alkyl, cycloalkyl, or (cycloalkyl) alkyl, any of which may be optionally substituted with one to three groups T1b, T2b and/or T3b;
R_{4b} is alkyl, alkenyl, aryl, (aryl) alkyl, heteroaryl, (heteroaryl) alkyl, cycloalkyl, (cycloalkyl) alkyl, heterocyclo, or (heterocyclo) alkyl, any of which may be optionally substituted with one to three groups T1a, T2a and/or T3a;
Z is N or CH;
T1-1b, T2-2b, and T3-3b are each independently;
   (1) hydrogen or T6, where T6 is (i) alkyl, (hydroxy)alkyl, (alkoxy)alkyl, alkenyl, alkynyl, cycloalkyl, (cycloalkyl)alkyl, cycloalkenyl, (cycloalkenyl)alkyl, aryl, (aryl)alkyl, heterocyclo, (heterocyclo)alkyl, heteroaryl, or (heteroaryl)alkyl; (ii) a group (i) which is itself substituted by one or more of the same or different groups (i); or (iii) a group (i) or (ii) which is independently substituted by one or more of the following groups (2) to (13) of the definition of T1-1b, T2-2b and T3-3b;
   (2) -OH or -OT6;
   (3) -SH or -ST6;
   (4) -C(O)ₜH, -C(O)ₜT6, or -O-C(O)T6, where t is 1 or 2;
   (5) -SO₃H, -S(O)ₜT6, or S(O)ₜN(T9)T6;
   (6) halo;
   (7) cyano;
   (8) nitro;
   (9) -T4-NT7T8;
   (10) -T4-N(T9)-T5-NT7T8;
   (11) -T4-N(T10)-T5-T6;
   (12) -T4-N(T10)-T5-H; and
   (13) oxo;
T4 and T5 are each independently a single bond, T11S(O)ₜT12-, T11C(O)T12-, T11C(S)T12, T11OT12, T11ST12, T11OC(O)T12, T11C(O)OT12, T11C(=NT9a)T12, or T11C(O)C(O)T12;
T7, T8, T9, T9a and T10 are:
   (1) each independently hydrogen or a group provided in the definition of T6, or
   (2) T7 and T8 may together be alkylene or alkenylene, completing a 3- to 8-membered saturated or unsaturated ring together with the atoms to which they are attached, which ring is unsubstituted or substituted with one or more groups listed in the description of T1-1b, T2-2b and T3-3b, or
   (3) T7 or T8, together with T9, may be alkylene or alkenylene completing a 3-to 8-membered saturated or unsaturated ring together with the nitrogen atoms to which they are attached, which ring is unsubstituted or substituted with one or more groups listed in the description of T1-1b, T2-2b and T3-3b, or
   (4) T7 and T8 or T9 and T10 together with the nitrogen atom to which they are attached may combine to form a group N=CT13T14 where T13 and T14 are each independently H or a group provided in the definition of T6; and T11 and T12 are each independently a single bond, alkylene, alkenylene, or alkynylene.

The preparation of these compounds is described in US 20030092721, U.S. Patent No. 7,022,849, WO 2002/102315, and US 2006116516.

In another case, PDE7 inhibitors useful in the methods described herein are selected from those compounds generally or specifically disclosed in U.S. Patent No. 6,838,559, U.S. 20030100571, and WO 2002/102314. In one case, PDE7 inhibitors useful in the methods described herein have the formulas:

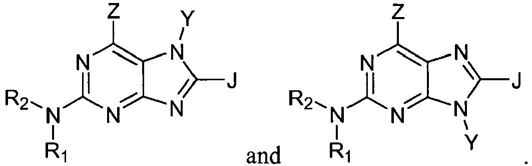

The substituents for the above compounds are defined as follows:
R₁ is H or alkyl;
R₂ is (a) heteroaryl, or heterocyclo, either of which may be optionally substituted with one to three groups T1, T2, T3; (b) aryl substituted with one to three groups T1, T2, T3 provided that at least one of T1, T2, T3 is other than H; or (c) aryl fused to a heteroaryl or heterocyclo ring wherein the combined ring system may be optionally substituted with one to three groups T1, T2, T3;
Y is alkyl, alkenyl, alkynyl, cycloalkyl, aryl, heterocyclo, heteroaryl, (aryl)alkyl or (heteroaryl) alkyl any of which may be optionally substituted with one to three groups T1a, T2a, T3a;
J is (a) hydrogen, halo, or OR₄, or (b) alkyl, alkenyl, alkynyl, aryl, heteroaryl, heterocyclo, or, cycloalkyl any of which may be optionally substituted with one to three groups T1b, T2b, T3b;
Z is (a) OR₄, SR₄, NR₃R₄, NR₃SO₂R₄ₐ halogen, nitro, haloalkyl; or (b) alkyl, aryl, heteroaryl, heterocyclo, or cycloalkyl any of which may be optionally substituted with one to three groups T1c, T2c, T3c;
R₃ is H, alkyl, alkenyl, aryl, (aryl)alkyl, heteroaryl, (heteroaryl)alkyl, cycloalkyl, (cycloalkyl)alkyl, heterocyclo or (heterocyclo)alkyl any of which may be optionally independently substituted where valance allows with one to three groups T1c, T2c, T3c;
R₄ is alkyl, alkenyl, aryl, (aryl)alkyl, heteroaryl, (heteroaryl)alkyl, cycloalkyl, (cycloalkyl)alkyl, heterocyclo or (heterocyclo)alkyl any of which may be optionally independently substituted where valance allows with one to three groups T1d, T2d, or T3d; or
R₃ and R₄ together with the nitrogen atom to which they are attached may combine to form a 4 to 8 membered heterocyclo ring optionally substituted with one to three groups T1c, T2c, or T3c;
R₄ₐ is hydrogen, alkyl, alkenyl, aryl, heteroaryl, (aryl)alkyl, (heteroaryl)alkyl, heterocyclo, (heterocyclo)alkyl, cycloalkyl or (cycloalkyl)alkyl any of which may be optionally substituted with one to three groups T1d, T2d or T3d;
T1, T1a, T1b, T1c, T1d, T2, T2a, T2b, T2c, T2d, T3, T3a, T3b, T3c, and T3d (hereinafter abbreviated as T1-1d, T2-2d, and T3-3d) are independently
   (1) hydrogen or T6, where T6 is
      (a) alkyl, (hydroxy) alkyl, (alkoxy) alkyl, alkenyl, alkynyl, cycloalkyl, (cycloalkyl) alkyl, cycloalkenyl, (cycloalkenyl) alkyl, aryl, (aryl) alkyl, heterocyclo, (heterocyclo) alkyl, heteroaryl, or (heteroaryl) alkyl;
      (b) a group (a) which is itself substituted by one or more of the same or different groups (a); or
      (c) a group (a) or (b) which is independently substituted by one or more (preferably 1 to 3) of the following groups (2) to (13) of the definition of T1-1d, T2-2d and T3-3d,
   (2) OH or OT6,
   (3) SH or ST6,
   (4) C(O)t H, C(O)t T6, or OC(O)T6, where t is 1 or 2;
   (5) SO3 H, S(O)t T6, or S(O)t N(T9)T6,
   (6) halo,
   (7) cyano,
   (8) nitro,
   (9) T4NT7 T8,
   (10) T4N(T9)-T5NT7T8,
   (11) T4N(T10)-T5-T6,
   (12) T4N(T10)-T5H,
   (13) oxo,
T4 and T5 are each independently a single bond, T11-S(O)ₜ-T12, T11-C(O)-T12, T11-C(S)-T12, T11-O-T12, -T11S-T12, -T11OC(O)-T12, -T11-C(O)O-T12, -T11C(=NT9a)-T12, or T11-C(O)-C(O)-T12;
T7, T8, T9, T9a and T10 are
   (1) each independently hydrogen or a group provided in the definition of T6, or
   (2) T7 and T8 may together be alkylene or alkenylene, completing a 3- to 8-membered saturated or unsaturated ring together with the atoms to which they are attached, which ring is unsubstituted or substituted with one or more groups listed in the description of T1-1d, T2-2d and T3-3d, or
   (3) T7 or T8, together with T9, may be alkylene or alkenylene completing a 3- to 8-membered saturated or unsaturated ring together with the nitrogen atoms to which they are attached, which ring is unsubstituted or substituted with one or more groups listed in the description of T1-1d, T2-2d and T3-3d, or
   (4) T7 and T8 or T9 and T10 together with the nitrogen atom to which they are attached may combine to form a group N=CT13 T14 where
T13 and T14 are each independently H or a group provided in the definition of T6; and
T11 and T12 are each independently a single bond, alkylene, alkenylene, or alkynylene.

The preparation of these compounds is described in U.S. Patent No. 6,838,559, U.S. 20030100571, and WO 2002/102314.

In another case, PDE7 inhibitors useful in the methods described herein are selected from those compounds generally or specifically disclosed in U.S. Patent No. 7,087,614, U.S. 20030162802, and WO 2002/192313. In one case, PDE7 inhibitors useful in the methods described herein have the formula:

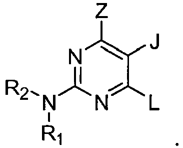

The substituents for the above compounds are defined as follows:
R₁ₐ is hydrogen or alkyl; R₂ₐ is 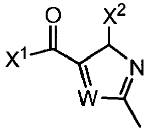 W is S; X₁ is alkoxy; and X₂ is alkyl;
Z is halogen, haloalkyl, oxazolyl, NR₃ₐR₄ₐ, C(O)-N(H)-alkylene-COOH, or phenyl which is unsubstituted or substituted with heteroaryl, COₜH, or COₜT₆;
R₃ₐ is hydrogen or alkyl;
R₄ₐ is alkyl, alkoxy, unsubstituted or substituted (heteroaryl) alkyl, unsubstituted or substituted heterocyclo, unsubstituted or substituted (heterocyclo) alkyl, or (aryl) alkyl wherein the aryl group is substituted with one or two groups T1 and/or T2 and/or further substituted with a group T3; or R₃ₐ and R₄ₐ together with the nitrogen atom to which they are attached combine to form an unsubstituted or substituted heterocyclo ring;
R₅ₐ is an unsubstituted or substituted (heteroaryl) alkyl, or (aryl) alkyl wherein the aryl group is substituted with one or two groups T1 and/or T2 and/or further substituted with a group T3; or R₅ₐ and R₆ₐ together with the nitrogen atom to which they are attached combine to form an unsubstituted or substituted heterocyclo ring; R₆ₐ is hydrogen or alkyl; J is hydrogen or alkyl; T1 and T2 are independently alkoxy, alkoxycarbonyl, heteroaryl, SO₃H, or SO₂R₈ₐ where R₈ₐ is alkyl, amino, alkylamino or dialkylamino; or T1 and T2 together with the aryl ring to which they are attached combine to form a bicyclic ring; T3 is H, alkyl, halo, haloalkyl, or cyano; t is 1 or 2; and T6 is alkyl, haloalkyl, cycloalkyl, alkoxy, or heteroaryl.

The preparation of these compounds is described in U.S. Patent No. 7,087,614, U.S. 20030162802, and WO 2002/192313.

In another case, PDE7 inhibitors useful in the methods described herein are selected from those compounds generally or specifically disclosed in US 20030104974, WO 2002/088080, and WO 2002/088079. In one case, PDE7 inhibitors useful in the methods described herein have the formulas:

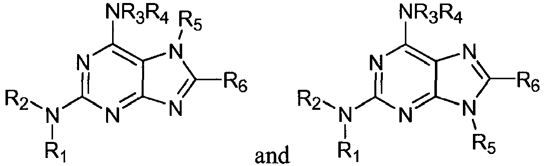

The substituents for the above compounds are defined as follows:
R₁ is H or alkyl; R₂ is optionally substituted heteroaryl, or 4-substituted aryl; R₃ is hydrogen or alkyl; R₄ is alkyl, optionally substituted (aryl)alkyl, optionally substituted (heteroaryl)alkyl, optionally substituted heterocyclo, or optionally substituted (heterocyclo)alkyl; or R₃ and R₄ together with the nitrogen atom to which they are attached may combine to form an optionally substituted heterocyclo ring; R₅ is alkyl, optionally substituted (aryl)alkyl, or optionally substituted (heteroaryl)alkyl; and R₆ is hydrogen or alkyl.

In a related case, PDE7 inhibitors useful in the methods described herein have the formula: 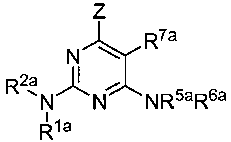 wherein R₁ₐ is H or alkyl; R₂ₐ is optionally substituted heteroaryl; Z is halogen, alkyl, substituted alkyl, haloalkyl, or NR₃ₐR₄ₐ; R₃ₐ is hydrogen or alkyl; R₄ₐ is alkyl, optionally substituted (heteroaryl) alkyl, optionally substituted heterocyclo, optionally substituted (heterocyclo) alkyl, or (aryl) alkyl wherein the aryl group is substituted with one or two groups T1 and T2 and optionally further substituted with a group T3; or R₃ₐ and R₄ₐ together with the nitrogen atom to which they are attached may combine to form an optionally substituted heterocyclo ring; R₅ₐ is (aryl) alkyl wherein the aryl group is substituted with one or two groups T1 and T2 and optionally further substituted with a group T3; R₆ₐ is hydrogen or alkyl; R₇ₐ is hydrogen or alkyl; T1 and T2 are independently alkoxy, alkoxycarbonyl, heteroaryl or SO₂R₈ₐ where R₈ₐ is alkyl, amino, alkylamino or dialkylamino; or T1 and T2 together with the atoms to which they are attached may combine to form a ring (e.g., benzodioxole); T3 is H, alkyl, halo, haloalkyl or cyano.

In another related case, PDE7 inhibitors useful in the methods described herein have the formula: 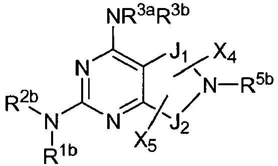 wherein R_{1b} is H or alkyl; R_{2b} is optionally substituted heteroaryl; R_{3b} is H or alkyl; R_{4b} is optionally substituted (aryl)alkyl; R_{5b} is H, alkyl, or C(O)(CH₂)ᵥOYR_{6b}, where Y is a bond or C(O), R_{6b} is hydrogen or alkyl, and v is an integer from 0 to 2; J₁ and J₂ are independently optionally substituted C₁₋₁₃ alkylene, provided that J₁ and J₂ are not both greater than C₂ alkylene; X₄ and X₅ are optional substituents bonded to any available carbon atom in one or both of J₁ and J₂, independently selected from hydrogen, OR₇, NR₈R₉, alkyl, substituted alkyl, alkenyl, substituted alkenyl, alkynyl, substituted alkynyl, cycloalkyl, substituted cycloalkyl, aryl, substituted aryl, heterocycloalkyl, or heteroaryl; R7 is hydrogen, alkyl, substituted alkyl, alkenyl, alkynyl, cycloalkyl, substituted cycloalkyl, C(O)alkyl, C(O)substituted alkyl, C(O)cycloalkyl, C(O) substituted cycloalkyl, C(O)aryl, C(O)substituted aryl, C(O)O-alkyl, C(O)O-substituted alkyl, C(O)heterocycloalkyl, C(O)heteroaryl, aryl, substituted aryl, heterocycloalkyl and heteroaryl; and R₈ and R₉ are independently selected from the group consisting of hydrogen, alkyl, substituted alkyl, cycloalkyl, substituted cycloalkyl, alkenyl, alkynyl, C(O) alkyl, C(O) substituted alkyl, C(O) cycloalkyl, C(O)substituted cycloalkyl, C(O)aryl, C(O)substituted aryl, C(O)O alkyl, C(O)O substituted alkyl, C(O) heterocycloalkyl, C(O) heteroaryl, S(O)₂alkyl, S(O)₂ substituted alkyl, S(O)₂ cycloalkyl, S(O)₂ substituted cycloalkyl, S(O)₂aryl, S(O)₂substituted aryl, S(O)₂ heterocycloalkyl, S(O)₂ heteroaryl, aryl, substituted aryl, heterocycloalkyl, and heteroaryl, or R₈ and R₉ taken together with the nitrogen atom to which they are attached complete an optionally substituted heterocycloalkyl or heteroaryl ring.

In a further related case, PDE7 inhibitors useful in the methods described herein have the formula: 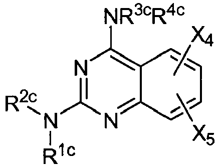 wherein R_{1c} is H or alkyl; R_{2c} is optionally substituted heteroaryl; R_{3c} is H or alkyl; R_{4c} is optionally substituted (aryl)alkyl; and X₄ and X₅ are optional substituents bonded to any available carbon atom in one or both of J₁ and J₂, independently selected from hydrogen, OR₇, NR₈R₉, alkyl, substituted alkyl, alkenyl, substituted alkenyl, alkynyl, substituted alkynyl, cycloalkyl, substituted cycloalkyl, aryl, substituted aryl, heterocycloalkyl, or heteroaryl.

The preparation of these compounds is described in US 20030104974, WO 2002/088080, and WO 2002/088079.

In another case, PDE7 inhibitors useful in the methods described herein are selected from those compounds generally or specifically disclosed in US 20030092908 and WO 2002/087513. In one case, PDE7 inhibitors useful in the methods described herein have the formula:

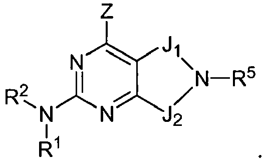

The substituents for the above compounds are defined as follows:
R₁ is hydrogen or alkyl;
R₂ is (a) heteroaryl, or heterocyclo, either of which may be optionally substituted with one to three groups T1, T2, T3; (b) aryl substituted with one to three groups T1, T2, T3 provided that at least one of T1, T2, T3 is other than H; or (c) aryl fused to a heteroaryl or heterocyclo ring wherein the combined ring system may be optionally substituted with one to three groups T1, T2, T3;
Z is NR₃R₄, NR₃SO₂R₄ₐ, OR₄, SR₄, haloalkyl, or halogen;
R₃ and R₄ are independently H, alkyl, alkenyl, aryl, (aryl)alkyl, heteroaryl, (heteroaryl)alkyl, cycloalkyl, (cycloalkyl)alkyl, heterocyclo or (heterocyclo)alkyl any of which may be optionally independently substituted where valance allows with one to three groups T1a, T2a, or T3a; or
R₃ and R₄ may be taken together with the nitrogen atom to which they are attached to form a heterocyclo or heteroaryl ring optionally independently substituted where valance allows with one to three groups T1a, T2a, or T3a;
R₄ₐ is alkyl, alkenyl, aryl, (aryl)alkyl, heteroaryl, (heteroaryl)alkyl, cycloalkyl, (cycloalkyl)alkyl, heterocyclo or (heterocyclo)alkyl any of which may be optionally independently substituted where valance allows with one to three groups T1a, T2a, or T3a;
R_{3b} and R_{4b} are independently H, alkyl, alkenyl, aryl, (aryl)alkyl, heteroaryl, (heteroaryl)alkyl, cycloalkyl, (cycloalkyl)alkyl, heterocyclo or (heterocyclo)alkyl;
R₅ is
   (1) hydrogen, or cyano;
   (2) alkyl, alkenyl, alkynyl, cycloalkyl, (cycloalkyl)alkyl, aryl, (aryl)alkyl, heterocyclo, (heterocyclo)alkyl, heteroaryl or (heteroaryl)alkyl, any of which may be optionally independently substituted where valance allows with one to three groups T1b, T2b, or T3b; or
   (3) C(O)R₆, C(O)OR₆, C(O)-C(O)OR, or SO₂R₆ₐ;
R₆ is H, alkyl, alkenyl, NR_{3b}R_{4b}, heterocyclo, (heterocyclo)alkyl, (hydroxy)alkyl, (alkoxy)alkyl, (aryloxy)alkyl, (NR_{3b}R_{4b})alkyl, heteroaryl, aryl or (aryl)alkyl, any of which may be optionally independently substituted where valance allows with one to three groups T1b, T2b, or T3b;
R₆ₐ is alkyl, alkenyl, NR_{3b}R_{4b}, heterocyclo, (heterocyclo)alkyl, (hydroxy)alkyl, (alkoxy)alkyl, (aryloxy)alkyl, (NR_{3b}R_{4b})alkyl, heteroaryl, aryl or (aryl)alkyl, any of which may be optionally independently substituted where valance allows with one to three groups T1b, T2b, or T3b;
J₁ and J₂ are independently optionally substituted C₁₋₃ alkylene, provided that J₁ and J₂ are not both greater than C₂ alkylene; and
T1-1b, T2-2b, and T3-3b are each independently
   (1) hydrogen or T6, where T6 is (i) alkyl, (hydroxy)alkyl, (alkoxy)alkyl, alkenyl, alkynyl, cycloalkyl, (cycloalkyl)alkyl, cycloalkenyl, (cycloalkenyl)alkyl, aryl, (aryl) alkyl, heterocyclo, (heterocyclo)alkyl, heteroaryl, or (heteroaryl)alkyl; (ii) a group (i) which is itself substituted by one or more of the same or different groups (i); or (iii) a group (i) or (ii) which is independently substituted by one or more (preferably 1 to 3) of the following groups (2) to (13) of the definition of T1-1b, T2-2b, and T3-3b,
   (2) OH or OT6,
   (3) SH or ST6,
   (4) C(O)tH, C(O)ₜT6, or OC(O)T6, where t is 1 or 2,
   (5) SO₃H, S(O)ₜT6, or S(O)ₜN(T9)T6,
   (6) halo,
   (7) cyano,
   (8) nitro,
   (9) T4-NT7T8,
   (10) T4-N(T9)-T5-NT7T8,
   (11) T4-N(T10)-T5-T6,
   (12) T4-N(T10)-T5H,
   (13) oxo,
T4 and T5 are each independently (1) a single bond, (2) T11-S(O)ₜ-T12, (3) T11-C(O)-T12, (4) T11-C(S)-T12, (5) -T11-O-T12, (6) T11-S-T12, (7) T11-O-C(O)-T12, (8) T11-C(O)-O-T12, (9) T11-C(=NT9a)-T12, or (10) T11-C(O)-C(O)-T12,
T7, T8, T9, T9a and T10,
   (1) are each independently hydrogen or a group provided in the definition of T6, or
   (2) T7 and T8 may together be alkylene or alkenylene, completing a 3- to 8-membered saturated or unsaturated ring together with the atoms to which they are attached, which ring is unsubstituted or substituted with one or more groups listed in the description of T1-1b, T2-2b, and T3-3b, or
   (3) T7 or T8, together with T9, may be alkylene or alkenylene completing a 3- to 8-membered saturated or unsaturated ring together with the nitrogen atoms to which they are attached, which ring is unsubstituted or substituted with one or more groups listed in the description of T1-1b, T2-2b, and T3-3b, or
   (4) T7 and T8 or T9 and T10 together with the nitrogen atom to which they are attached may combine to form a group N=CT13T14 where T13 and T14 are each independently H or a group provided in the definition of T6; and
T11 and T12 are each independently a single bond, alkylene, alkenylene, or alkynylene.

The preparation of these compounds is described in US 20030092908 and WO 2002/087513.

In another case, PDE7 inhibitors useful in the methods described herein are selected from those compounds generally or specifically disclosed in US 20040127707 and WO 2002/085906. In one case, PDE7 inhibitors useful in the methods described herein have the formula:

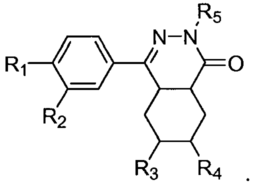

The substituents for the above compounds are defined as follows:
R₁ is 1-2C-alkoxy or 1-2C-alkoxy which is completely or predominantly substituted by fluorine,
R₂ is fluorine, bromine, or chlorine,
R₃ and R₄ are both hydrogen or together form an additional bond,
R₅ is R₆, CₘH₂ₘ-R₇, CₙH₂ₙ-C(O)R₈, CH(R₉)₂, CₚH₂p-Y-Aryl1, R₁₂ or R₂₆, wherein
R₆ 1-8C-alkyl, 3-10C-cycloalkyl, 3-7C-cycloalkylmethyl, 3-7C-alkenyl, 3-7C-alkinyl, phenyl-3-4C-alkenyl, 7-10C-polycycloalkyl, naphthyl, pyridyl, pyrazinyl, pyridazinyl, pyrimidyl, quinazolinyl, quinoxalinyl, cinnolinyl, isoquinolinyl, quinolinyl, indanyl, indazolyl, benzoxazolyl, benzothiazolyl, oxazolyl, thiazolyl, N-methylpiperidyl, tetrahydropyranyl, 6-methyl-3-trifluoromethyl-pyridin-2-yl, 1,3,4-trimethyl-1H-pyrazolo[3,4-b]pyridin-6-yl, 3-thiophen-2-yl[1,2,4]thiadiazol-5-yl, 1,1-dioxide-tetrahydrothiophen-3-y-l, 1-oxo-1,3-dihydro-isobenzofuran-5-yl, 4-(4-yl-but-1-oxy)benzoic acid, or an unsubstituted or by R₆₁ and/or R₅₂ substituted phenyl radical, wherein
R₆₁ is hydroxyl, 1-4C-alkyl, 1-4C-alkoxy, nitro, cyano, halogen, carboxyl, hydroxycarbonyl-1-4C-alkyl, 1-4C-alkoxycarbonyl, hydroxy-1-4C-alkyl, amino, mono- or di-1-4C-alkylamino, 1-4C-alkylcarbonylamino, aminocarbonyl, mono- or di-1-4C-alkylaminocarbon-yl, aminosulfonyl, mono- or di-1-4C-alkylaminosulfonyl, 4-methylphenylsulfonamido, imidazolyl; tetrazol-5-yl, 2-(1-4C-alkyl)tetrazol-5-yl or 2-benzyltetrazol-5-yl and
R₆₂ is 1-4C-alkyl, 1-4C-alkoxy, nitro, or halogen,
R₇ is hydroxyl, halogen, cyano, nitro, nitroxy(O-NO₂), carboxyl, carboxyphenyloxy, phenoxy, 1-4C-alkoxy, 3-7C-cydoalkoxy, 3-7C-cycloalkylmethoxy, 1-4C-alkylcarbonyl, 1-4C-alkylcarbonyloxy, 1-4C-alkylcarbonylamino, 1-4C-alkoxycarbonyl, aminocarbonyl, mono- or di-1-4C-alkylaminocarbonyl, amino, mono- or di-1-4C-alkylamino, or an unsubstituted or by R₇₁ and/or R₇₂ substituted piperidyl, piperazinyl, pyrrolidinyl or morpholinyl radical, wherein
R₁₁ is hydroxyl, 1-4C-alkyl, hydroxy-1-4C-alkyl or 1-4C-alkoxycarbonyl, and
R₇₂ is 1-4C-alkyl, carboxyl, aminocarbonyl or 1-4C-alkoxycarbonyl,
R₈ is an unsubstituted or by R₈₁ and/or R₈₂ substituted phenyl, naphthyl, phenanthrenyl or anthracenyl radical, wherein
R₈₁ is hydroxyl, halogen, cyano, 1-4C-alkyl, 1-4C-alkoxy, carboxyl, aminocarbonyl, mono- or di-1-4C-alkylaminocarbonyl, 1-4C-alkylcarbonyloxy, 1-4C-alkoxycarbonyl, amino, mono- or di-1-4C-alkylamino, 1-4C-alkylcarbonylamino, or 1-4C-alkoxy which is completely or predominantly substituted by fluorine, and
R₈₂ is hydroxyl, halogen, 1-4C-alkyl, 1-4C-alkoxy or 1-4C-alkoxy which is completely or predominantly substituted by fluorine,
R₉ is C_{q}H_{2q}-phenyl,
Y is a bond or O (oxygen),
Aryl₁ is an unsubstituted phenyl, naphthyl, pyridyl, pyrazinyl, pyridazinyl, pyrimidinyl, quinazolinyl, quinoxalinyl, cinnolinyl, isoquinolyl, quinolyl, coumarinyl, benzimidazolyl, benzoxazolyl, benzothiazolyl, benzotriazolyl, N-benzosuccinimidyl, imidazolyl, pyrazolyl, oxazolyl, thiazolyl, furyl, thienyl, pyrrolyl, a 2-(1-4C-alkyl)-thiazol-4-yl radical, or a phenyl radical substituted by R₁₀ and/or R₁₁, wherein
R₁₀ is hydroxyl, halogen, nitro, cyano, 1-4C-alkyl, trifluoromethyl, 1-4C-alkoxy, carboxyl, hydroxycarbonyl-1-4C-alkyl, 1-4C-alkylcarbonyloxy, 1-4C-alkoxycarbonyl, amino, mono- or di-1-4C-alkylamino, 1-4C-alkylcarbonylamino, aminocarbonyl, mono-or di-1-4C-alkylamino-carbonyl, imidazolyl or tetrazolyl, and R₁₁ is hydroxyl, halogen, nitro, 1-4C-alkyl or 1-4C-alkoxy,
m is an integer from 1 to 8, n is an integer from 1 to 4, p is an integer from 1 to 6, q is an integer from 0 to 2,
R₁₂ is a radical of formula (a) 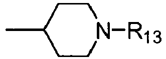 wherein R₁₃ is S(O)₂-R₁₄, S(O)₂-(CH₂)ᵣ-R15, (CH₂)ₛ-S(O)₂R16, C(O)R₁₇, C(O)-(CH₂)ᵣR₁₈, (CH₂)ₛ-C(O)-R₁₉, Hetaryl1, Aryl₂ or Aryl₃-1-4C-alkyl, R₁₄ is 1-4C-alkyl, 5-dimethylaminonaphthalin-1-yl, N(R₂₀)R₂₁, phenyl or phenyl substituted by R₂₂ and/or R₂₃, R₁₅ is N(R₂₀)R₂₁, R₁₆ is N(R₂₀)R₂₁,
R₁₇ is 1-4C-alkyl, hydroxycarbonyl-1-4C-alkyl, phenyl, pyridyl, 4-ethyl-piperazin-2,3-dion-1-yl, 2-oxo-imidazolidin-1-yl or N(R₂₀)R₂₁, R₁₈ is N(R₂₀)R₂₁, R₁₉ is N(R₂₀)R₂₁, phenyl, phenyl substituted by R₂₂ and/or R₂₃ and/or R₂₄, R₂₀ and R₂₁ are independent from each other hydrogen, 1-7C-alkyl, 3-7C-cycloalkyl, 3-7C-cycloalkylmethyl or phenyl, or R₂₀ and R₂₁ together and with inclusion of the nitrogen atom to which they are bonded, form a 4-morpholinyl-ring, 1-pyrrolidinyl-ring, 1-piperidinyl-ring, 1-hexahydroazepino-ring or a 1-piperazinyl-ring of formula (b) 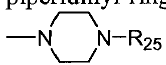 wherein R₂₅ is pyrid-4-yl, pyrid4-ylmethyl, 1-4C-alkyl-dimethylamino, dimethylaminocarbonylmethyl, N-methyl-piperidin-4-yl, 4-morpholino-ethyl or tetrahydrofuran-2-ylmethyl-, R₂₂ is halogen, nitro, cyano, carboxyl, 1-4C-alkyl, trifluoromethyl, 1-4C-alkoxy, 1-4C-alkoxycarbonyl, amino, mono-or di-1 4C-alkyiamino, aminocarbonyl 1-4C-alkylcarbonylamino or mono-or di-1-4C-alkyiaminocarbon- yl, R₂₃ is halogen, amino, nitro, 1-4C-alkyl or 1-4C-alkoxy, R₂₄ is halogen,
Hetaryl₁ is pyrimidin-2-yl, thieno-[2,3-d]pyrimidin-4-yl, 1-methyl-1H-pyrazolo-[3,4-d]pyrimidin-4-yl, thiazolyl, imidazolyl or furanyl, Aryl₂ is pyridyl, phenyl or phenyl substituted by R₂₂ and/or R₂₃, Aryl₃ is pyridyl, phenyl, phenyl substituted by R₂₂ and/or R₂₃, 2-oxo-2H-chromen-7-yl or 4-(1,2,3-thiadiazol-4-yl)phenyl,
r is an integer from 1 to 4, s is an integer from 1 to 4,
R₂₆ is a radical of formula (c) 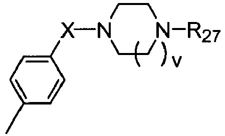 wherein R₂₇ is C(O)R₂₈, (CH₂)ₜ-C(O)R₂₉, (CH₂)ᵤR₃₀, Aryl₄, Hetaryl₂, phenylprop-1-en-3-yl or 1-methylpiperidin-4-yl, R₂₈ hydrogen, 1-4C-alkyl, OR₃₁, furanyl, indolyl, phenyl, pyridyl, phenyl substituted by R₃₄ and/or R₃₅ or pyridyl substituted by R₃₆ and/or R₃₇, R₂₉ is N(R₃₂)R₃₃, R₃₀ is N(R₃₂)R₃₃, tetrahydrofuranyl or pyridinyl, R₃₁ is 1-4C-alkyl, R₃₂ is hydrogen, 1-4C-alkyl, 3-7C-cycloalkyl or 3-7C-cycloalkylmethyl, R₃₃ is hydrogen, 1-4C-alkyl, 3-7C-cycloalkyl or 3-7C-cycloalkylmethyl, or
R₃₂ and R₃₃ together and with inclusion of the nitrogen atom to which they are bonded, form a 4-morpholinyl-, 1-pyrrolidinyl-, 1-piperidinyl- or 1-hexahydroazepinyl-ring,
Aryl₄ is phenyl, pyridyl, pyrimidinyl, phenyl substituted by R₃₄ and/or R₃₅, pyridyl substituted by R₃₆ and/or R₃₇, R₃₄ is halogen, nitro, 1-4C-alkyl, trifluoromethyl or 1-4C-alkoxy, R₃₅ is halogen or 1-4C-alkyl, R₃₆ is halogen, nitro, 1-4C-alkyl, trifluoromethyl or 1-4C-alkoxy, R₃₇ is halogen or 1-4C-alkyl,
Hetaryl₂ is indol-4-yl, 2-methyl-quinolin-4-yl, 5-chloro-6-oxo-1-phenyl-1,6-dihydro-pyridazin-4-y-1,3-phenyl-1,2,4-thiadiazol-5-yl or 3-o-tolyl-1,2,4-thiadiazol-5-yl,
t is an integer from 1 to 4, u is an integer from 1 to 4, v is an integer from 1 to 2, X is -C(O)- or -S(O)₂-, and the salts of these compounds.

The preparation of these compounds is described in US 20040127707 and WO 2002/085906.

In another case, PDE7 inhibitors useful in the methods described herein are selected from those compounds generally or specifically disclosed in U.S. Patent No. 6,818,651, US 20040044212, and WO 2002/040450. In one case, PDE7 inhibitors useful in the methods described herein have the formula:

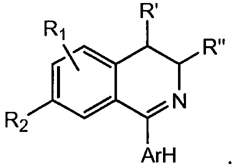

The substituents for the above compounds are defined as follows:
either R₁ denotes hydrogen, and R₂ denotes fluorine, chlorine, bromine, cyano, trifluoromethyl or phenoxy, or R₁ denotes hydrogen, fluorine, chlorine, bromine, trifluoromethyl or cyano, and R₂ denotes hydrogen, R' and R" both denote hydrogen or together represent a bond, and Ar represents a phenyl radical of the formulae IIa, IIb, or IIc 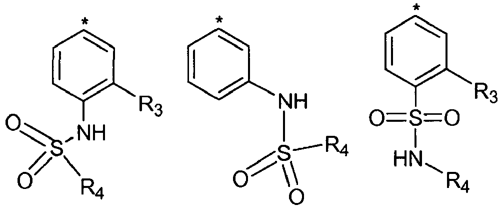 wherein R₃ denotes hydrogen, hydroxyl, nitro, amino, carboxyl, aminocarbonyl, 1-4C-alkoxy, trifluoromethoxy, 1-4C-alkoxycarbonyl or mono- or di-1-4C-alkylaminocarbonyl,
R₄ represents 1-4C-alkyl, naphthalenyl, 5-dimethylaminonaphthalen-1-yl, phenylethen-2-yl, 3,5-dimethylisoxazol-4-yl, 5-chloro-3-methylbenzo[b]thiophen-2-yl, 6-chloro-imidazo[2,1b]-thiazol-5-yl, or represents a phenyl or thiophene radical which is unsubstituted or is substituted by one or more identical or different radicals selected from the group halogen, cyano, 1-4C-alkyl, trifluoromethyl, 1-4C-alkoxy which is substituted entirely or mainly by fluorine, 1-4C-alkoxy, 1-4C-alkylcarbonylamino, 1-4C-alkoxycarbonyl, phenylsulfonyl or isoxazolyl, or
a hydrate, solvate, salt, hydrate of a salt, or solvate of a salt thereof.

The preparation of these compounds is described in U.S. Patent No. 6,818,651, US 20040044212, and WO 2002/040450.

In another case, PDE7 inhibitors useful in the methods described herein are selected from those compounds generally or specifically disclosed in WO 2002/040449. In one case, PDE7 inhibitors useful in the methods described herein have the formula:

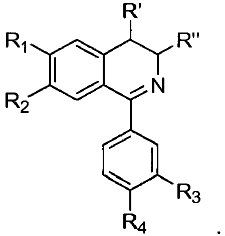

The substituents for the above compounds are defined as follows:
either R₁ denotes hydrogen and R₂ denotes fluorine, chlorine, bromine, cyano, trifluoromethyl or phenoxy, or
R₁ denotes hydrogen, fluorine, chlorine, bromine, trifluoromethyl or cyano and R₂ denotes hydrogen,
R' and R" both denote hydrogen or together represent a bond,
R₃ denotes hydrogen, hydroxyl, nitro, amino, carboxyl, aminocarbonyl, 1-4C-alkoxy, trifluoromethoxy, 1-4C-alkoxycarbonyl or mono- or di-1-4C-alkylaminocarbonyl and R₄ denotes C(O)-X-R₅, N(H)-C(O)-R₆ or N(H)-C(O)-N(H)-R₂, wherein
X denotes 0 or N(H),
R₅ denotes hydrogen, 1-4C-alkyl, 3-7C-cycloalkylmethyl, 6,6-dimethylbicyclo[3,3,I]hept-2-yl, 3-7C-alkynyl, 1-4C-alkylcarbonyl-1-4C-alkyl, aminocarbonyl-1-4C-alkyl, furan-2-ylmethyl, 2-pyridin-2-yleth-1-yl, 2-pyridin-3-ylmethyl, N-methylpiperidin-3-yl, 1-benzylpiperidin-4-yl, morpholin-4-yl-eth-2-yl, morpholin-4-yl-eth-1-yl, 2-benzo[1,3]dioxol-4-yl-eth-1-yl, chroman-4-yl, 1-methoxycarbonyl-2-indol-3-yl-eth-1-yl, 1,3-bis-methoxycarbonylprop-1-yl, 1-methoxycarbonyl-3-methylsulfanyl-eth-1-yl, 1-methoxycarbonyl-2-thiazol-2-yl-eth-1-yl, or 4-methylthiazol-5-yl-eth-2-yl, or represents a benzyl-, phenyl-eth-1-yl or 1-methoxycarbonyl-2-phenyl-eth-2-yl radical which is unsubstituted or substituted by one or more radicals selected from the group halogen, trifluoromethyl and phenyl,
R₆ denotes 2,4-dichlorophenoxymethyl, 2-tert-butoxycarbonylamino-eth-1-yl, 1-acetylpiperidin-4-yl, Ar1 or Ar2-CH=CH-,
where Ar1 represents 3-chlorophenyl, 4-trifluoromethoxyphenyl, 3-phenoxyphenyl, indol5-yl, 2-methylpyridin-5-yl, quinolin-6-yl or 2-benzothiazol-6-yl, Ar2 represents furan-2-yl,furan-3-yl, thiophen-2-yl, indol-3-yl, 3-trifluoromethylphenyl, 3-methoxyphenyl or pyridin-3-yl,
R₇ represents 1-4C-alkyl, 3-7C-alkenyl, 3-7C-cycloalkyl, 1-ethoxycarbonyl-2-phenyl-eth-1-yl, thiophen-2-yleth-1-yl or a phenyl radical which is unsubstituted or substituted by one or more radicals selected from the group halogen, cyano, 1-4C-alkyl, trifluoromethyl, 1-4C-alkylthio, 1-4C-alkoxy, 1-4C-alkoxy which is entirely or predominantly substituted by fluorine, 1-4C-alkylcarbonyl and phenoxy, or
a salt thereof.

The preparation of these compounds is described in WO 2002/040449.

In another case, PDE7 inhibitors useful in the methods described herein are selected from those compounds generally or specifically disclosed in WO 2001/098274. In one case, PDE7 inhibitors useful in the methods described herein have the formula:

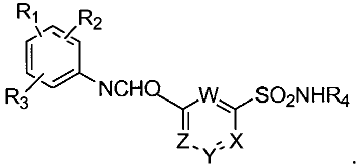

The substituents for the above compounds are defined as follows:
W, X, Y and Z, which may be the same or different, each represents a nitrogen atom or a C(R⁵) group [wherein R₅ is a hydrogen or halogen atom or an alkyl, haloalkyl, alkoxy, haloalkoxy, hydroxy, -NO₂ or -CN group] provided that two or more of W, X, Y, and Z are C(R₅) groups;
R₁, R₂ and R₃, which may be the same or different, each is an atom or group -L₁(Alk₁)ᵣL₂(R₆)ₛ wherein L₁ and L₂, which may be the same or different, is each a covalent bond or a linker atom or group, r is zero or the integer 1, Alk₁ is an aliphatic or heteroaliphatic chain, s is an integer 1, 2 or 3 and R6 is a hydrogen or halogen atom or a group selected from alkyl, -OR₇ [where R₇ is a hydrogen atom or an optionally substituted alkyl group], -SR₇, NR₇R₈ [where R₈ is as just defined for R₇ and may be the same or different], -NO₂, CN, CO₂R₇, SO₃H, S(O)R₇, SO₂R₇, OCO₂R₇, CONR₇R₈, OCONR₇R₈, CSNR₇R₈, OCR₇, OCOR₇, N(R₇)COR₈, N(R₇)CSR₈, S(O)NR₇R₈, SO₂NR₇R₈, N(R₇)SO₂R₈, N(R₇)CON(R₈)(R₉) [where R₉ is a hydrogen atom or an optionally substituted alkyl group], N(R₇)CSN(R₈)(R₉), N(R₇)SO₂N(R₈)(R₉), C(R₇)=NO(R₈), cycloaliphatic, heterocycloaliphatic, aryl or heteroaryl group]; provided that one or more of R₁, R₂, or R₃ is a substituent other than a hydrogen atom;
R₄ represents an optionally substituted phenyl, 1- or 2- naphthyl, pyridyl, pyrimidinyl, pyridazinyl, or pyrazinyl group; and
the salts, solvates, hydrates and N-oxides thereof.

The preparation of these compounds is described in WO 2001/098274.

In another case, PDE7 inhibitors useful in the methods described herein are selected from those compounds generally or specifically disclosed in WO 2001/074786. In one case, PDE7 inhibitors useful in the methods described herein have the formula:

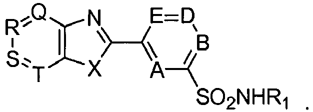

The substituents for the above compounds are defined as follows:
R₁ represents an aryl or heteroaryl group;
A, B, P, and E, which may be the same or different, each represents a nitrogen atom or a C(R₂) group [wherein R₂ is a hydrogen or halogen atom or an alkyl, haloalkyl, alkoxy, haloalkoxy, hydroxy, -NO₂ or -CN group] provided that two or more of A, B, D, and E are C(R₂) groups; X represents an oxygen or sulphur atom or a N(R₃) group wherein R₃ is a hydrogen atom or an alkyl group;
Q, R, S, and T, which may be the same or different each represents a nitrogen atom or a group C(R₄) [wherein R₄ is an atom or group -L₁(Alk₁)rL₂(R₅)s wherein L₁ and L₂, which may be the same or different, is each a covalent bond or a linker atom or group, r is zero or the integer 1, Alkyl is an aliphatic or heteroaliphatic chain, s is an integer 1, 2 or 3 and R₅ is a hydrogen or halogen atom or a group selected from alkyl, OR₆ [where R₆ is a hydrogen atom or an optionally substituted alkyl group], SR₆, NR₆R₇ [where R₇ is as just defined for R₆ and may be the same or different], NO₂, CN, CO₂R₆, SO₃H, S(O)R₆, SO₂R₆, OCO₂R₆, CONR₆R₇, OCONR₆R₇, CSNR₇R₇, OCR₆, OCOR₆, N(R₆)COR₇, N(R₆)CSR₇, S(O)NR₆R₇, SO₂NR₆R₇, N(R₆)SO₂R₇; N(R₆)CON(R₇)(R₈) [where R₈ is a hydrogen atom or an optionally substituted alkyl group], N(R₆)CSN(R₇)(R₈), N(R₆)SO₂N(R₇)(R₈), C(R₆)=NO(R₇) cycloaliphatic, heterocycloaliphatic, aryl or heteroaryl group] provided that two or more of Q, R, S, and T are C(R₄) groups; and the salts, solvates, hydrates and N-oxides thereof.

The preparation of these compounds is described in WO 2001/074786.

In another case, PDE7 inhibitors useful in the methods described herein are selected from those compounds generally or specifically disclosed in WO 2000/068230. In one case, PDE7 inhibitors useful in the methods described herein have the formula:

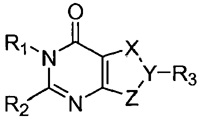

The substituents for the above compounds are defined as follows:
X-Y-Z represents NR₄-C=N or N=C-NR₄;
R₁ represents H, alkyl, cycloalkyl, cycloalkylalkyl, arylalkyl, heteroarylalkyl or heterocycloalkyl;
R₂ represents OR₈, NR₈R₉, SR₁₃, alkyl or CF₃;
R₃ represents halogen, alkyl, CF₃ or OR₈;
R₄, which can be attached to either X or Z, is a residue selected from 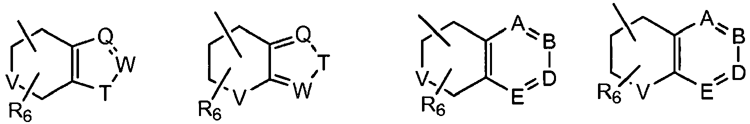 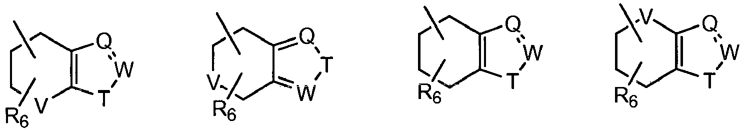 wherein attachment is through any position on the saturated ring, provided the attachment is not at a position adjacent to V, and the saturated ring may be substituted at any position with one or more R₆;
A, B, D, and E are the same or different and each represents ClₙR₅, N or N-O;
V represents O, S, NR₇ or C(L¹ₘR₁₄)(L²ₙR₁₄);
Q and W are the same or different and each represents CLₙR₅ or N;
T represents O, S or NR₇;
L¹ and L² are the same or different and each represents C(R₁₅)₂;
m and n are the same or different and each represents 0, 1, 2, 3, 4 or 5;
the R₅s are the same or different and each represents H, halogen, alkyl, cycloalkyl, OR₈, NR₈R₉, CO₂R₁₀, CONR₁₁R₁₂, CONHOH, SO₂NR₁₁R₁₂, SON₁₁R₁₂, COR₁₃, SO₂R₁₃, SOR₁₃, SR₁₃, CF₃, NO₂ or CN;
R₆ represents H, alkyl, cycloalkyl, OR₈, NR₈R₉, CO₂R₁₀, CONR₁₁R₁₂, SO₂NR₁₁R₁₂, SON₁₁R₁₂, COR₁₃, SO₂R₁₃, SOR₁₃, SR₁₃, CF₃, CN or =O;
R₇ represents H or alkyl;
R₈ represents H, alkyl, cycloalkyl, cycloalkylalkyl, aryl, arylalkyl, heteroaryl, heteroarylalkyl, heterocyclo or heterocycloalkyl;
R₉ represents R₈ or alkylcarbonyl, alkoxycarbonyl, alkylsulphonyl, cycloalkylcarbonyl, cycloalkoxycarbonyl, cycloalkylsulphonyl, cycloalkylalkylcarbonyl, cycloalkylalkoxycarbonyl, cycloalkylalkylsulphonyl, arylcarbonyl, arylsulphonyl, heteroarylcarbonyl, heteroarylsulphonyl, heterocyclocarbonyl, heterocyclosulphonyl, arylalkylcarbonyl, arylalkoxycarbonyl, arylalkylsulphonyl, heteroarylalkylcarbonyl, heteroarylalkoxycarbonyl, heteroarylsulphonyl, heterocycloalkylcarbonyl, heterocycloalkoxycarbonyl or heterocycloalkylsulphonyl; or
NR₈R₉ represents a heterocyclic ring such as morpholine;
R₁₀ represents H, alkyl, cycloalkyl, cycloalkylalkyl, arylalkyl, heteroarylalkyl or heterocycloalkyl;
R₁₁ and R₁₂ are the same or different and are each R₈, or NR₁₁R₁₂ represents a heterocyclic ring such as morpholine;
R₁₃ represents alkyl, cycloalkyl, cycloalkylalkyl, aryl, arylalkyl, heteroaryl, heteroarylalkyl, heterocyclo or heterocycloalkyl;
the R₁₄s are the same or different and are each selected from H alkyl, cycloalkyl, OR₈, NR₈R₉, CO₂R₁₀, CONR₁₁R₁₂, CONHOH, SO₂NR₁₁R₁₂, SON₁₁R₁₂, COR₁₃, SO₂R₁₃, SOR₁₃, SR₁₃, CF₃, NO₂ and CN, provided that when both m and n represent 0, if one R₁₄ is OR₈, NR₈R₉ or SR₁₃, the other is not ORg, NR₈R₉ or SR₁₃; and
R₁₅ represents H, alkyl or F; or
a pharmaceutically acceptable salt thereof.

The preparation of these compounds is described in WO 2000/068230, incorporated herein by reference in its entirety.

In another case, PDE7 inhibitors useful in the methods described herein are selected from those compounds generally or specifically disclosed in US 20040106631, EP 1 400 244, and WO 2004/026818. In one case, PDE7 inhibitors useful in the methods described herein have the formula:

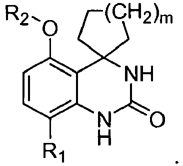

The substituents for the above compounds are defined as follows:
m is 1, 2 or 3; R₁ is methyl, chloro, bromo or fluoro; R₂ is -Q¹-Q²-Q³-Q⁴ or (C₁-C₆) alkyl, said (C₁-C₆) alkyl is substituted with one to three OR₄, COOR₄, NR₄R₅, NRC(=O)R₄, C(=O)NR₄R₅ or SO₂NR₄R₅;
R₄ is (C₁-C₆) alkyl substituted with one to three F, CN, S(=O)R₆, SO₃H, SO₂R₆, SR₇, C(=O)-NH-SO₂-CH₃, C(=O)R₇, NR'C(=O)R₇, NR'SO₂R₆, C(=O)NR₇R₈, O-C(=O)NR₇R₈ or SO₂NR₇R₈;
R₅ is H or (C₁-C₆) alkyl optionally substituted with one to three F, CN, S(=O)R₆, SO₃H, SO₂R₆, SR₇, C(=O)-NH-SO₂-CH₃, C(=O)R₇, NR'C(=O)R₇, NR'SO₂R₆, C(=O)NR₇R₈, O-C(=O)NR₇R₈ or SO₂NR₇R₈; or
said (C₁-C₆) alkyl is
   (1) substituted with one to three OC(=O)R₄ₐ, SR₄ₐ, S(=O)R₃, C(=NR₉)R₄ₐ, C(=NR₉)-NR₄ₐR₅ₐ, NR-C(=NR₉)-NR₄ₐR₅ₐ, NRCOOR₄ₐ, NR-C(=O)NR₄ₐR₅ₐ, NR-SO₂-NR₄ₐR₅ₐ, NR-C(=NR₉)-R₄ₐ or NR-SO₂-R₃; and
   (2) optionally substituted with one or two OR₄ₐ, COOR₄ₐ, C(=O)-R₄ₐ, NR₄ₐR₅ₐ, NRC(=O)R₄ₐ, C(=O)NR₄R₅ₐ or SO₂NR₄ₐR₅ₐ;
R₉ is H, CN, OH, OCH₃, SO₂CH₃ SO₂NH₂ or (C₁-C₆) alkyl; and R₃ is (C₁-C₆) alkyl optionally substituted with one to three F, CN, S(=O)R₆, SO₃H, SO₂R₆, C(=O)-NH-SO₂-CH₃, OR₇, SR₇, COOR₇, C(=O)R₇, O-C(=O)NR₇R₈, NR₇R₈, NR'C(=O)R₇, NR'SO₂R₆, C(=O)NR₇R₈ or SO₂NR₇R₈;
R₄ₐ and R₅ₐ are the same or different and are H or (C₁-C₆) alkyl optionally substituted with one to three F, CN, S(=O)R₆, SO₃H, SO₂R₆, C(=O)-NH-SO₂-CH₃, OR₇, SR₇, COOR₇, C(=O)R₇, O-C(=O)NR₇R₈, NR₇R₈, NR'C(=O)R₇, NR'SO₂R₆, C(=O)NR₇R₈ or SO₂NR₇R₈;
Q¹ is a single bond or (C₁-C₆) alkylene; Q² is a saturated 4- to 6-membered heterocyclyl comprising one or two O or N; Q³ is (C₁-C₆) alkylene; Q⁴ is a 4 to 8-membered, aromatic or non aromatic, heterocyclyl comprising 1 to 4 O, S, S(=O), SO₂, or N, said heterocyclyl being optionally substituted with one to three OR, NRR', -CN or (C₁-C₆)alkyl;
R is H or (C₁-C₆) alkyl;
R₆ is (C₁-C₆) alkyl optionally substituted with one or two OR';
R₇ and R₈ are the same or different and are H or (C₁-C₆) alkyl optionally substituted with one or two OR';
R₉ is H, CN, OH, OCH₃, SO₂CH₃, SO₂NH₂ or (C₁-C₆) alkyl;
R' is H or (C₁-C₆) alkyl; and R" is H or (C₁-C₆) alkyl;
provided that (1) the atom of Q² bound to Q¹ is a carbon atom; and (2) the atom of Q⁴ bound to Q³ is a carbon atom;
or a racemic form, isomer, pharmaceutically acceptable derivative thereof.

The preparation of these compounds is described in US 20040106631, EP 1 400 244, and WO 2004/026818.

In another case, PDE7 inhibitors useful in the methods described herein are selected from those compounds generally or specifically disclosed in U.S. Patent No. 6,936,609 and US 20040249148. In one case, PDE7 inhibitors useful in the methods described herein have the formula:

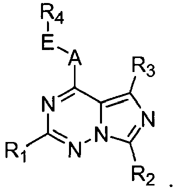

The substituents for the above compounds are defined as follows:
R₁ represents (C₆-C₁₀)-aryl, which is optionally identically or differently substituted by radicals selected from the group consisting of halogen, formyl, carbamoyl, cyano, hydroxyl, trifluoromethyl, trifluoromethoxy, nitro, (C₁-C₆)-alkyl or (C₁-C₆)-alkoxy, and optionally by a radical of the formula SO₂NR₅R₆, wherein R₅ and R₆ independently of one another denote hydrogen or (C₁-C₆)-alkyl, or NR₅R₆ denotes 4- to 8-membered heterocyclyl, bonded via a nitrogen atom, optionally identically or differently substituted by radicals selected from the group consisting of oxo, halogen, (C₁-C₆)-alkyl and (C₁-C₆)-acyl,
R₂ represents a saturated or partially unsaturated hydrocarbon radical having 1 to 10 carbon atoms,
R₃ represents methyl or ethyl,
A represents O, S, or NR₇, wherein R₇ denotes hydrogen or (C₁-C₆)-alkyl optionally substituted by (C₁-C₃)-alkoxy,
E represents a bond or (C₁-C₃)-alkanediyl,
R₄ represents (C₆-C₁₀)-aryl or 5- to 10-membered heteroaryl, where aryl and heteroaryl are optionally identically or differently substituted by radicals selected from the group consisting of halogen, formyl, carboxyl, carbamoyl, -SO₃H, aminosulphonyl, cyano, hydroxyl, trifluoromethyl, trifluoromethoxy, nitro, (C₁-C₆)-alkyl, (C₁-C₆)-alkoxy, 1,3-dioxa-propane-1,3-diyl, (C₁-C₆)-alkylthio, (C₁-C₆)-allcylsulphinyl and (C₁-C₆)-alkyl-sulphonyl, -NR₈R₉ end optionally methyl-substituted, 5- to 6-membered heteroaryl or phenyl,
wherein R₈ and R₉ independently of one another denote hydrogen, (C₁-C₆)-alkyl or (C₁-C₆)-acyl, or salt thereof.

The preparation of these compounds is described in U.S. Patent No. 6,936,609 and US 20040249148.

In another case, PDE7 inhibitors useful in the methods described herein are selected from those compounds generally or specifically disclosed in WO 2006/092692. In one case, PDE7 inhibitors useful in the methods described herein have the formulas: 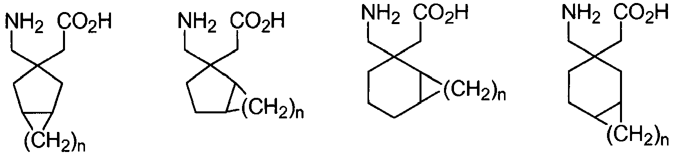 wherein n is an integer of from 1 to 4, and where there are stereocenters, each center may be independently R or S.

The preparation of these compounds is described in WO 2006/092692.

In another case, PDE7 inhibitors useful in the methods described herein are selected from those compounds generally or specifically disclosed in US 2006229306 and WO 2004/065391. In one case, PDE7 inhibitors useful in the methods described herein have the formula:

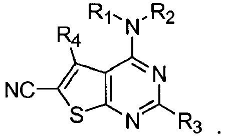

The substituents for the above compounds are defined as follows:
R₁ and R₂ either
   (1) independently represent:
      (a) a hydrogen atom;
      (b) a group selected from alkyl, alkenyl and alkynyl groups, wherein each alkyl, alkenyl and alkynyl group is independently optionally substituted by one or more substituents selected from halogen atoms, hydroxy, alkoxy, aryloxy, alkylthio, hydroxycarbonyl, alcoxycarbonyl, mono- and di-alkylaminoacyl, oxo, amino, and mono- and di-alkylamino groups; or
      (c) a group of formula (CH₂)ₙ-R₆, wherein n is an integer from 0 to 4 and R₆ represents a cycloalkyl or cycloalkenyl group;
   (2) R₁ and R₂ form, together with the nitrogen atom to which they are attached, a 3- to 8-membered ring comprising from 1 to 4 heteroatoms selected from nitrogen, oxygen and sulphur, which ring is saturated or unsaturated and optionally substituted by one or more substituents selected from halogen atoms, alkyl, hydroxy, alkoxy, acyl, hydroxycarbonyl, alkoxycarbonyl, alkylenedioxy, amino, mono- and di-alkylamino, mono- and di-alkylaminoacyl, nitro, cyano and trifluoromethyl groups;
R₃ is a group of formula (CH₂)_{n-G}, wherein n is an integer from 0 to 4 and G represents a monocyclic or bicyclic aryl or heteroaryl group comprising from zero to four heteroatoms which group is optionally substituted by one or more substituents selected from:
   (1) halogen atoms;
   (2) alkyl and alkylene groups, wherein each alkyl and alkylene group is independently optionally substituted by one or more substituents selected from halogen atoms; and
   (3) phenyl, hydroxy, hydroxyalkyl, alkoxy, alkylenedioxy, aryloxy, alkylthio, amino, mono- and di-alkylamino, acylamino, nitro, acyl, hydroxycarbonyl, alkoxycarbonyl, cyano, difluoromethoxy and trifluoromethoxy groups;
R₄ represents a hydrogen atom, an alkyl or an aryl group.

The preparation of these compounds is described in US 2006229306 and WO 2004/065391.

Other compounds useful in the methods described herein include imidazopyridine derivatives (WO 2001/34601), dihydropurine derivatives (WO 2000/68203), pyrrole derivatives (WO 2001/32618), benzothiopyranoimidazolone derivatives (DE 19950647), heterocyclic compounds (WO 2002/87519), guanine derivatives *(*Bioorg. Med. Chem. Lett. 11:1081, 2001), and benzothienothiadiazine derivatives (Eur. J. Med. Chem. 36:333, 2001).

### Polypeptide or Peptide Inhibitors

In some cases, the PDE7 inhibitory agent comprises isolated PDE7 polypeptide or peptide inhibitors, including isolated natural peptide inhibitors and synthetic peptide inhibitors that inhibit PDE7 activity. As used herein, the term "isolated PDE7 polypeptide or peptide inhibitors" refers to polypeptides or peptides that inhibit PDE7 dependent cleavage of cAMP by binding to PDE7, competing with PDE7 for binding to a substrate, and/or directly interacting with PDE7 to inhibit PDE7-dependent cleavage of cAMP, that are substantially pure and are essentially free of other substances with which they may be found in nature to an extent practical and appropriate for their intended use.

Peptide inhibitors have been used successfully *in vivo* to interfere with protein-protein interactions and catalytic sites. For example, peptide inhibitors to adhesion molecules structurally related to LFA-1 have recently been approved for clinical use in coagulopathies (Ohman, E.M., et al., European Heart J. 16:50-55, 1995). Short linear peptides (<30 amino acids) have been described that prevent or interfere with integrin-dependent adhesion (Murayama, O., et al., J. Biochem. 120:445-51, 1996). Longer peptides, ranging in length from 25 to 200 amino acid residues, have also been used successfully to block integrin-dependent adhesion (Zhang, L., et al., J. Biol. Chem. 271(47):29953-57, 1996). In general, longer peptide inhibitors have higher affinities and/or slower off-rates than short peptides and may therefore be more potent inhibitors. Cyclic peptide inhibitors have also been shown to be effective inhibitors of integrins *in vivo* for the treatment of human inflammatory disease (Jackson, D.Y., et al., J. Med. Chem. 40:3359-68, 1997). One method of producing cyclic peptides involves the synthesis of peptides in which the terminal amino acids of the peptide are cysteines, thereby allowing the peptide to exist in a cyclic form by disulfide bonding between the terminal amino acids, which has been shown to improve affinity and half-life *in vivo* for the treatment of hematopoietic neoplasms (e.g., U.S. Patent No. 6,649,592 to Larson).

### Synthetic PDE7 Peptide Inhibitors

PDE7 inhibitory peptides useful in the methods described herein are exemplified by amino acid sequences that mimic the target regions important for PDE7 enzyme activity, such as the catalytic domain of PDE7. PDE7A and PDE7B have an identity of 70% in the catalytic domain. (Hetman, J.M., et al., PNAS 97(1):472-476, 2000.) The catalytic domain of PDE7A1 is from amino acid residue 185 to 456 of SEQ ID NO:2. The catalytic domain of PDE7A2 is from amino acid residue 211 to 424 of SEQ ID NO:4. The catalytic domain of PDEB is from amino acid residue 172 to 420 of SEQ ID NO:6. The inhibitory peptides useful in the practice of the methods described herein range in size from about 5 amino acids to about 250 amino acids. One may also use molecular modeling and rational molecular design to generate and screen for peptides that mimic the molecular structure of the PDE7 catalytic regions and inhibit the enzyme activity of PDE7. The molecular structures used for modeling include the CDR regions of anti-PDE7 monoclonal antibodies. Methods for identifying peptides that bind to a particular target are well known in the art. For example, molecular imprinting may be used for the *de novo* construction of macromolecular structures such as peptides that bind to a particular molecule. *See,* for example, Shea, K.J., "Molecular Imprinting of Synthetic Network Polymers: The De Novo Synthesis of Macromolecular Binding and Catalytic Sties," TRIP 2(5):[no page numbers], 1994.

As an illustrative example, one method of preparing mimics of PDE7 binding peptides is as follows. Functional monomers of a binding region of an anti-PDE7 antibody that exhibits PDE7 inhibition (the template) are polymerized. The template is then removed, followed by polymerization of a second class of monomers in the void left by the template, to provide a new molecule that exhibits one or more desired properties that are similar to the template. In addition to preparing peptides in this manner, other PDE7 binding molecules that are PDE7 inhibitory agents, such as polysaccharides, nucleosides, drugs, nucleoproteins, lipoproteins, carbohydrates, glycoproteins, steroids, lipids, and other biologically active materials, can also be prepared. This method is useful for designing a wide variety of biological mimics that are more stable than their natural counterparts because they are typically prepared by free radical polymerization of functional monomers, resulting in a compound with a nonbiodegradable backbone.

The PDE7 inhibitory peptides can be prepared using techniques well known in the art, such as the solid-phase synthetic technique initially described by Merrifield in J. Amer. Chem. Soc. 85:2149-2154, 1963. Automated synthesis may be achieved, for example, using Applied Biosystems 431A Peptide Synthesizer (Foster City, Calif.) in accordance with the instructions provided by the manufacturer. Other techniques may be found, for example, in Bodanszky, M., et al., Peptide Synthesis, second edition, John Wiley & Sons, 1976, as well as in other reference works known to those skilled in the art. The peptides can also be prepared using standard genetic engineering techniques known to those skilled in the art.

A candidate PDE7 inhibitory peptide may be tested for the ability to function as a PDE7 inhibitory agent in one of several assays, including, for example, a PDE7 phosphodiesterase assay as described in Example 1.

### Expression Inhibitors of PDE7

In some cases of the methods described herein, the PDE7 inhibitory agent is a PDE7 expression inhibitor capable of inhibiting PDE7-dependent cAMP cleavage (PDE7A, PDE7B, or both). In the practice of this case described herein, representative PDE7 expression inhibitors include PDE7 antisense nucleic acid molecules (such as antisense mRNA, antisense DNA, or antisense oligonucleotides), PDE7 ribozymes, and PDE7 RNAi molecules.

Anti-sense RNA and DNA molecules act to directly block the translation of PDE7 mRNA by hybridizing to PDE7 mRNA and preventing translation of PDE7 protein. An antisense nucleic acid molecule may be constructed in a number of different ways provided that it is capable of interfering with the expression of PDE7. For example, an antisense nucleic acid molecule can be constructed by inverting the coding region (or a portion thereof) of PDE7A1 cDNA (SEQ ID NO:1), PDE7A2 cDNA (SEQ ID NO:3) or PDE7B cDNA (SEQ ID NO:5) relative to its normal orientation for transcription to allow for the transcription of its complement. Methods for designing and administering antisense oligonucleotides are well known in the art and are described, e.g., in Mautino et al., Hum Gene Ther 13:1027-37, 2002; and Pachori et al., Hypertension 39:969-75, 2002.

The antisense nucleic acid molecule is usually substantially identical to at least a portion of the target gene or genes. The nucleic acid, however, need not be perfectly identical to inhibit expression. Generally, higher homology can be used to compensate for the use of a shorter antisense nucleic acid molecule. The minimal percent identity is typically greater than about 65%, but a higher percent identity may exert a more effective repression of expression of the endogenous sequence. Substantially greater percent identity of more than about 80% typically is preferred, though about 95% to absolute identity is typically most preferred.

The antisense nucleic acid molecule need not have the same intron or exon pattern as the target gene, and non-coding segments of the target gene may be equally effective in achieving antisense suppression of target gene expression as coding segments. A DNA sequence of at least about 8 or so nucleotides may be used as the antisense nucleic acid molecule, although a longer sequence is preferable. In the present disclosure, a representative example of a useful inhibitory agent of PDE7 is an antisense PDE7 nucleic acid molecule that is at least ninety percent identical to the complement of a portion of the PDE7A1 cDNA consisting of the nucleic acid sequence set forth in SEQ ID NO:1. Another representative example of a useful inhibitory agent of PDE7 is an antisense PDE7 nucleic acid molecule which is at least ninety percent identical to the complement of a portion of the PDE7A2 cDNA consisting of the nucleic acid sequence set forth in SEQ ID NO:3. Another representative example of a useful inhibitory agent of PDE7 is an antisense PDE7 nucleic acid molecule which is at least ninety percent identical to the complement of a portion of the PDE7B cDNA consisting of the nucleic acid sequence set forth in SEQ ID NO:5.

The targeting of antisense oligonucleotides to bind PDE7 mRNA is another mechanism that may be used to reduce the level of PDE7 protein synthesis. For example, the synthesis of polygalacturonase and the muscarine type 2 acetylcholine receptor is inhibited by antisense oligonucleotides directed to their respective mRNA sequences (U.S. Patent No. 5,739,119 to Cheng, and U.S. Patent No. 5,759,829 to Shewmaker). Furthermore, examples of antisense inhibition have been demonstrated with the nuclear protein cyclin, the multiple drug resistance gene (MDG1), ICAM-1, E-selectin, STK-1, striatal GABA_{A} receptor and human EGF (see, e.g., U.S. Patent No. 5,801,154 to Baracchini; U.S. Patent No. 5,789,573 to Baker; U.S. Patent No. 5,718,709 to Considine; and U.S. Patent No. 5,610,288 to Reubenstein).

A system has been described that allows one of ordinary skill to determine which oligonucleotides are useful in the methods described herein, which involves probing for suitable sites in the target mRNA using Rnase H cleavage as an indicator for accessibility of sequences within the transcripts. Scherr, M., et al., Nucleic Acids Res. 26:5079-5085, 1998; Lloyd, et al., Nucleic Acids Res. 29:3665-3673, 2001. A mixture of antisense oligonucleotides that are complementary to certain regions of the PDE7 transcript is added to cell extracts expressing PDE7 and hybridized in order to create an RNAseH vulnerable site. This method can be combined with computer-assisted sequence selection that can predict optimal sequence selection for antisense compositions based upon their relative ability to form dimers, hairpins, or other secondary structures that would reduce or prohibit specific binding to the target mRNA in a host cell. These secondary structure analysis and target site selection considerations may be performed using the OLIGO primer analysis software (Rychlik, I, 1997) and the BLASTN 2.0.5 algorithm software (Altschul, S.F., et al., Nucl. Acids Res. 25:3389-3402, 1997). The antisense compounds directed towards the target sequence preferably comprise from about 8 to about 50 nucleotides in length. Antisense oligonucleotides comprising from about 9 to about 35 or so nucleotides are particularly preferred. The inventors contemplate all oligonucleotide compositions in the range of 9 to 35 nucleotides (i.e., those of 9, 10, 11, 12, 13, 14, 15, 16, 17, 18, 19, 20, 21, 22, 23, 24, 25, 26, 27, 28, 29, 30, 31, 32, 33, 34, or 35 or so bases in length) are highly preferred for the practice of antisense oligonucleotide-based methods of the disclosure. Highly preferred target regions of the PDE7 mRNA are those that are at or near the AUG translation initiation codon, and those sequences that are substantially complementary to 5' regions of the mRNA, e.g., between the 0 and+10 regions of the PDE7 gene nucleotide sequence (SEQ ID NO:1, SEQ ID NO:3, SEQ ID NO:5).

The term "oligonucleotide" as used herein refers to an oligomer or polymer of ribonucleic acid (RNA) or deoxyribonucleic acid (DNA) or mimetics thereof. This term also covers those oligonucleobases composed of naturally occurring nucleotides, sugars and covalent internucleoside (backbone) linkages as well as oligonucleotides having non-naturally occurring modifications. These modifications allow one to introduce certain desirable properties that are not offered through naturally occurring oligonucleotides, such as reduced toxic properties, increased stability against nuclease degradation and enhanced cellular uptake. In illustrative cases, the antisense compounds of the invention differ from native DNA by the modification of the phosphodiester backbone to extend the life of the antisense oligonucleotide in which the phosphate substituents are replaced by phosphorothioates. Likewise, one or both ends of the oligonucleotide may be substituted by one or more acridine derivatives that intercalate between adjacent basepairs within a strand of nucleic acid.

Another alternative to antisense is the use of "RNA interference" (RNAi). Double-stranded RNAs (dsRNAs) can provoke gene silencing in mammals *in vivo.* The natural function of RNAi and co-suppression appears to be protection of the genome against invasion by mobile genetic elements such as retrotransposons and viruses that produce aberrant RNA or dsRNA in the host cell when they become active (see, e.g., Jensen, J., et al., Nat. Genet. 21:209-12, 1999). The double-stranded RNA molecule may be prepared by synthesizing two RNA strands capable of forming a double-stranded RNA molecule, each having a length from about 19 to 25 (e.g., 19-23 nucleotides). For example, a dsRNA molecule useful in the methods described herein may comprise the RNA corresponding to a portion of at least one of SEQ ID NO:1, SEQ ID NO:3, SEQ ID NO:5 and its complement. Preferably, at least one strand of RNA has a 3' overhang from 1-5 nucleotides. The synthesized RNA strands are combined under conditions that form a double-stranded molecule. The RNA sequence may comprise at least an 8 nucleotide portion of SEQ ID NO:1, SEQ ID NO:3 or SEQ ID NO:5 with a total length of 25 nucleotides or less. The design of siRNA sequences for a given target is within the ordinary skill of one in the art. Commercial services are available that design siRNA sequence and guarantee at least 70% knockdown of expression (Qiagen, Valencia, CA). Exemplary PDE7 shRNAs and siRNAs are commercially available from Sigma-Aldrich Company (product # SHDNA_-NM_002603; SASI_Hs01_00183420 to SASI_Hs01_00010490).

The dsRNA may be administered as a pharmaceutical composition and carried out by known methods, wherein a nucleic acid is introduced into a desired target cell. Commonly used gene transfer methods include calcium phosphate, DEAE-dextran, electroporation, microinjection and viral methods. Such methods are taught in Ausubel et al., Current Protocols in Molecular Biology, John Wiley & Sons, Inc., 1993. Therapeutic nucleic acid molecules may be modified to cross the blood-brain barrier. For example, it has been demonstrated that a phosphorothiolate antisense oligonucleotide directed towards the Abeta midregion of amyloid precursor protein (APP) given by i.c.v. administration can reverse the learning and memory deficits in an Alzheimer mouse model. Banks W.A. et al., Journal of Pharm. and Exp. Therapeutics, 297(3):1113-1121, 2001.

### Ribozymes:

In some cases, a PDE7 inhibitory agent is a ribozyme that specifically cleaves the mRNA of a target PDE7, such as PDE7A, PDE7B or both. Ribozymes that target PDE7 may be utilized as PDE7 inhibitory agents to decrease the amount and/or biological activity of PDE7. Ribozymes are catalytic RNA molecules that can cleave nucleic acid molecules having a sequence that is completely or partially homologous to the sequence of the ribozyme. It is possible to design ribozyme transgenes that encode RNA ribozymes that specifically pair with a target RNA and cleave the phosphodiester backbone at a specific location, thereby functionally inactivating the target RNA. In carrying out this cleavage, the ribozyme is not itself altered, and is thus capable of recycling and cleaving other molecules. The inclusion of ribozyme sequences within antisense RNAs confers RNA-cleaving activity upon them, thereby increasing the activity of the antisense constructs.

Ribozymes useful in the practice of the methods described herein typically comprise a hybridizing region of at least about nine nucleotides, which is complementary in nucleotide sequence to at least part of the target PDE7 mRNA, and a catalytic region that is adapted to cleave the target PDE7 mRNA (see generally, European patent No. 0 321 201; WO 88/04300; Haseloff, J., et al., Nature 334:585-591, 1988; Fedor, M.J., et al., Proc. Natl. Acad. Sci. USA 87:1668-1672, 1990; Cech, T.R., et al., Ann. Rev. Biochem. 55:599-629, 1986).

Ribozymes can either be targeted directly to cells in the form of RNA oligonucleotides incorporating ribozyme sequences, or introduced into the cell as an expression vector encoding the desired ribozymal RNA. Ribozymes may be used and applied in much the same way as described for antisense polynucleotides.

Anti-sense RNA and DNA, ribozymes and RNAi molecules useful in the methods of the invention may be prepared by any method known in the art for the synthesis of DNA and RNA molecules. These include techniques for chemically synthesizing oligodeoxyribonucleotides and oligoribonucleotides well known in the art, such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Various well known modifications of the DNA molecules may be introduced as a means of increasing stability and half-life. Useful modifications include, but are not limited to, the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone.

### IV. SCREENING METHODS FOR PDE7 INHIBITORS USEFUL TO TREAT A MOVEMENT ABNORMALITY ASSOCIATED WITH THE PATHOLOGY OF A NEUROLOGICAL MOVEMENT DISORDER

Methods are provided for identifying an agent that inhibits PDE7 activity useful for treating a movement abnormality associated with the pathology of a neurological movement disorder in a mammalian subject in need thereof. Such methods comprise: (a) determining the IC₅₀ for inhibiting PDE7 activity for a plurality of each of a plurality of agents; (b) selecting agents from the plurality of agents having an IC₅₀ for inhibition of PDE7 activity of less than about 1 µM; (c) determining the IC₅₀ for inhibiting PDE4 activity of the agents having an IC₅₀ for inhibiting PDE7 activity of less than about 1 µM; (d) identifying agents useful for treating a movement disorder by selecting compounds having an IC₅₀ for inhibiting PDE4 activity greater than 10 times the IC₅₀ for inhibiting PDE7; and (e) evaluating the activity of the identified compounds in a neurological movement disorder model assay, wherein an agent that has an IC₅₀ for PDE7 inhibition of less than about 1 µM, and an IC₅₀ for inhibiting PDE4 activity greater than 10 times the IC₅₀ for inhibiting PDE7, and is determined to be effective to treat at least one movement abnormality in a model assay is indicative of a PDE7 inhibitory agent useful for treating a movement abnormality associated with the pathology of a neurological movement disorder in a mammalian subject.

Representative agents that may be used in the practice such methods include molecules that bind to PDE7 and inhibit the enzyme activity of PDE7 (such as small molecule inhibitors or blocking peptides that bind to PDE7 and reduce enzymatic activity), and molecules that decrease the expression of PDE7 at the transcriptional and/or translational level (such as PDE7 antisense nucleic acid molecules, PDE7 specific RNAi molecules and PDE7 ribozymes), thereby preventing PDE7 from cleaving cAMP.

### V. General Composition Description and Definitions.

Described herein is treating a movement abnormality associated with the pathology of a neurological movement disorder comprising administering to a patient in need thereof an amount of a PDE7 inhibitory agent effective to inhibit the enzymatic activity of PDE7, wherein such inhibition of PDE7 enzymatic activity is the principal therapeutic mode of action of the PDE7 inhibitor in the treatment of the movement abnormality. In some cases of this method, the neurological movement disorder is treatable with a dopamine receptor agonist or a precursor of a dopamine receptor agonist. In some cases of this method, the neurological movement disorder is selected from the group consisting of Parkinson's disease, Post-Encephalitic Parkinsonism, Dopamine-Responsive Dystonia, Shy-Drager Syndrome, Periodic Limb Movement Disorder (PLMD), Periodic Limb Movements in Sleep (PLMS), and Restless Leg(s) Syndrome (RLS).

In other cases, the neurological movement disorder is selected from the group consisting of Tourrette's syndrome, Huntington's disease (i.e., Huntington's chorea), and drug-induced Parkinsonism.

For each of the PDE7 inhibitory chemical compounds useful in the method described herein, all possible stereoisomers and geometric isomers are included. The compounds include not only racemic compounds, but also the optically active isomers. When a PDE7 inhibitory agent is desired as a single enantiomer, it can be obtained either by resolution of the final product or by stereospecific synthesis from either isomerically pure starting material or use of a chiral auxiliary reagent, for example, see Ma, Z., et al., Tetrahedron: Asymmetry 8(6):883-888, 1997. Resolution of the final product, an intermediate, or a starting material can be achieved by any suitable method known in the art. Additionally, in situations where tautomers of the compounds are possible, the present invention is intended to include all tautomeric forms of the compounds.

The PDE7 inhibitory agents that contain acidic moieties can form pharmaceutically acceptable salts with suitable cations. Suitable pharmaceutically acceptable cations include alkali metal (e.g., sodium or potassium) and alkaline earth metal (e.g., calcium or magnesium) cations. The pharmaceutically acceptable salts of the PDE7 inhibitory agents, which contain a basic center, are acid addition salts formed with pharmaceutically acceptable acids. Examples include the hydrochloride, hydro bromide, sulfate or bisulfate, phosphate or hydrogen phosphate, acetate, benzoate, succinate, fumarate, maleate, lactate, citrate, tartarate, gluconate, methanefulgonate, bezenesulphonate, and p-toluenesulphonate salts. In light of the foregoing, any reference to compounds useful in the method described herein is intended to include PDE7 inhibitory agents, as well as pharmaceutically acceptable salts and solvates thereof.

The compounds for use in the methods described herein can be therapeutically administered as the neat chemical, but it is preferable to administer the PDE7 inhibitory agents as a pharmaceutical composition or formulation. Accordingly, described herein are pharmaceutical compositions or formulations comprising a PDE7 inhibitory agent, or pharmaceutically acceptable salts thereof, together with one or more pharmaceutically acceptable carriers and, optionally, other therapeutic and/or prophylactic ingredients. Suitable carriers are compatible with the other ingredients of the formulation and not deleterious to the recipient thereof. Compounds for use in the methods described herein may also be carried in a delivery system to provide for sustained release or enhanced uptake or activity of the compound, such as a liposomal or hydrogel system for injection, a microparticle, nanopartical, or micelle system for oral or parenteral delivery, or a staged capsule system for oral delivery.

In particular, a selective PDE7 inhibitory agent useful in the method described herein is useful alone or in combination with one or more additional therapeutic agent, for example: a dopamine receptor agonist, a precursor of a dopamine receptor agonist, another dopaminergic agent, or combinations of the foregoing. Examples of dopamine receptor agonists and precursors include for example levodopa (also referred to as "L-dopa"), carbidopa, bromocriptine, pergolide, pramipexole, ropinirole cabergoline, apomorphine and lisuride. Other agents useful in combination with a selective PDE7 inhibitory agent include anticholinergic medications, such as biperidenHCl, benztropine mesylate, procyclidine and trihexyphenidyl; monoamine oxidase B inhibitors, such as Eldepryl™, Atapryl™ and Carbex™ and the NMDA antagonist amantadine (Symmetrel™).

In one case, a selective PDE7 inhibitory agent is useful in combination with one or more additional therapeutic agents or precursors of therapeutic agents that activate the dopamine D1 receptor and/or increase the concentration of dopamine in the nigrostriatal nerve terminals and/or the nigrostriatal synaptic cleft. Such agents include L-dopa, nonselective dopamine receptor agonists such as apomorphine, bromocryptine, and pergolide; and D1 selective agents such as ABT-431, A86929, and SKF38393.

In one case, there is described treating a movement abnormality associated with the pathology of Parkinson's disease comprising administering to a patient in need thereof an amount of a PDE7 inhibitory agent effective to inhibit the enzymatic activity of PDE7, wherein such inhibition of PDE7 is the principal therapeutic mode of action of the PDE7 inhibitor in the treatment of the movement abnormality. As demonstrated in Examples 5-7 herein, selective PDE7 inhibitors are useful in the treatment of a movement abnormality associated with the pathology of Parkinson's disease. In the context of Parkinson's disease, treatment includes symptomatic therapy to lessen, alleviate, mask, or prevent the symptoms of at least one movement abnormality selected from the group consisting of tremor at rest, rigidity, bradykinesia, or deficiency of postural reflexes.

Compounds and pharmaceutical compositions suitable for use in the methods described herein include those wherein the active ingredient is administered in an effective amount to achieve its intended purpose. More specifically, a "therapeutically active amount" means an amount effective to prevent development of symptoms in, or to alleviate the existing symptoms of, the subject being treated. Determination of the effective amounts is well within the capability of those skilled in the art, especially in light of the detailed disclosure provided herein. It is further appreciated that the amount of a compound required for use in treatment varies with the nature of the condition being treated, and with the age and condition of the patient, and is ultimately determined by the attendant physician. Generally, however, doses employed for adult human treatment typically are in the range of 0.001 mg/kg to about 100 mg/kg per day.

A "therapeutically effective dose" refers to that amount of the compound that results in achieving the desired effect to treat or ameliorate a movement abnormality associated with the pathology of a neurological movement disorder. Toxicity and therapeutic efficacy of such compounds can be determined by standard pharmaceutical procedure in cell cultures or experimental animals, e.g., for determining the LD50 (the dose lethal to 50% of the population) and the ED50 (the dose therapeutically effective in 50% of the population).

In one case, a therapeutically effective dose is an amount of PDE7 inhibitory agent sufficient to inhibit PDE7 enzyme activity in a neuronal cell. In another case of the methods described herein, a therapeutically effective dose is an amount of PDE7 inhibitory agent sufficient to inhibit PDE7 enzyme activity in striatal neurons. The determination of an effective dose of a PDE7 inhibitory agent sufficient to cross a cellular membrane and inhibit PDE7 enzyme activity within a cell may be determined using a cellular assay for PDE7 inhibition, such as described by Smith S.J. et al., Molecular Pharmacology 66(6): 1679-1689 (2004). The determination of an effective dose of a PDE7 inhibitory agent sufficient to inhibit PDE7 enzyme activity in the striatum may be determined using an assay for measuring the effect of a PDE inhibitory agent on cAMP levels in the striatum, as described in Siuciak J.A. et al., Neuropharmacology 51: 386-396 (2006).

The dose ratio between toxic and therapeutic effects is the therapeutic index, which is expressed as the ratio between LD50 and ED50. Compounds which exhibit high therapeutic indices are preferred. The data obtained from such analysis can be used in formulating a dosage range for use in humans. The dosage of such compounds preferably lies within a range of circulating concentrations that include the ED50 range depending upon the dosage form employed, and the route of administration utilized.

Toxicity and therapeutic efficacy of PDE7 inhibitory agents can be determined by standard pharmaceutical procedures employing experimental animal models, such as the murine MPTP model described in Examples 5-7. Using such animal models, the NOAEL (no observed adverse effect level) and the MED (the minimally effective dose) can be determined using standard methods. The dose ratio between NOAEL and MED effects is the therapeutic ratio, which is expressed as the ratio NOAEL/MED. PDE7 inhibitory agents that exhibit large therapeutic ratios or indices are most preferred. The data obtained from the cell culture assays and animal studies can be used in formulating a range of dosages for use in humans. The dosage of the PDE7 inhibitory agent preferably lies within a range of circulating concentrations that include the MED with little or no toxicity. The dosage may vary within this range depending upon the dosage form employed and the route of administration utilized.

For any compound formulation, the therapeutically effective dose can be estimated using animal models. For example, a dose may be formulated in an animal model to achieve a circulating plasma concentration or brain tissue range that includes the MED. Quantitative levels of the PDE7 inhibitory agent in plasma or brain may also be measured, for example, as described in Example 4 herein.

The exact formulation, route of administration, and dosage can be chosen by the individual physician in view of the patient's condition. Dosage amount and interval can be adjusted individually to provide plasma levels of the active moiety which are sufficient to maintain the therapeutic effects.

The desired dose can be conveniently administered in a single dose, or as multiple doses administered at appropriate intervals, for example as two, three, four, or more sub-doses per day. In practice, the physician determines the actual dosing regimen that is most suitable for an individual patient, and the dosage varies with the age, weight, and response of the particular patient. The dosages described above are exemplary of the average case, but there can be individual instances in which higher or lower dosages are merited, and such are within the scope of the present invention.

Formulations described herein can be administered in a standard manner for the treatment of the indicated diseases, such as orally, parenterally, transmucosal (e.g., sublingually or via buccal administration), topically, transdermally, rectally, via inhalation (e.g., nasal or deep lung inhalation). Parenteral administration includes, but is not limited to intravenous, intra-arterial, intraperitoneal, subcutaneous, intramuscular, intrathecal, and intraarticular. Parenteral administration also can be accomplished using a high pressure technique, such as a POWDERJECT™ system.

Also disclosed are pharmaceutical compositions comprising a PDE7 inhibitory agent for treating neurological movement disorders such as Parkinson's disease, Post-Encephalitic Parkinsonism, Dopamine-Responsive Dystonia, Shy-Drager Syndrome, Periodic Limb Movement Disorder (PLMD), Periodic Limb Movements in Sleep (PLMS), or Restless Leg(s) Syndrome (RLS).

For buccal or oral administration, the pharmaceutical composition can be in the form of tablets or lozenges formulated in conventional manner. For example, tablets and capsules for oral administration can contain conventional excipients such as binding agents (for example, syrup, acacia, gelatin, sorbitol, tragacanth, mucilage of starch, or polyvinylpyrrolidone), fillers (for example, lactose, sugar, microcrystalline, cellulose, maize-starch, calcium phosphate, or sorbitol), lubricants (for example, magnesium, stearate, stearic acid, talc, polyethylene glycol, or silica), disintegrants (for example, potato starch or sodium starch glycollate), or wetting agents (for example, sodium lauryl sulfate). The tablets can be coated according to methods well known in the art.

Alternatively, the compounds for use in the methods described herein can be incorporated into oral liquid preparations such as aqueous or oily suspensions, solutions, emulsions, syrups, or elixirs, for example. Moreover, formulations containing these compounds can be presented as a dry product for constitution with water or other suitable vehicle before use. Such liquid preparations can contain conventional additives, such as suspending agents, such as sorbitol syrup, methyl cellulose, glucose/sugar syrup, gelatin, hydroxyethylcellulose, hydroxypropylmethylcellulose, carboxymethyl cellulose, aluminum stearate gel, and hydrogenated edible fats; emulsifying agents, such as lecithin, sorbitan monooleate, or acacia; nonaqueous vehicles (which can include edible oils), such as almond oil, fractionated coconut oil, oily esters, propylene glycol, and ethyl alcohol; and preservatives, such as methyl or propyl p-hydroxybenzoate and sorbic acid.

Such preparations also can be formulated as suppositories, e.g., containing conventional suppository bases, such as cocoa butter or other glycerides. Compositions for inhalation typically can be provided in the form of a solution, suspension or emulsion that can be administered as a dry powder or in the form of an aerosol using a conventional propellant, such as dichlorodifluoromethane or trichlorofluoromethane. Typically topical and transdermal formulations comprise conventional aqueous or nonaqueous vehicles, such as eye drops, creams, ointments, lotions, pastes, or in the form of medicated plaster, patch, or membrane.

Additionally, compositions for use in the methods described herein can be formulated for parenteral administration by injection or continuous infusion. Formulations for injection can be in the form of suspensions, solutions, or emulsions in oily or aqueous vehicles, and can contain formulation agents, such as suspending, stabilizing, and/or dispersing agents. Alternatively, the active ingredient can be in powder form for constitution with a suitable vehicle (e.g., sterile, hydrogen-free water) before use.

A composition for use in the methods described herein also can be formulated as a depot preparation. Such long acting formulations can be administered by implantation (for example, subcutaneously or intramuscularly) or by intra-muscular injection. Accordingly, the compounds of the invention can be formulated with suitable polymeric or hydrophobic materials (e.g., an emulsion in an acceptable oil), ion exchange resins, or as sparingly soluble derivatives (e.g., a sparing soluble salt).

Thus, described herein is a pharmaceutical composition comprising a PDE7 inhibitor, together with pharmaceutically acceptable diluents or carrier therefor. Described herein is a process of preparing a pharmaceutical composition comprising a PDE7 inhibitor, which process comprises mixing a PDE7 inhibitor, together with a pharmaceutically acceptable diluent or carrier therefor.

### Blood-Brain Barrier:

In some cases, the PDE7 inhibitory agent is administered so as to either pass through, or by-pass the blood-brain barrier. Preferably the inhibitory agent, compound or composition administered in the method of treatment can cross through the blood-brain barrier in sufficient quantities and at a sufficient rate so as to allow the treatment of the movement disorder. Methods for allowing agents to pass through the blood-brain barrier are known in the art, and include minimizing the size of the agent, providing hydrophobic factors which facilitate passage, and conjugation to a carrier molecule that has substantial permeability across the blood brain barrier.

In some cases, an effective amount of a PDE7 inhibitory agent is an amount that achieves a concentration within brain tissue at or above the IC₅₀ for activity of a given PDE7 inhibitory agent. In some cases, the PDE7 inhibitory agent is administered in a manner and dosage that gives a peak concentration of about 1, 1.5, 2, 2.5, 5, 10, 20 or more times the IC₅₀ concentration for inhibiting the greater of PDE7A or PDE7B.

In some instances, a PDE7 inhibitory agent or combination of agents is administered by a surgical procedure implanting a catheter coupled to a pump device. The pump device can be implanted or be extracorporally positioned. Administration of a PDE7 inhibitory agent can be in intermittent pulses or as a continuous infusion. Devices for injection to discrete areas of the brain are known in the art. In certain cases, the PDE7 inhibitory agent or a composition comprising a PDE7 inhibitory agent is administered locally to the ventricle of the brain, substantia nigra, striatum, locus ceruleus, nucleus basalis Meynert, pedunculopontine nucleus, cerebral cortex, and/or spinal cord by injection.

### EXAMPLES

### Example 1

This Example describes an assay for measuring the potency of PDE7 inhibitors and demonstrates the potency of PDE7 inhibition of several representative PDE7 inhibitory agents useful in the methods described herein.

### Methods:

The compounds listed in TABLE 1 were tested for inhibitory activity in a PDE7 phosphodiesterase assay performed using recombinant human PDE7A and 7B enzymes expressed in a baculoviral system. The recombinant human PDE7A enzyme was purchased from BPS Bioscience (Catalog #60070), Genbank Accession No. NM_002603 (amino acid 121-end) with an N-terminal GST tag, MW=66 kDa, expressed in a Baculovirus infected Sf9 cell expression system, with a specific activity of 302 pmol/min/µg. The recombinant human PDE7B enzyme was purchased from BPS Bioscience (Catalog #60071), Genbank Accession No. NM_018945 (amino acid 107-end), with an N-terminal GST tag, MW=66 kDa, expressed in a Baculovirus infected Sf9 cell expression system, with a specific activity of 53 pmol/min/µg.

**TABLE 1: PDE7 Inhibitory Compounds**

| **ID Number** | **Compound Reference Number** | **MW** |
|---|---|---|
| OM69 | 1 | 353.42 |
| OM056 | 2 | 353.42 |
| OM955 | 3 | 310.78 |
| OM956 | 4 | 330.45 |

### PDE7 Activity Assay:

The assay method used was a scintillation proximity assay (SPA) (obtained from GE Healthcare, Product Code TRKQ7100), with [³H]- cGMP as the substrate (SPA manual, Amersham Biosciences). Purified human PDE7A and PDE7B (obtained from BPS Bioscience, San Diego, CA) were diluted and stored in a solution containing 25 mM Tris-Cl (pH 8.0), 100 mM NaCl, 0.05% Tween 20, 50% glycerol, and 3 mM DTT. PDE7 assays were carried out in the following reaction mixture (final concentrations): 50 mM Tris-Cl (pH 8.0), 8.3 mM MgCl₂, 1.7 mM EGTA, 0.5 mg/ml BSA, 5% DMSO (except for OM056 which was in 2.5% DMSO) and 2 ng PDE7A or 0.2 ng PDE7B recombinant protein in a final volume of 0.1 mL.

For determination of IC₅₀ values against PDE7A or PDE7B, assays were run in duplicate on a single plate at eight concentrations of inhibitor, with a known PDE7 inhibitor (BRL50481) as a positive control. The inhibitor concentrations ranged from 0.61 nM to 10,000 nM, except in the case of OM056, for which the range was 0.24 nM to 4,000 nM. Reactions were initiated by addition of enzyme, incubated for 20 minutes at 30°C, and then terminated by the addition of 50 µl of SPA beads containing Zn²⁺.

The mixture was shaken, allowed to settle for 3 hours, then the production of [³H]-5'AMP from the substrate was quantitated by scintillation counting in a Wallac^{®} plate counter. The results in net cpm values were fitted to a four parameter logistic model using Excel Solver^{®}.

### Results:

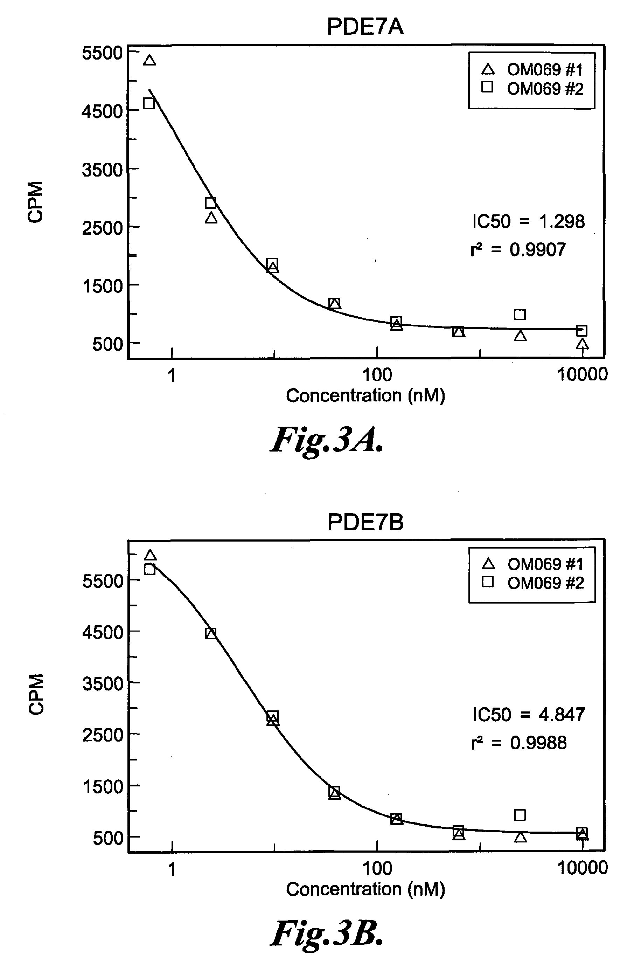

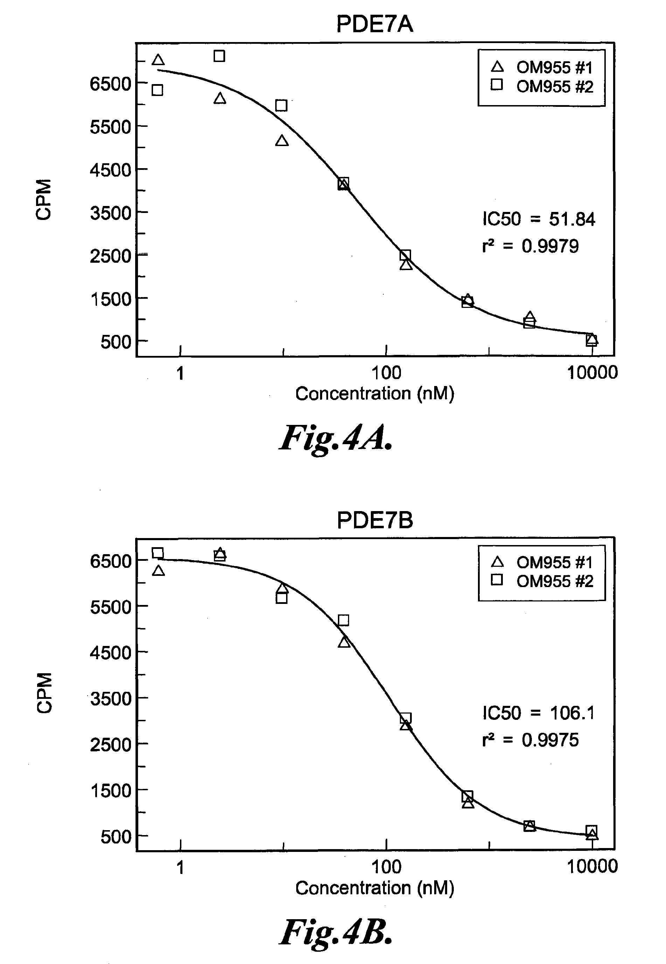

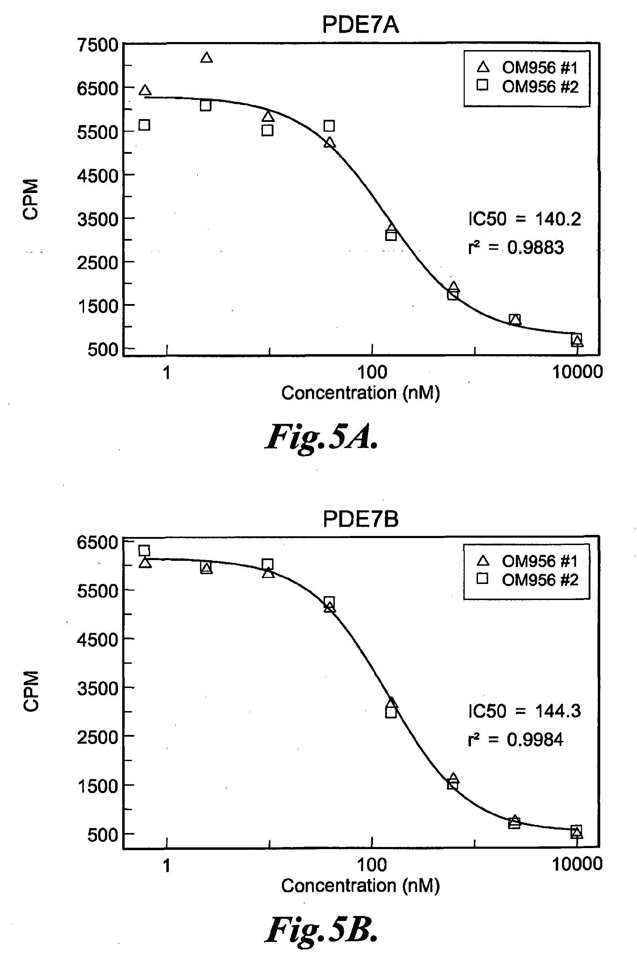

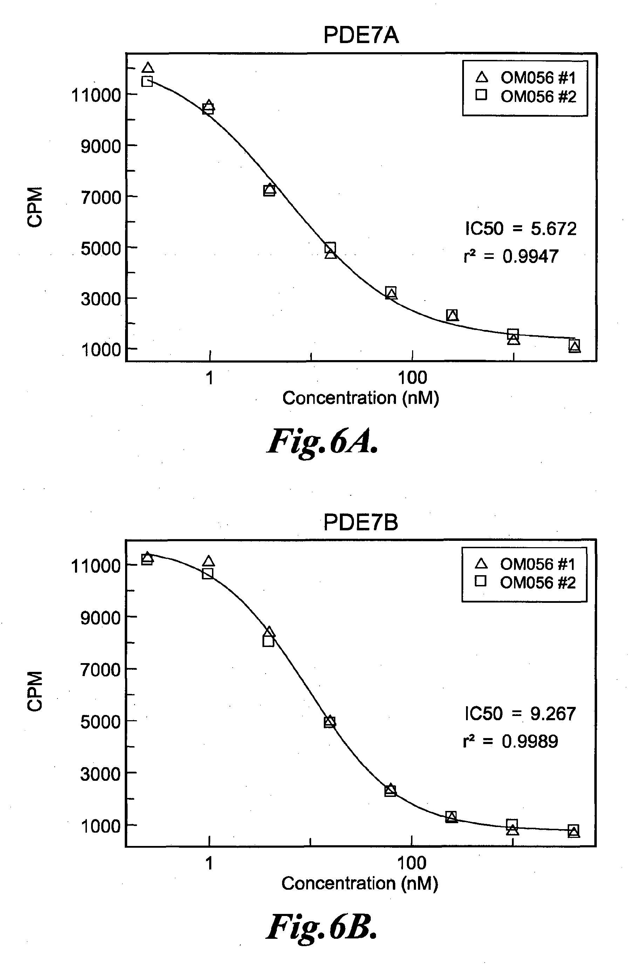

The results of the PDE7 phosphodiesterase enzyme inhibition assay are summarized below in TABLE 2, and shown in FIGURES 3A to 6B.

**TABLE 2: IC₅₀ Values for Representative PDE7 Inhibitory Compounds With Respect to PDE7A and PDE7B Inhibition**

| **Compound ID** | **Compound Number** | **PDE7A IC50** | **PDE7B IC50** | **7A/7B Ratio** |
|---|---|---|---|---|
| OM69 | 1 | 1.30 nM | 4.8 nM* | 3.69 |
| OM056 | 2 | 5.67 nM | 9.27 nM | 1.63 |
| OM955 | 3 | 51.8 nM | 106 nM | 2.05 |
| OM956 | 4 | 140 nM | 144 nM | 1.03 |

*OM69 was previously assayed by the inventors for inhibition of the activity of PDE7A and PDE7B. It is noted with regard to the IC₅₀ value for PDE7B inhibition with OM69 that in the initial assay, using a different assay methodology, the IC₅₀ value was determined to be 96.9 nM. In that initial assay, the background signal (counts per minute) was high relative to the maximal signal and the Hill coefficient was low, findings that call into question the reliability of the initial assay results.

The assay results shown above in TABLE 2, indicating an IC₅₀ value for PDE7B inhibition with OM69 of 4.8 nM is believed to be a more accurate value because, as shown in FIGURE 3B, the r² value for the fit to a 4- parameter logistic dose-response model is 0.9988, as would be expected for a simple competitive inhibitor. The IC50 value for PDE7A inhibition with OM69 in the initial assay work was 3.5 nM, not inconsistent with the value for PDE7A inhibition of 1.30 nM set forth in Table 2.

FIGURES 3A, 4A, 5A, and 6A are graphs illustrating the PDE7A inhibitory activity (IC₅₀), expressed as counts per minute ("CPM") versus the concentration of the representative PDE7 inhibitory agents OM69, OM955, OM956, and OM056, respectively, over a concentration range of 0.61 nM to 10,000 nM (for OM69, OM955 and OM956), or a concentration range of 0.24 nM to 4,000 nM (for OM056).

FIGURES 3B, 4B, 5B, and 6B are graphs illustrating the PDE7B inhibitory activity (IC₅₀), expressed as counts per minute ("CPM") versus the concentration of the representative PDE7 inhibitory agents OM69, OM955, OM956, and OM056, respectively, over a concentration range of 0.61 nM to 10,000 nM (for OM69, OM955 and OM956), or a concentration range of 0.24 nM to 4,000 nM (for OM056).

These results indicate that OM69 and OM056 inhibit both PDE7A and PDE7B with high potency, while OM955 and OM956 inhibit both these enzymes with moderate potency. The OM69, OM056, OM955 and OM956 compounds display little or no selectivity between PDE7A and PDE7B.

### Example 2

This Example describes a set of assays for measuring the selectivity of PDE7 inhibitors, and demonstrates that OM69 (compound 1), OM056 (compound 2), OM955 (compound 3), and OM956 (compound 4) each selectively inhibit PDE7 as compared to all other phosphodiesterase enzymes tested. These assays included a representative of every PDE family with the exception of PDE6.

### Methods:

Phosphodiesterase activity was measured by a scintillation proximity assay (SPA, GE Healthcare: Product Code TRKQ7100) with [³H]- cAMP as the substrate for PDEs 3A, 4A, 8A, or with [³H]-cGMP as the substrate for PDEs 1B, 2A, 5A, 9A, 10A and 11A. Purified human PDE1B, PDE2A, PDE3A, PDE4A, PDE5A, PDE8A, PDE9A and PDE11A4 were obtained from BPS Bioscience, San Diego, CA. Purified murine PDE10A was also obtained from BPS Biosciences. It is noted that murine PDE10A exhibits kinetic and inhibitory behavior that is indistinguishable from human PDE10. Therefore, the results obtained using murine PDE10A are believed to be representative of human PDE 10.

The purified PDE enzymes were each diluted and stored in a solution containing 25 mM Tris-Cl (pH 8.0), 100 mM NaCl, 0.05% Tween 20, 50% glycerol, and 3 mM DTT. PDE assays were carried out in the following assay cocktail (final concentrations): 50 mM Tris-Cl (pH 8.0), 8.3 mM MgCl₂, 1.7 mM EGTA, 0.5 mg/ml BSA, 2.5% DMSO and between 0.2 ng and 6 ng of the PDE protein (depending on the enzyme activity) in a final volume of 0.1 mL.

Assays were performed in duplicate at four concentrations (10 µM, 1.25 µM, 0.156 µM, and 0.019 µM) of the PDE7 inhibitors OM69 (compound 1), OM056 (compound 2), OM955 (compound 3) and OM956 (compound 4). Reactions were initiated by addition of enzyme, incubated for 20 minutes at 30°C and then terminated by the addition of 50 µl of SPA beads containing Zn²⁺.

The mixture was shaken, and allowed to settle for 3 hours. Then the production of [³H]-AMP or [³H]-GMP from the substrate was quantitated by scintillation counting in a Wallac^{®} plate counter. The results in net cpm values were fitted to a four parameter logistic model using Excel Solver^{®}. For calculation of selectivity ratios, the IC₅₀ values obtained with each enzyme were divided by the IC₅₀ values obtained with PDE7A in Example 1.

### Results:

TABLE 3 shows the results of selectivity assays with the four PDE7 inhibitory compounds assayed against the panel of PDEs tested. The values in TABLE 3 are shown in units of fold selectivity for PDE7A versus the other PDEs, and were determined by dividing the IC₅₀ value against the indicated PDE by the IC₅₀ value against PDE7A in Example 1. Thus, for example, the value of 342-fold for OM955 with PDE1B means that OM955 is 342-fold less potent as an inhibitor of PDE1B as compared to PDE7A. The numbers shown in parenthesis are IC₅₀ values against PDE7A of the various compounds from Example 1. The fold selectivity values provided in TABLE 3 as "greater than" reflect situations in which the highest concentration of compound inhibited the enzyme target only slightly (i.e., less than 50%), and higher concentrations could not be used because the compound became insoluble in the assay mixture. As a result, only a minimum estimate of selectivity could be generated.

**TABLE 3: PDE7A Selectivity of Representative PDE7 Inhibitory Compounds**

| **Target** | **OM69 (Compound 1) Fold Selectivity** | **OM056 (Compound 2) Fold Selectivity** | **OM955 (Compound 3) Fold Selectivity** | **OM956 (Compound 4) Fold Selectivity** |
|---|---|---|---|---|
| PDE1B | 29,608 | >1750 | 342 | >71 |
| PDE2A | 11,100 | 5670 | 209 | 546 |
| PDE3A | 8170 | >1750 | >192 | >71 |
| PDE4A | 6500 | 942 | 113 | >71 |
| PDE5A | 4630 | 3300 | 355 | >71 |
| PDE7A | 1 | 1 | 1 | 1 |
| | (1.30 nM) | (5.67 nM) | (51.8 nM) | (140 nM) |
| PDE7B | 3.7 | 1.6 | 2.0 | 1.0 |
| PDE8A | >7700 | >1750 | >192 | >71 |
| PDE9A | >7700 | >1750 | >192 | >71 |
| PDE10A | 1050 | 428 | 379 | 106 |
| PDE11A | 7070 | 2600 | >192 | >71 |

### Discussion of Results:

As shown above in TABLE 3, the representative PDE7 inhibitory agents OM69, OM056, OM955, and OM956 are all selective for PDE7A and PDE7B as compared to PDE1B, PDE2A, PDE3A, PDE4A, PDE5A, PDE8A, PDE9A, PDE10A, and PDE11A.

As shown in TABLE 3, OM69 (compound 1) is a potent inhibitor of both PDE7A (IC₅₀ = 1.3 nM) and PDE7B (IC₅₀ = 4.8 nM), displays a greater than 1000-fold selectivity for inhibition of the PDE7A enzyme as compared to the other PDEs, and a 250-fold selectivity for inhibition of the PDE7B enzyme as compared to the other PDEs.

As further shown in TABLE 3, OM056 (compound 2) is a potent inhibitor of both PDE7A (IC₅₀ = 5.7 nM) and PDE7B (IC₅₀ = 9.27), displays a greater than 400-fold selectivity for inhibition of the PDE7A enzyme as compared to the other PDES, and a greater than 200-fold selectivity for inhibition of the PDE7B enzyme as compared to the other PDEs.

As further shown in TABLE 3, OM955 (compound 3) is a moderately potent inhibitor of both PDE7A (IC₅₀ = 51.8 nM) and PDE7B (IC₅₀ = 106 nM), and displays a greater than 100-fold selectivity for inhibition of the PDE7A enzyme as compared to the other PDEs, and a greater than 50-fold selectivity for inhibition of the PDE7B enzyme as compared to the other PDEs.

As also shown in TABLE 3, OM956 (compound 4) is a moderately potent inhibitor of both PDE7A (IC₅₀ = 140 nM) and PDE7B (IC₅₀ = 144 nM), and displays a greater than 71-fold selectivity for inhibition of the PDE7A and PDE7B enzymes as compared to the other PDEs.

These results, taken together with the results described in Examples 4-7, demonstrate that inhibition of PDE7 activity is necessary and sufficient for the improvement in movement abnormalities observed after administration of these compounds in the MPTP mouse model. Moreover, the use of selective inhibitors of PDE7 rather than non-selective PDE inhibitors provides the advantage of less toxicity, because they will not significantly inhibit PDEs known to cause unwanted effects, such as PDE3 and PDE4.

### Example 3

This Example describes a method for evaluating the metabolic stability of PDE7 inhibitors and the ability of PDE7 inhibitors to partition into mouse brain *in vivo.*

### Animal Testing Protocol

*Mouse sprain:* Adult male C57BL/6J; retired breeders, aged between 7-9 months (Jackson Laboratory, Bar Harbor, ME, USA), singly housed.

*Compound administration:* Each PDE7 inhibitor (OM69 (compound 1), OM056 (compound 2), OM955 (compound 3), and OM956 (compound 4)) was dissolved in a vehicle consisting of DMSO, 0.03 M phosphoric acid and Tween 80 (5:95:0.2) and injected via the intraperitoneal route at a dose of 10 mg/kg.

*Collection of blood and brain samples:* Just prior to the harvesting of tissues, mice were anesthetized with avertin. Samples of whole brain or plasma were collected from three animals per time point at 15 minutes, 30 minutes, 60 minutes, 120 minutes, and 240 minutes after injection. Blood samples were collected by retro-orbital bleeding. Plasma was prepared and red cells were removed by centrifugation. The mice were perfused with saline to remove contaminating blood. The brains were removed and rapidly frozen in liquid nitrogen for subsequent analysis. The plasma and brain samples were stored at -80°C or on dry ice and the concentration of compound was determined by LC/MS/MS analysis as follows:
Whole brain tissue was homogenized using a Mini-Beaddeater-8TM (BioSpec Products, Bartlesville, OK). Plasma or homogenized brain samples were precipitated with acetonitrile and filtered in 96-well format using Captiva^{®} 0.20 micron filtration plates (Varian Corp, Lake Forest, CA). Filtrates were evaporated under nitrogen to dryness at 50°C and were then reconstituted for LC-MS analysis.
Quantitative measurements for inhibitory compound in plasma and brain samples were obtained using a Thermo Ultra triple quadrupole mass spectrometer (Thermo Fisher Scientific, San Jose, CA), utilizing electrospray ionization in the multiple reaction monitoring mode of data acquisition. Sample extracts were injected onto a 2.1 x 30 mm packed Xterra C18-MS high pressure liquid chromatography (HPLC) column (Waters Corp, Milford, MA). The mobile phase eluting the compound from the HPLC column was applied using a Thermo Surveyor MS Plus HPLC quaternary pumping system (Thermo Fisher Scientific, San Jose, CA) and a HTC PAL autoinjector (LEAP Technologies, Chapel Hill, NC).

### Results:

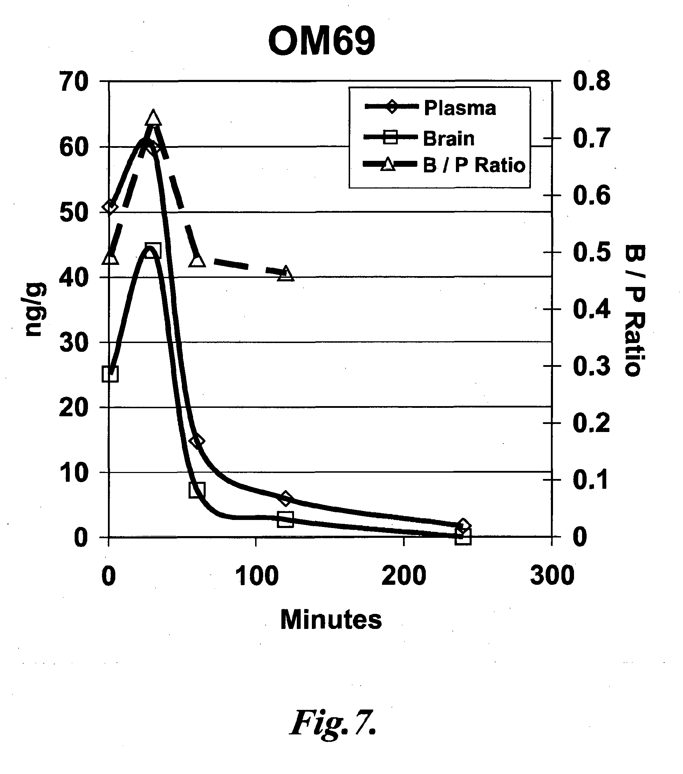

The data on tissue concentration of OM69 (compound 1), OM056 (compound 2), OM955 (compound 3) and OM956 (compound 4) were collected from 3 mice per time point and averaged. FIGURE 7 graphically illustrates the plasma concentration, the brain concentration, and the brain/plasma ratio of compound OM69 (compound 1) over a 240 minute time course. As shown in FIGURE 7, within the first 15 minutes of exposure, OM69 was detected in plasma (51 ng/g) and brain tissue (25 ng/g). Peak levels were seen at 30 minutes post IP injection (plasma 59 ng/g and brain 44 ng/g) after which both plasma and brain levels dropped rapidly. At all time points, when both brain and plasma levels were greater than the lower limit of quantitation in the LC/MS/MS assay (2 ng/g), brain to plasma ratios were 45-75%.

Using the approach described for compound OM69, a ratio was calculated of the total exposure in brain (area under the curve: AUC) divided by the total exposure in plasma for each compound tested. The results are shown below in TABLE 4. Ratio values greater than "1" indicate that the compound was concentrated in the brain. Values that are shown as ">1" indicate that the compound levels were already high in the brain by the time the first measurement was taken. As a result, the ratio of 1 represents a minimum estimate in these cases.

**TABLE 4: Brain/Plasma Ratio of PDE7 Inhibitory Compounds**

| **Compound** | **Ratio of Total Brain Exposure/Total Plasma Exposure** |
|---|---|
| OM69 (compound 1) | 0.78 |
| OM056 (compound 2) | 1.8 |
| OM955 (compound 3) | >1 |
| OM956 (compound 4) | >1 |

### Discussion of Results:

The data shown in FIGURE 7 suggests that OM69 (compound 1) reaches concentrations in the brain of 44 ng/g, which converts to 124 nM at 30 minutes after injection of a 1 mg/kg dose. Assuming linearity of uptake and uniform brain distribution, doses of 0.1 mg/kg to a mouse would be expected to yield maximal concentrations of 12.4 nM, which is sufficient to achieve PDE7 inhibition in the brain based on the IC₅₀ values of 1.3 nM and 4.8 nM reported in Example 1. The analogous calculations for the three other compounds tested in the MPTP model (as described in Examples 5 to 7), yield the following results for maximal brain concentrations at doses and time points where efficacy was observed: OM955: 241 nM at 0.5 mg/kg; OM956: 328 nM at 0.5 mg/kg; and OM056: 71 nM at 0.5 mg/kg. In each case, it is noted that these levels are at least 2-fold in excess of the greater of the IC₅₀ values of the compounds to inhibit PDE7A or PDE7B.

### Example 4

This Example demonstrates that the representative PDE7 inhibitor OM69 (compound 1) does not interact significantly with known Parkinson's disease targets.

### Methods:

A representative PDE7 inhibitor, OM69 (compound 1) was tested against a panel of known Parkinson's disease targets to determine whether there was evidence of interaction with any of the tested targets.

### Assays:

### 1. Catechol-O-Methyltransferase (COMT) Enzyme Assay

Catechol-O-Methyltransferase (COMT) was assayed as described in Zurcher, G., et al., J. Neurochem 38:191-195, 1982, with the following modifications. The source of COMT was isolated from pig liver. The substrate was 3 mM Catechol + S-Adenosyl-L-[methyl-³H]methionine. The vehicle control was 1% DMSO. Compound OM69 was tested at 10 µM in incubation buffer (100 mM Potassium Phosphate, 10 mM MgCl₂, 3 mM DTT containing 12 U/ml ADA, pH 7.4). The reaction was pre-incubated for 15 minutes at 37°C, COMT enzyme was added, and the reaction was incubated for 60 minutes at 37°C. [³H]-Guaiacol was counted and quantitated. The positive control reference compound 3,5-Dinitrocatechol was run in this assay, with a historical IC₅₀ value of 0.31 µM.

### 2. Monoamine Oxidase MAO-B Enzyme Assay

Monoamine Oxidase MAO-B was assayed as described in Urban, P., et al., FEBS Lett 286(1-2):142-146, 1991, with the following modifications. Human recombinant MAO-B was isolated from insect Hi5 cells. The substrate was 50 µM Kynuramine. The vehicle control was 1% DMSO. Compound OM69 was tested at 10 µM in incubation buffer (100 mM potassium phosphate, pH 7.4) in Experiment 1. In Experiment 2, compound OM69 was tested at 10 µM and 1 µM. The reaction was pre-incubated for 15 minutes at 37°C, MAO-B enzyme was added, and the reaction was incubated for 60 minutes at 37°C. Spectrofluorimetric quantitation of 4-hydroxyquinoline was carried out. The positive control reference compound R(-)-Deprenyl was run in this assay, with a historical IC₅₀ value of 5.3 nM.

### 3. Tyrosine Hydroxylase Enzyme Assay

Tyrosine Hydroxylase was assayed as described in Roskoski, R., Jr., et al., J. Biochem 218:363-370 (1993) with the following modifications. Tyrosine Hydroxylase was isolated from Wistar rat brain. The substrate was 100 µM L-[3,5-³H]Tyrosine + L-Tyrosine. The vehicle control was 1% DMSO. Compound OM69 was tested at 10 µM in incubation buffer (125 mM MES, pH 6.0, 0.5 mg/ml Catalase, 12.5 mM DTT, 0.5 mM (6R)-5,6,7,8-Tetrahydrobiopterin). The reaction was pre-incubated for 15 minutes at 37°C. Tyrosine Hydroxylase enzyme was added, and the reaction was incubated for 20 minutes at 37°C. [³H]-H₂O was quantitated. The positive control reference compound α-Methyl-L-P-Tyrosine was run in this assay, with a historical IC₅₀ value of 20 µM.

### 4. Dopamine D₁ Radioligand Binding Assay

A Dopamine D₁ radioligand binding assay was carried out as described in Dearry, A., et al., Nature 347:72-76, 1990, with the following modifications. Human recombinant Dopamine D1 receptor was expressed in CHO cells. The ligand used in the assay was 1.4 nM [³H] SCH-23390. The non-specific ligand was 10 µM (+)-butaclamol. The vehicle control was 1% DMSO. Compound OM69 was tested at 10 µM in incubation buffer (50 mM Tris-HCL, pH 7.4, 1.4 mM Ascorbic Acid, 0.001% BSA, 150 mM NaCl). The reaction was incubated for 2 hours at 37°C. Radioligand binding was quantitated. The positive control reference compound R(+)-SCH-23390 was run in this assay, with a historical IC₅₀ value of 1.4 nM.

### 5. Dopamine D_{2L} Radioligand Binding Assay

A Dopamine D_{2L} radioligand binding assay was carried out as described in Grandy, D.K., et al., PNAS 86:9762-9766, 1989, with the following modifications. Human recombinant Dopamine D_{2L} receptor was expressed in CHO cells. The ligand used in the assay was 0.16 nM [³H]-spiperone. The non-specific ligand was 10 µM Haloperidol. The vehicle control was 1% DMSO. Compound OM69 was tested at 10 µM in incubation buffer (50 mM Tris-HCL, pH 7.4, 1.4 mM Ascorbic Acid, 0.001% BSA, 150 mM NaCl). The reaction was incubated for 2 hours at 25°C. Radioligand binding was quantitated. The positive control reference compound spiperone was run in this assay, with a historical IC₅₀ value of 0.26 nM.

### 6. Dopamine D₃ Radioligand Binding Assay

A Dopamine D₃ radioligand binding assay was carried out as described in Sokoloff, P., et al., Nature 347:146-151, 1990, with the following modifications. Human recombinant Dopamine D₃ receptor was expressed in CHO cells. The ligand used in the assay was 0.7 nM [³H]-spiperone. The non-specific ligand was 25 µM S(-)Sulpiride. The vehicle control was 1% DMSO. Compound OM69 was tested at 10 µM in incubation buffer (50 mM Tris-HCL, pH 7.4, 1.4 mM Ascorbic Acid, 0.001% BSA, 150 mM NaCl). The reaction was incubated for 2 hours at 37°C. Radioligand binding was quantitated. The positive control reference compound spiperone was run in this assay, with a historical IC₅₀ value of 0.36 nM.

### 7. Dopamine D_{4.2} Radioligand Binding Assay

A Dopamine D_{4.2} radioligand binding assay was carried out as described in Van tol, H.H.M., et al., Nature 350:610-614, 1991, with the following modifications. Human recombinant Dopamine D_{4.2} receptor was expressed in CHO-K1 cells. The ligand used in the assay was 0.5 nM [³H]-spiperone. The non-specific ligand was 10 µM Halperidol. The vehicle control was 1% DMSO. Compound OM69 was tested at 10 µM in incubation buffer (50 mM Tris-HCL, pH 7.4, 1.4 mM Ascorbic Acid, 0.001% BSA, 150 mM NaCl). The reaction was incubated for 2 hours at 25°C. Radioligand binding was quantitated. The positive control reference compound spiperone was run in this assay, with a historical IC₅₀ value of 0.5 nM.

### 8. Dopamine D₅ Radioligand Binding Assay

A Dopamine D₅ radioligand binding assay was carried out as described in Sunahara, R.K., et al., Nature 350:614-619, 1991, with the following modifications. Human recombinant Dopamine D₅ receptor was expressed in CHO cells. The ligand used in the assay was 2 nM [³H]-SCH-23390. The non-specific ligand was 10 µM Flupentixol. The vehicle control was 1% DMSO. Compound OM69 was tested at 10 µM in incubation buffer (50 mM Tris-HCL, pH 7.4, 1.4 mM Ascorbic Acid, 0.001% BSA, 150 mM NaCl). The reaction was incubated for 2 hours at 37°C. Radioligand binding was quantitated. The positive control reference compound R(+)-SCH23390 was run in this assay, with a historical IC₅₀ value of 1.5 nM.

### 9. Gabapentin Radioligand Binding Assay

A Gabapentin radioligand binding assay was carried out as described in Gee, N.S., et al., J. Biol. Chem. 271(10):5768-5776, 1996, with the following modifications. Gabapentin was obtained from Wistar Rat brain cortex. The ligand used in the assay was 0.02 µM [³H] Gabapentin. The non-specific ligand was 100 µM Gabapentin. The vehicle control was 1% DMSO. Compound OM69 was tested at 10 µM in incubation buffer (10 mM HEPES, pH 7.4). The reaction was incubated for 30 minutes at 25°C. Radioligand binding was quantitated. The positive control reference compound Gabapentin was run in this assay, with a historical IC₅₀ value of 0.04 µM.

### 10. Dopamine Transporter (DAT) Radioligand Binding Assay

A Dopamine Transporter (DAT) radioligand binding assay was carried out as described in Giros, B., et al., Trends Pharmacol Sci 14:43-49, 1993, with the following modifications. Human recombinant DAT was expressed in CHO-K1 cells. The ligand used in the assay was 0.15 nM [¹²⁵I]-RTI-55. The non-specific ligand was 10 µM Nomifensine. The vehicle control was 1% DMSO. Compound OM69 was tested at 10 µM in incubation buffer (50 mM Tris-HCL, pH 7.4, 100 mM NaCl, 1 µM Leupeptin, 10 µM PMSF). The reaction was incubated for 3 hours at 4°C. Radioligand binding was quantitated. The positive control reference compound GBR-12909 was run in this assay, with a historical IC₅₀ value of 1.7 nM.

### 11. Adenosine A_{2A} Receptor Radioligand Binding Assay

An Adenosine A_{2A} receptor radioligand binding assay was carried out as described in Varani, K., et al., Br. J. Pharmacol. 117:1693-1701, 1996, with the following modifications. Human recombinant A_{2A} receptor was expressed in HEK-293 cells. The ligand used in the assay was 0.05 µM [³H]CGS-21680. The non-specific ligand was 50 µM NECA. The vehicle control was 1% DMSO. Compound OM69 was tested at 10 µM in incubation buffer (50 mM Tris-HCL, pH 7.4, 10 mM MgCl₂, 1 mM EDTA, 2 U/mL Adenosine Deaminase). The reaction was incubated for 90 minutes at 25°C. Radioligand binding was quantitated. The positive control reference compound CGS-21680 was run in this assay, with a historical IC₅₀ value of 0.13 µM.

### Results:

The results of the assays described above are summarized below in TABLE 5. The amount of OM69 used in the assay is indicated in parentheses in the column labeled "OM69." The results are presented in terms of percent inhibition of ligand binding to, or enzymatic activity of, the target. Positive controls were included in each assay as indicated, as a check on the assay's performance.

**TABLE 5: Level of Inhibition of Known Parkinson's Disease Targets With Representative PDE7 Inhibitory Agent OM69 (Compound 1)**

| **Target** | **OM69 (compound 1)** | **Positive Control** | |
|---|---|---|---|
| | | **Expected IC₅₀** | **Observed IC₅₀** |
| Catechol-O-Methyltransferase (COMT) | 19.0% (10.0 µM) | 0.31 µM | 0.606 µM |
| Monoamine Oxidase MAO-B (Exp #1) | 49.0% (10.0 µM) | 5.3 nM | 6.69 nM |
| Monoamine Oxidase MAO-B (Exp #2) | 67.0% (10.0 µM) | 5.3 nM | 9.96 nM |
| Monoamine Oxidase MAO-B (Exp #2) | 27.0% (1.0 µM) | 5.3 nM | 9.96 nM |
| Tyrosine Hydroxylase | 4.0% (10.0 µM) | 20 µM | 20.2 µM |
| Dopamine D₁ | -5.0% (10.0 µM) | 1.4 nM | 1.83 nM |
| Dopamine D_{2L} | 4.0% (10.0 µM) | 0.26 nM | 0.277 nM |
| Dopamine D₃ | -13.0% (10.0 µM) | 0.36 nM | 0.774 nM |
| Dopamine D_{4.2} | -16.0% (10.0 µM) | 0.5 nM | 0.441 nM |
| Dopamine D₅ | 4.0% (10.0 µM) | 1.5 nM | 2.31 nM |
| Gabapentin | 15.0% (10.0 M) | 0.04 µM | 0.0796 µM |
| Dopamine Transporter (DAT) | 18.0% (10.0 µM) | 1.7 nM | 0.975 nM |
| Adenosine A_{2A} Receptor | 41.0% (10.0 µM) | 0.13 µM | 0.0924 µM |

### Discussion of Results:

The results shown in TABLE 5 demonstrate that, even at a concentration that is 2000-fold greater than its IC₅₀ for PDE7B, the representative PDE7 inhibitor OM69 does not substantially inhibit COMT, tyrosine hydroxylase, the dopamine transporter, the gabapentin receptor, or any of the dopamine receptor subtypes.

With regard to the Adenosine A_{2A} receptor result, although the results in TABLE 5 show that OM69 inhibited binding to A_{2A} receptors by 41% at a concentration of 10 µM, it is believed that A_{2A} is not a target of OM69 for at least the following reasons. As shown in Example 3, OM69 reaches levels in the mouse brain of 30 to 60 ng/g after a dose of 1 mg/kg. Assuming linear pharmacokinetics, then a dose of 0.1 mg/kg, which is highly effective in the mouse MPTP model, as demonstrated in Examples 5 and 6, would produce levels in the brain of 3 to 6 ng/g, which is equivalent to 3 to 6 ng/ml or 8.5 to 17 nM. At this concentration, using a conservative value of IC₅₀ for OM69 of 10 µM, the percent occupation of A_{2A} receptors would be only about 0.17%. Therefore, the conclusion is that A_{2A} is not a target for OM69.

With regard to the MAO-B assay, a percentage inhibition of 27% at a concentration of 1 µM would indicate an IC₅₀ value of approximately 2.7 µM. Therefore, if OM69 were present in the brain at a concentration of 17 nM (as discussed in the previous paragraph), it would inhibit MAO-B by less than 0.7%. Therefore, the conclusion is that MAO-B is not a target for OM69.

### Example 5

### Pharmacological Evaluation of a Representative PDE7 Inhibitory Compound in the MPTP Model of Parkinson's Disease

In this Example, representative PDE7 inhibitory agent, OM69 (compound 1), was evaluated in an initial study in the mouse MPTP model of Parkinson's disease.

### Animal Testing Protocol

*Mouse strain:* Adult male C57BL/6J; retired breeders, aged between 7-9 months (Jackson Laboratory, Bar Harbor, ME, USA), singly housed.

*Handling:* Animals were handled daily for 2 weeks prior to injection in order to make them amenable to behavioral testing. All mice were maintained on a standard 12-h light/dark cycle and given *ad libitum* access to lab chow and water.

*MPTP administration:* Mice received 2 subcutaneous injections of methylphenyltetrahydropyridine ("MPTP") at a dose of 15 mg/kg (free base) with a 10-12 hour interval between injections. Control mice (sham lesion groups) were administered saline instead of MPTP.

*Administration of PDE7 inhibitory compound:* Seven days after MPTP or saline administration, OM69 was administered to mice. For each dose, a 100x stock of OM69 compound was prepared in 100% DMSO and subsequently diluted 100x into phosphate buffered saline (PBS). Then 100 µL of this 1X solution was administered via intraperitoneal (IP) injection.

It was previously determined from pharmacokinetic studies that OM69 is washed out of mice within 12 hours, so dosing of various test compounds on consecutive days was used to maximize the information that could be obtained from each group of lesioned animals.

*Stride length:* Prior to injection with MPTP or saline, animals were pre-trained to walk across a clean sheet of paper into their home cage without stopping. For assessment of stride length, animals had their forepaws placed in black ink and the length of forepaw steps during normal walking (in a straight line only) was measured. The animals were immediately put back into their home cage upon completion of the task. Stride length was determined by measuring the distance between each step on the same side of the body, measuring from the middle toe of the first step to the heel of the second step. An average of at least four clear steps was calculated.

### Experimental Protocol

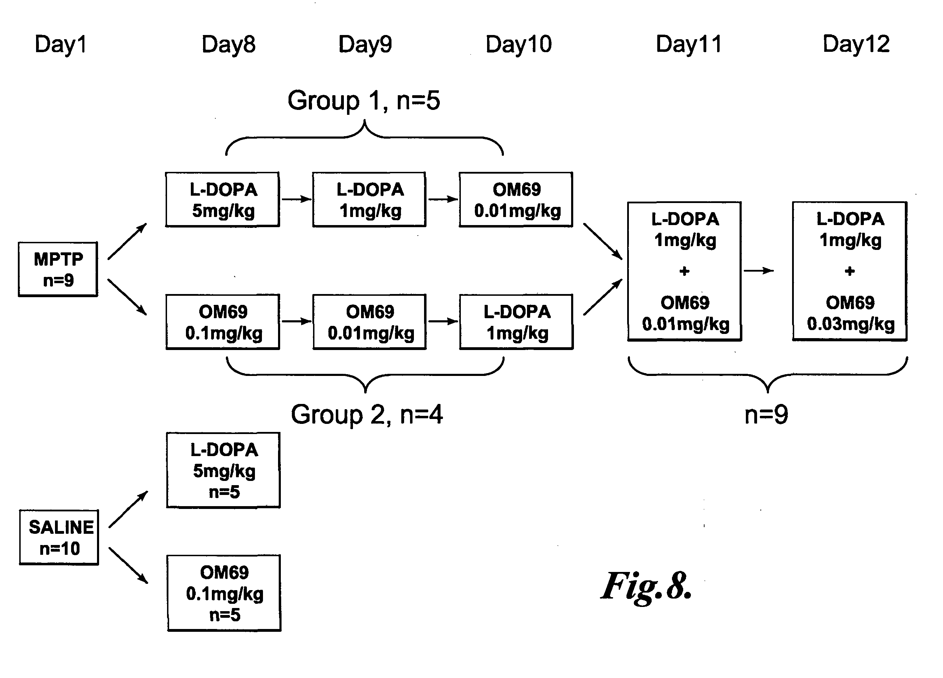

Retired breeder male mice (12-15 per group, ~48-60 animals total) were treated with either saline or 2 x 15 mg/kg MPTP (12 saline; 36 MPTP) to induce a Parkinsonian state (neurochemical loss of dopamine with corresponding behavioral deficits). A flow chart schematically illustrating the protocol is shown in FIGURE 8.

As shown in FIGURE 8, on day one baseline stride length was measured and then mice were treated with either MPTP (n=9) or with saline as a control (n=10). On day eight stride length was measured again, and the control value for stride length was derived from the stride length of saline-treated animals. Following this measurement the first treatments with L-dopa (5 mg/kg) or OM69 (0.1 mg/kg) were administered on day eight, as shown in FIGURE 8. Stride length was then measured for each animal 20 minutes after dosing. Neither L-dopa (5 mg/kg) nor OM69 (0.1 mg/kg) altered the stride length of saline-treated mice. Dosing with the treatments shown in FIGURE 8 was carried out on successive days, with stride length measured 20 minutes after each dosing. As mentioned above, it was previously determined from pharmacokinetic studies that OM69 is washed out of the mice within 12 hours after dosing, so the stride length test carried out 20 minutes after dosing was believed to be measuring the effect of only the compounds administered on the day of treatment.

As shown in FIGURE 8, on day eight Group 1 animals were treated with L-dopa (5 mg/kg) and Group 2 animals were treated with OM69 (0.1 mg/kg). On day nine Group 1 animals were treated with L-dopa (1 mg/kg), and Group 2 animals were treated with OM69 (0.01 mg/kg). On day ten the treatment groups were crossed over, and Group 1 animals were treated with OM69 (0.01 mg/kg), and Group 2 animals were treated with L-dopa (1 mg/kg).

As further shown in FIGURE 8, for the combination studies, all the mice in Group 1 and Group 2 were combined. On day 11 following MPTP administration, all the animals in Group 1 and Group 2 (n=9) were treated with the combination of L-dopa (1 mg/kg) and OM69 (0.01 mg/kg), with the stride length test carried out 20 minutes after dosing. On day 12 following MPTP administration, all the animals in Group 1 and Group 2 (n=9) were treated with the combination of L-dopa (1 mg/kg) and OM69 (0.03 mg/kg) with the stride length test carried out 20 minutes after dosing. In these combination studies carried out on day 11 and day 12, the average stride length data from two of the nine animals was not scorable because these two animals either ran rather than walking at a normal speed, or they kept starting and stopping rather than walking continuously. Therefore, these two animals were excluded, and only the remaining seven animals (n=7) were scored for the combination studies.

### Stride Length Increase

The MPTP model used in this Example is a generally accepted mouse model of PD that is viewed by those of skill in the art as being predictive for PD in humans (Tillerson, J.L., et al., Exp. Neurol. 178(1):80-90, 2002; Tillerson, J.L., et al., J. Neurosci Methods 123(2):189-200, 2003).

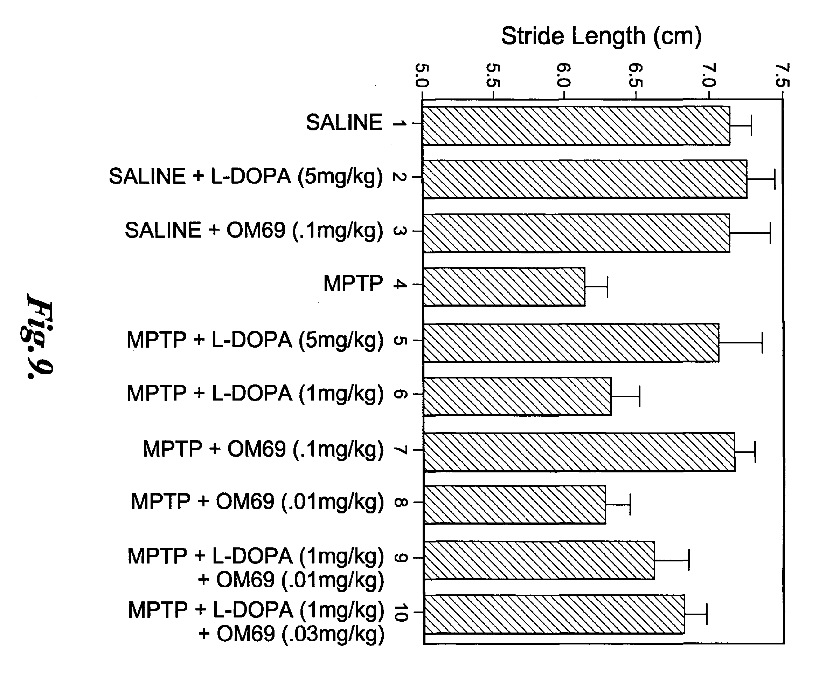

To assess and compare the effects of OM69 and L-dopa at these doses, data from mice with equivalent dosing was pooled. Thus, the average stride length for animals treated with L-dopa (1 mg/kg) was determined using measurements obtained from all nine animals. Similarly, the average stride length for animals treated with OM69 (0.01 mg/kg) was determined using measurements obtained from all nine animals. The results of the MPTP study described in this Example are provided in FIGURES 9-11.

FIGURES 9-11 are bar graphs illustrating the results of the testing of inked paw stride length. They demonstrate that representative PDE7 inhibitor OM69 (compound 1) increases stride length in MPTP lesioned mice and does so at significantly lower doses as compared to L-dopa.

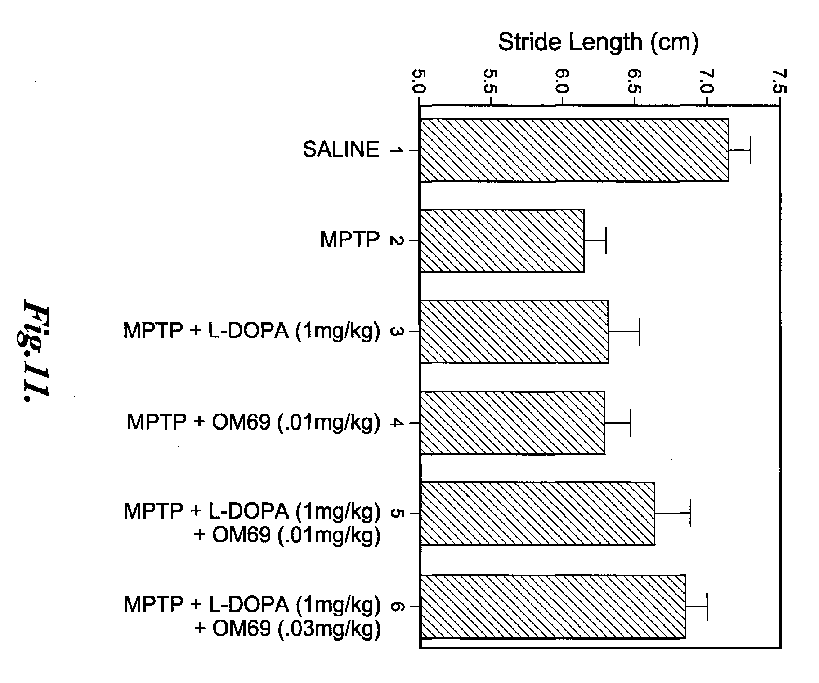

As shown in FIGURES 9 and 11, treatment of mice with MPTP decreases their stride length as measured from tracks left by inked mouse paws, in comparison with saline controls. See FIGURE 9 (#1 versus #4) and FIGURE 11 (#1 versus #2).

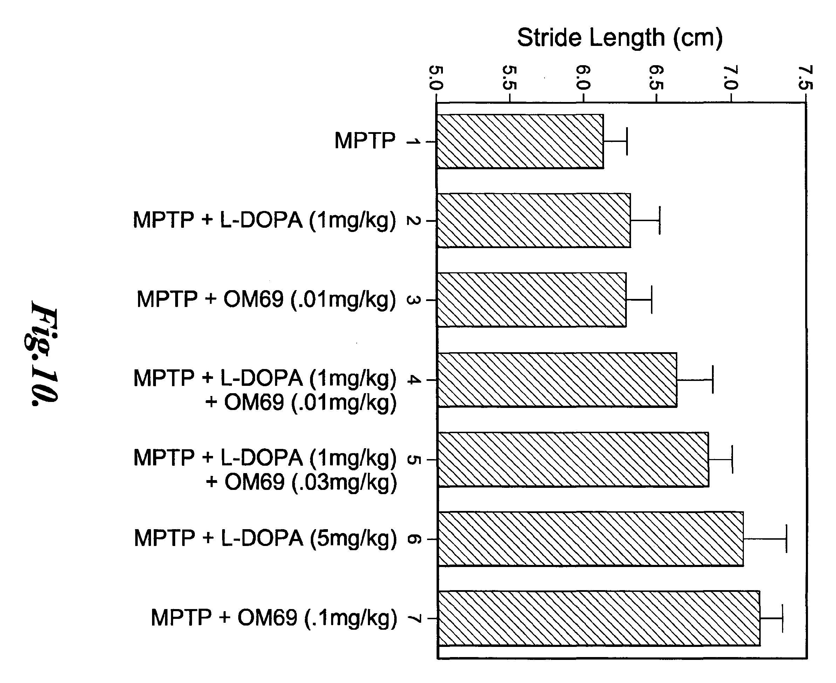

As shown in FIGURES 9 and 10, treatment of MPTP-treated mice with L-dopa increases their stride length in a dose-dependent manner. See FIGURE 9 (#4 versus #5 and #6) and FIGURE 10 (#1 versus #2 and #6).

It was unexpectedly determined that representative PDE7 inhibitor OM69 (compound 1), when administered IP at 0.1 mg/kg, has the same effect to increase stride length in the MPTP-treated mice as a 50-fold higher dose of L-dopa (5 mg/kg). As shown in FIGURE 9, at the above noted concentrations, both L-dopa (#5) and OM69 (#7) fully restore the stride length to that seen without MPTP treatment (#1). These results demonstrate the surprising finding that OM69 (compound 1), a representative PDE7 inhibitor useful in the method of the invention, is effective to increase stride length in MPTP mice at a significantly lower dose as compared to L-dopa.

As shown in FIGURES 9-11, at lower doses of L-dopa (1 mg/kg) (see FIGURE 9, #6; FIGURE 10, #2) or OM69 (0.01 mg/kg) (see FIGURE 9 #8; FIGURE 10, #3) there was only a small effect observed on the stride length of MPTP-treated mice. However, when these lower doses of OM69 and L-dopa were administered in combination, the increase in stride length trended toward a greater effect than the sum of the increases seen from either drug alone. See FIGURE 9, #9, and FIGURE 10 #4. These results demonstrate that the administration of representative PDE7 inhibitor OM69 (compound 1) is effective to increase stride length in MPTP lesioned mice at a significantly lower dose as compared to L-dopa, and is therefore useful in the methods of the invention directed to the treatment of a movement abnormality associated with the pathology of a movement disorder such as Parkinson's disease.

### Example 6

### Pharmacological Evaluation of a Representative PDE7 Inhibitory Compound in the MPTP Model of Parkinson's Disease: Confirmatory Study

In this Example, a representative PDE7 inhibitor, OM69 (compound 1), was evaluated in the mouse MPTP model of Parkinson's disease. This study was designed to confirm the findings in Example 5 and to provide statistical proof of the effects of OM69.

### Animal Tasting Protocol.

*Mouse strain:* Adult male C57BL/6J; retired breeders, aged between 7-9 months (Jackson Laboratory, Bar Harbor, ME, USA), singly housed.

*Handling:* Animals handled daily for 2 weeks prior to injection, in order to make them amenable to behavioral testing. All mice were maintained on a standard 12-h light/dark cycle, and given *ad libitum* access to lab chow and water.

*MPTP Administration:* MPTP Mice received 2 subcutaneous injections of methylphenyltetrahydropyridine ("MPTP") at a dose of 15 mg/kg (free base) with a 10-12 hour interval between injections. Control mice (sham lesion groups) were administered saline instead of MPTP.

*OM69 (compound 1) Administration:* Seven days after MPTP / saline administration, OM69 was administered to mice. For each dose, a 100x stock of OM69 was prepared in 100% DMSO and subsequently diluted 100x into phosphate buffered saline (PBS). Then 100 µL of this 1X solution was administered intraperitoneally (IP).

*Stride Length:* Prior to injection with MPTP or saline, animals were pre-trained to walk across a clean sheet of paper into their home cage without stopping. For assessment of stride length, animals had their forepaws placed in black ink and the length of forepaw steps during normal walking (in a straight line only) was measured. The animals were immediately put back into their home cage upon completion of the task. Stride length was determined by measuring the distance between each step on the same side of the body, measuring from the middle toe of the first step to the heel of the second step. An average of at least four clear steps was calculated.

### Statistical Analysis

Data was analyzed using Prism 4.0 software. Groups were analyzed by one way ANOVA and Student-Newman-Keuls post hoc test when appropriate. Significance was considered reached at p < 0.05. Treatment groups in the stride length graph that are statistically different are indicated by the letters in Table 6. Groups that share a letter are not statistically different; groups that do not share a letter are significantly different from each other.

### Experimental Protocol:

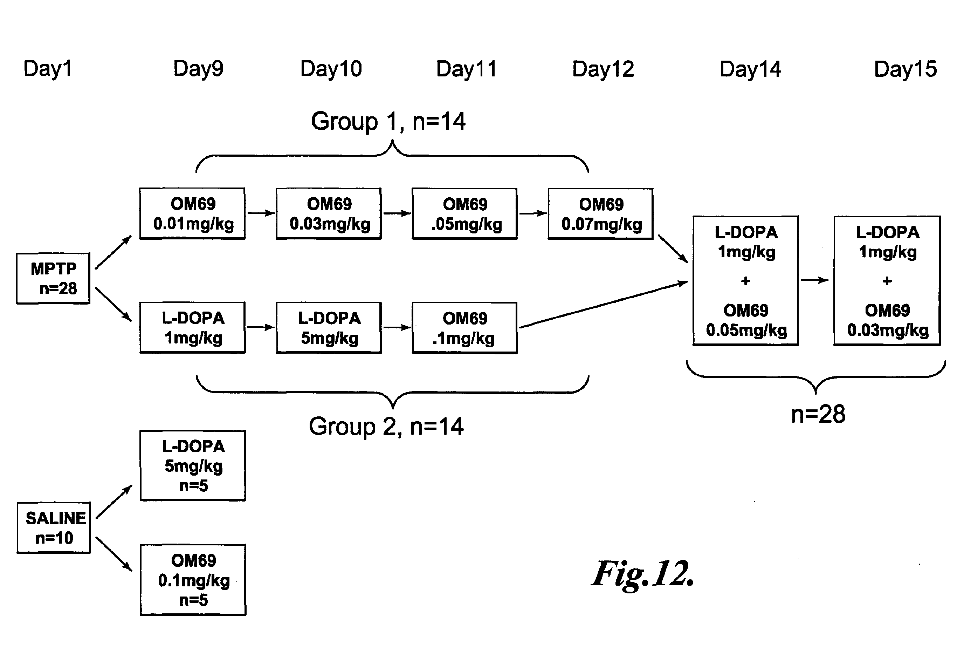

Retired breeder male mice (28 animals total) were treated with 2 x 15 mg/kg MPTP (12 saline; 36 MPTP) to induce a Parkinsonian state (neurochemical loss of dopamine with corresponding behavioral deficits). A flow chart schematically illustrating the protocol is shown in FIGURE 12. On day 1 baseline stride length was measured and then all mice were treated with MPTP or saline. On day 8 stride length was measured again, and then the MPTP-treated animals were divided randomly into two groups of fourteen. As described in Example 5, dosing with the successive treatments indicated in FIGURE 12 occurred on consecutive days, and in each case stride length was measured 20 minutes after dosing. The "n" values inside the boxes in the flow chart indicate the number of animals for which useful data was obtained on that day (occasionally, a few animals failed to perform the stepping task).

On Day 9, Group 1 animals were treated with OM69 (0.01 mg/kg) and Group 2 were treated with L-dopa (1 mg/kg). On Day 10, Group 1 animals were treated with OM69 (0.03 mg/kg) and Group 2 were treated with L-dopa (5 mg/kg). On Day 11, Group 1 animals were treated with OM69 (0.05 mg/kg) and Group 2 were treated with OM69 (0.1 mg/kg). On Day 12, Group 1 was treated with OM69 (0.07 mg/kg).

Next, for combination studies, the two groups were combined. On Day 14, all the animals were treated with both L-dopa (1 mg/kg) and OM69 (0.01 mg/kg). On Day 15, all the animals were treated with L-dopa (1 mg/kg) in combination with a higher dose of OM69 (0.03 mg/kg).

### Results:

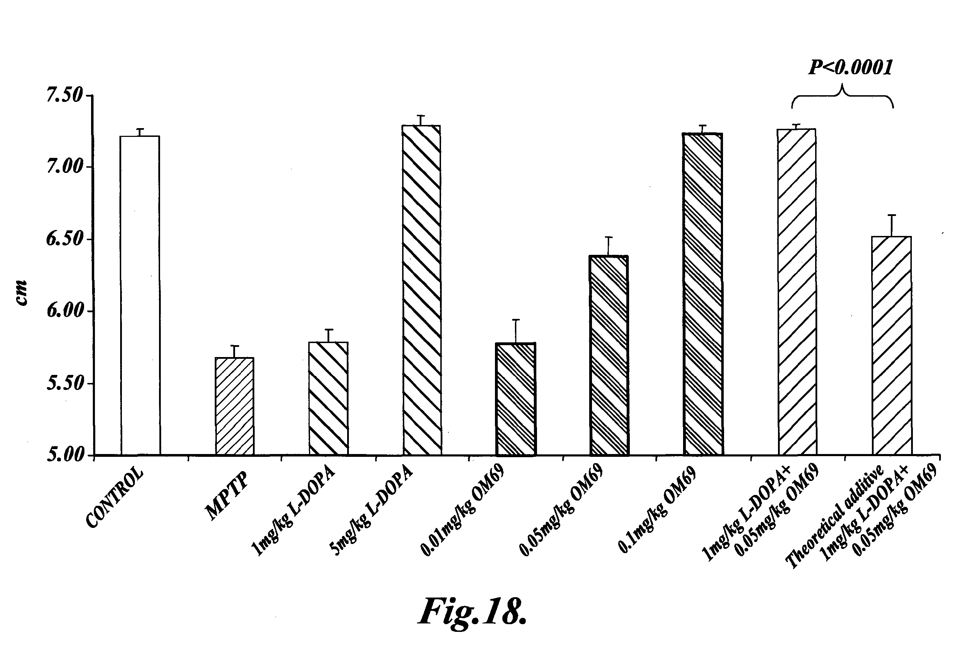

The results of these studies are summarized below in TABLE 6, in which the stride length of the MPTP-treated mice treated with L-dopa at two different dosages, OM69 (compound 1) at five different dosages, and the combination of L-dopa and OM69 are compared to the untreated control mice and the MPTP-treated mice receiving no drug. FIGURE 18 illustrates a subset of this data.

**TABLE 6: Stride Length in MPTP-lesioned Mice**

| **Treatment** | **Stride length (cm) MEAN±SEM** | **Number of animals successfully performing task** | **Letter Designation** |
|---|---|---|---|
| No treatment | 7.21 ± 0.06 | 28 | A |
| MPTP | 5.68 ± 0.09 | 25 | B |
| 1 mg/kg L-DOPA | 5.78 ± 0.09 | 13 | B |
| 5 mg/kg L-DOPA | 7.29 ± 0.07 | 14 | A |
| 0.01 mg/kg OM69 | 5.77 ± 0.17 | 11 | B |
| 0.03 mg/kg OM69 | 5.93 ± 0.14 | 14 | B |
| 0.05 mg/kg OM69 | 6.38 ± 0.14 | 13 | C |
| 0.07 mg/kg OM69 | 6.65 ± 0.18 | 13 | C |
| 0.1 mg/kg OM69 | 7.23 ± 0.07 | 14 | A |
| 1 mg/kg L-DOPA + 0.03 mg/kg OM69 | 6.81 ± 0.07 | 26 | C |
| 1 mg/kg L-DOPA + 0.05 mg/kg OM69 | 7.26 ± 0.04 | 25 | A |

### Discussion of Results:

### Comparison of OM69 to L-dopa:

Referring to TABLE 6, in this experiment, MPTP caused a significant decrease in stride length (letter "B" vs. "A"). Treatment with 1 mg/kg L-dopa did not result in a significant increase in stride length, but treatment with 5 mg/kg L-dopa returned stride length to control (unlesioned) values ("A" vs. "A"). Treatment with low doses of OM69 (0.01 mg/kg or 0.03 mg/kg) also did not increase stride length, but treatment with intermediate doses resulted in a statistically significant (p<0.05) increase in stride length ("C" vs. "B"). Treatment with a higher dose (0.1 mg/kg) of OM69 also resulted in a statistically significant (p<0.05) increase in stride length and, furthermore, returned stride length to control (unlesioned) values ("A" vs. "A"). These results demonstrate that the PDE7 inhibitory compound OM69 is as effective as L-dopa in restoring stride length in MPTP-treated mice and, moreover, is approximately 50-fold more potent than L-dopa.

### OM69 and L-dopa administered in combination:

Two dose combinations of OM69 with L-dopa were also tested in this experiment. The first combination (1 mg/kg L-dopa plus 0.03 mg/kg OM69) caused a statistically significant (p<0.05) increase in stride length of 1.13 cm, which was greater than the sum of the non-significant increases with those agents alone at those doses (0.35 cm). The second combination (1 mg/kg L-dopa plus 0.05 mg/kg OM69) also resulted in a statistically significant (p<0.05) increase in stride length and, furthermore, returned stride length to control (unlesioned) values ("A" vs. "A"). The magnitude of this increase (1.58 cm) was statistically significantly greater (p<0.0001) than the sum of the increase with 0.05 mg/kg OM69 plus the non-significant increase with 1 mg/kg L-dopa (0.8 cm), as represented by the theoretical additive bar on the right hand side of the chart in FIGURE 18. These results strongly suggest that the PDE7 inhibitory compound OM69 and L-dopa interact in a greater-than-additive manner to correct the stride length of MPTP-treated mice.

### Example 7

### Pharmacological Evaluation of a Panel of Representative PDE7 Inhibitory Compounds in the MPTP Model of Parkinson's Disease

In this Example, a panel of representative PDE7 inhibitory agents were evaluated in the mouse MPTP model of Parkinson's disease in order to test the hypothesis that inhibition of PDE7 will improve Parkinsonian symptoms in this model regardless of the particular chemical structure of the inhibitor used, and that therefore, inhibition of PDE7 is sufficient for the observed improvement in stride length.

### Animal Testing Protocol.

*Mouse strain:* Adult male C57BL/6J; retired breeders, aged between 7-9 months Charles River Laboratories, Wilmington, MA), singly housed. Three groups of 35 mice were tested in series one week apart.

*Handling:* Animals were handled daily for 2 weeks prior to injection, in order to make them amenable to behavioral testing. All mice were maintained on a standard 12-h light/dark cycle, and given *ad libitum* access to lab chow and water.

*MPTP administration:* Mice received 2 subcutaneous injections of methylphenyltetrahydropyridine ("MPTP") at a dose of 15 mg/kg (free base) with a 10-12 hour interval between injections. Control mice (sham lesion groups) were administered saline instead of MPTP.

*Stride Length:* Prior to injection with MPTP or saline, animals were pre-trained to walk across a clean sheet of paper into their home cage without stopping. For assessment of stride length, animals had their forepaws placed in black ink and the length of forepaw steps during normal walking (in a straight line only) was measured. The animals were immediately put back into their home cage upon completion of the task. Stride length was determined by measuring the distance between each step on the same side of the body, measuring from the middle toe of the first step to the heel of the second step. An average of at least four clear steps was calculated. The schedule for this experiment was as follows:
Week 0 -- Training to striding task: (at least 4 sessions)
Day 1 - collect baseline stride
Day 2a - collect 2^{nd} baseline stride
Day 2b - MPTP injections
Day 7 - collect and screen for stride deficit
Day 8a - collect 2^{nd} deficit strides
Day 8b - begin compound trials
Day 9 onward. - different doses of compound were administered on successive days.

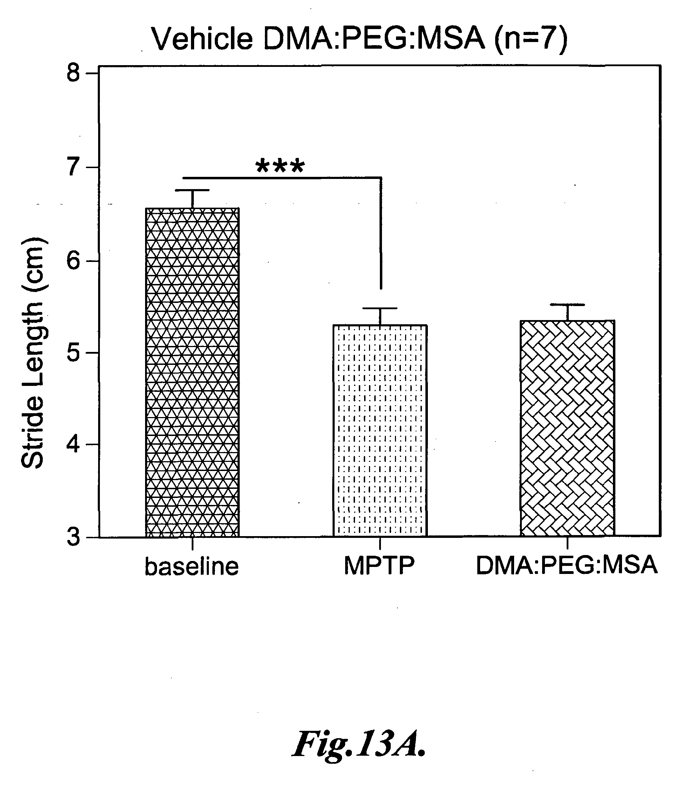

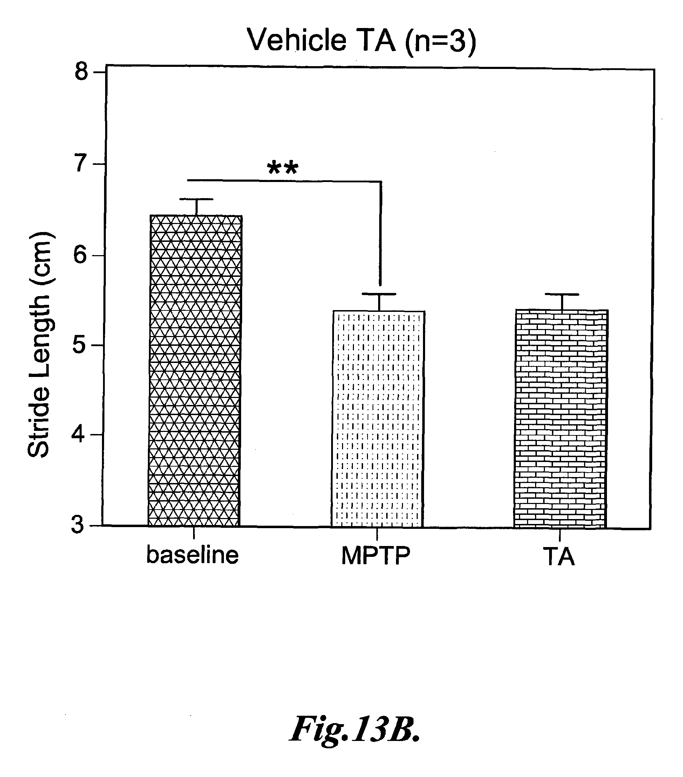

*Compound Administration:* Because of the wide variation in aqueous solubility among these compounds, a number of different formulations were required. L-dopa was administered in 100 µl of phosphate buffered saline. For animals treated with L-dopa, the dopa decarboxylase inhibitor benserazide (100 µL of a 12.5 mg/kg solution) was administered 15 minutes prior to treatment with L-dopa in order to minimize the destruction of L-dopa in the peripheral blood. OM955 (compound 3) and OM056 (compound 2) were administered in 200 µl of dimethyl acetamide: polyethylene glycol 400:0.03M methane sulfonic acid (DMA:PEG:MSA-10%:40%:50%). OM956 (compound 4) was administered in 200 µl of 0.03 M Tartaric acid (TA). FIGURES 13A and 13B show that neither of these vehicles, when administered by themselves, alter stride length in MPTP-treated mice. Therefore, the treatment effects observed are due solely to the PDE7 inhibitory compounds themselves. For each compound tested, preliminary experiments were performed to identify the most effective dose in the MPTP model. In some cases, it was observed that doses higher than the optimal dose did not yield significant increases in stride length. This phenomenon of "overshoot" has also been observed with L-dopa in MPTP-treated mice in which lower doses ameliorate hypoactivity while higher doses induce dyskinesias or uncontrolled movements (Lundblad M. et al., Exp Neurol.194(1):66-75 (2005); Pearce R.K. et al., Mov Disord. 10(6):731-40 (1995); Fredriksson, A. et al, Pharmacol-Toxicol. 67(4): 295-301 (1990)).

### Experimental Protocol:

Retired breeder male mice were treated with either saline or 2 x 15 mg/kg MPTP to induce a Parkinsonian state (neurochemical loss of dopamine with corresponding behavioral deficits). On day one baseline stride length was measured and then mice were treated with either MPTP or with saline as a control. On day eight stride length was measured again, and the control value for stride length was derived from the stride length of saline-treated animals. The animals were then divided randomly into treatment groups. Compound OM955 (compound 3) was tested at 0.1 mg/kg, at 0.5 mg/kg, and at 0.1 mg/kg in combination with 1 mg/kg L-dopa. Compound OM956 (compound 4) was tested at 0.1 mg/kg and 0.5 mg/kg. Compound OM056 (compound 2) was tested at 0.05 mg/kg, 0.1 mg/kg, 0.5 mg/kg, and 1 mg/kg.

### Results:

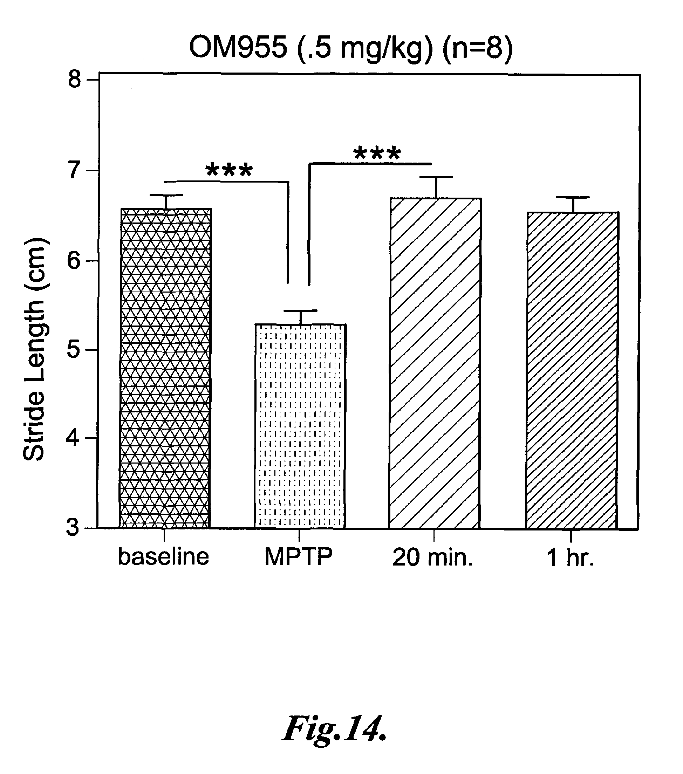

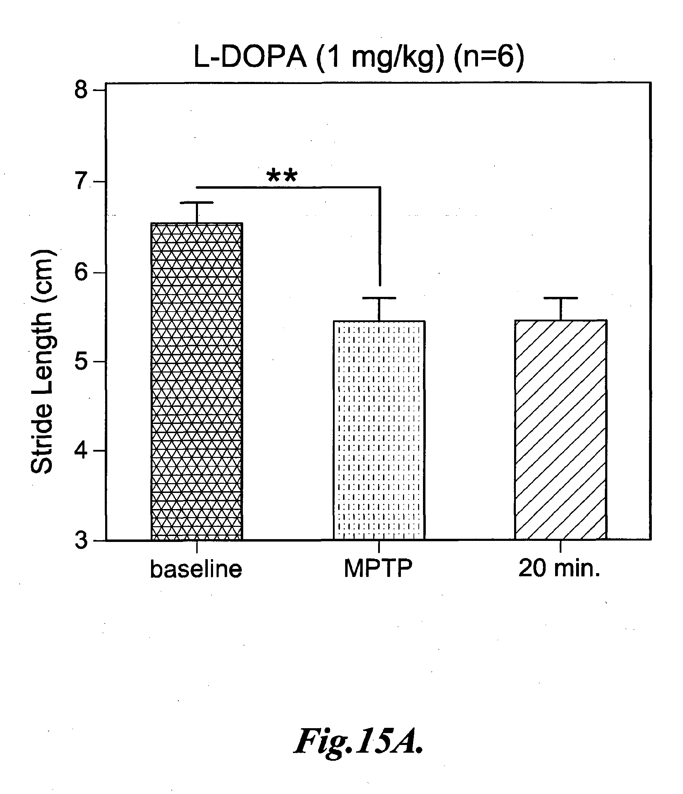

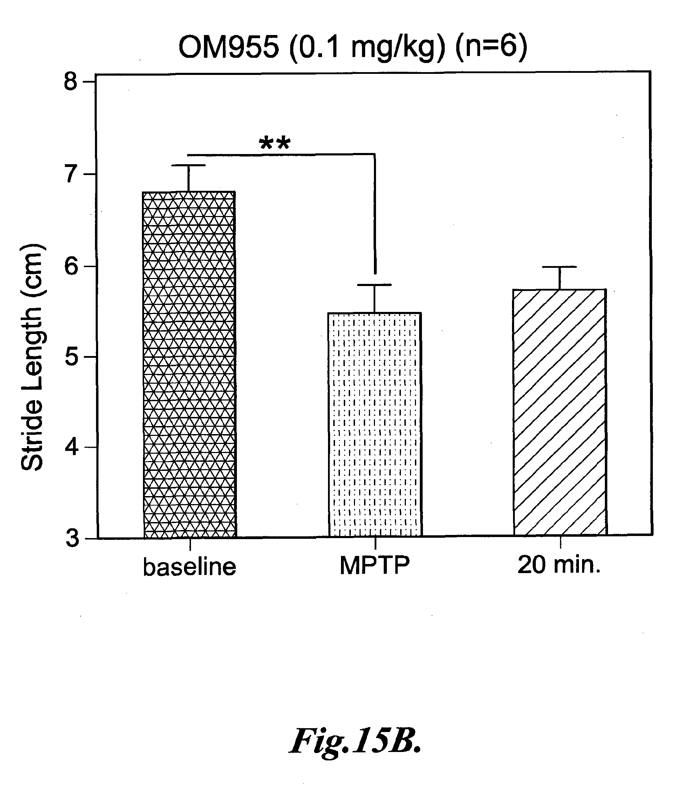

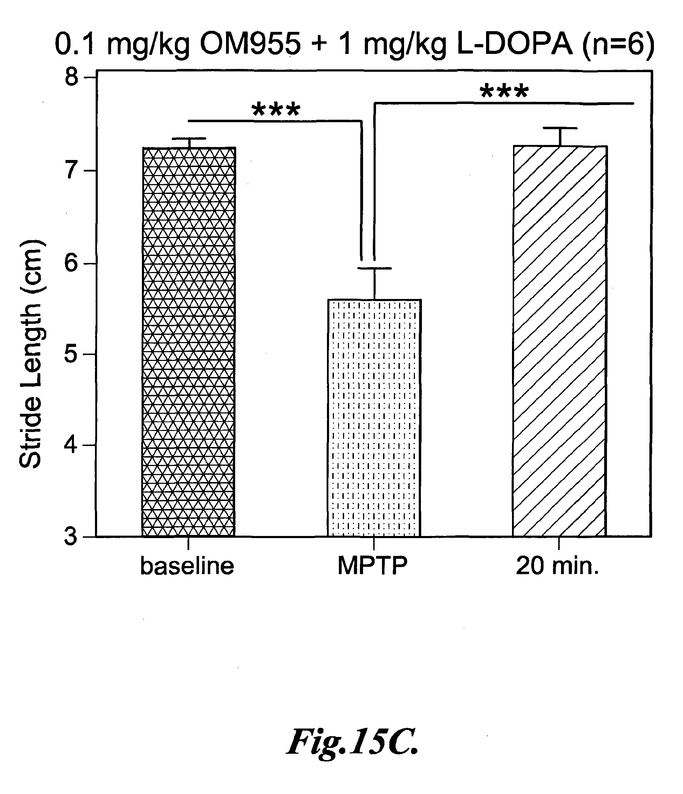

The results of this study are shown in FIGURES 14-17. FIGURE 14 shows that OM955 at a dose of 0.5 mg/kg causes a statistically significant improvement in the stride length of MPTP-treated mice (p<0.005 compared to MPTP group). At both 20 minutes and one hour after injection, the stride length is fully restored to that of unlesioned animals. FIGURES 15A-C demonstrate that the combination of OM955 and L-dopa exerts greater-than-additive effects to restore stride length. FIGURES 15A and 15B show, respectively, that low doses of L-dopa (1 mg/kg) or OM955 (0.1 mg/kg) do not increase stride length when administered alone. However, FIGURE 15C shows that when these low doses are given together, stride length is completely restored to that of unlesioned animals (p<0.005 compared to MPTP group).

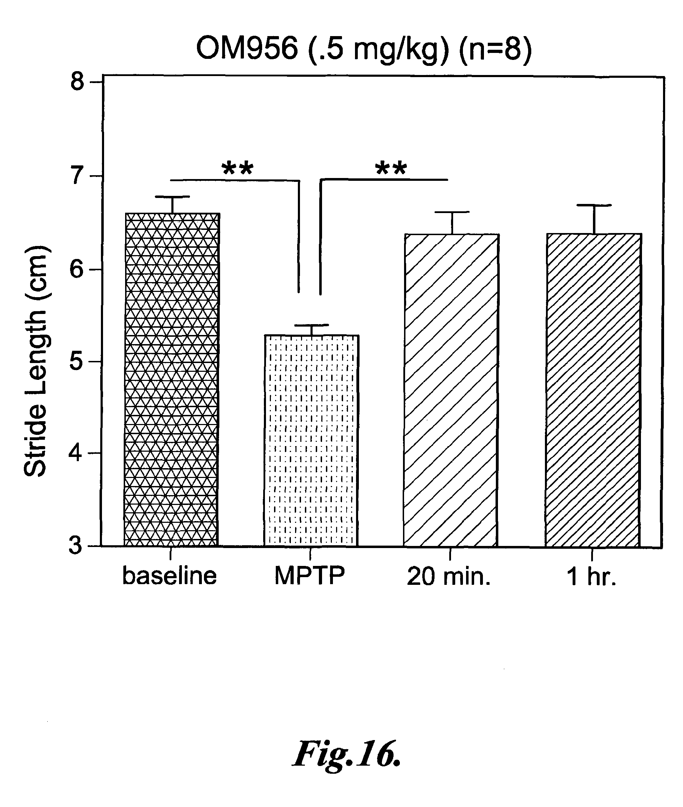

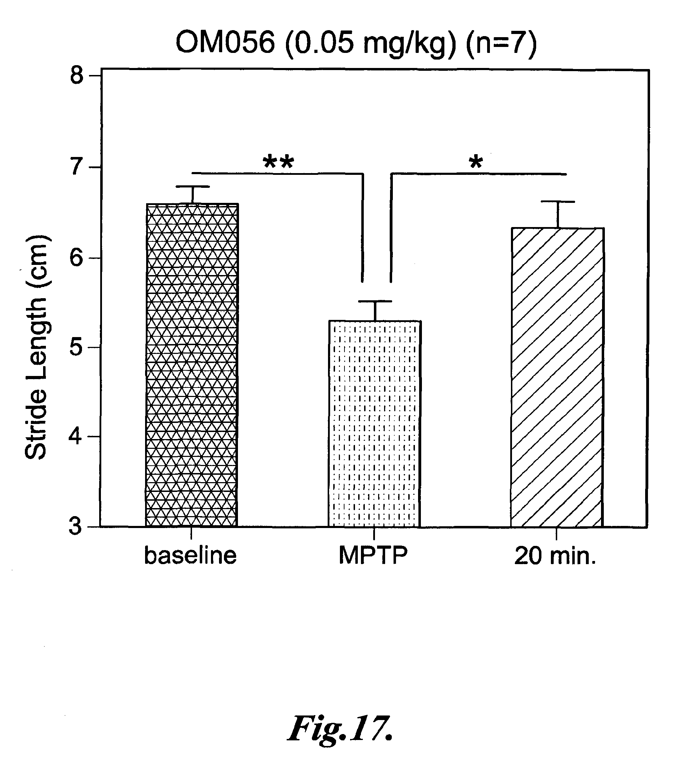

FIGURE 16 shows that OM956, also at a dose of 0.5 mg/kg, causes a statistically significant increase (p<0.01 compared to MPTP group) in the stride length of MPTP-treated animals. FIGURE 17 shows that OM056 at the lower dose of 0.05 mg/kg also causes a statistically significant increase (p<0.05 compared to MPTP group) in the stride length of MPTP-treated animals.

The results in this Example show that three different PDE7 inhibitory compounds, which are structurally unrelated to each other and structurally unrelated to OM69, all cause the same complete recovery of stride length in MPTP-treated animals that was observed with OM69 (as described in Examples 5 and 6). Because the only known common property of these compounds is their ability to inhibit PDE7, and because one of these compounds (OM69) was tested for its interaction with other known Parkinson's disease targets and found not to interact significantly with them, it is concluded that PDE7 inhibitory activity is both necessary and sufficient for the improvement in stride length reported with these compounds.

## Claims

1. A compound which is a PDE7 inhibitory agent for use in the treatment of movement abnormalities in Parkinson's disease, Post-Encephalitic Parkinsonism, Dopamine-Responsive Dystonia, Shy-Drager Syndrome, Period Limb Movement Disorder (PLMD), Periodic Limb Movements in Sleep (PLMS), and Restless Leg(s) Syndrome (RLS), by inhibition of the enzymatic activity of PDE7, which PDE7 inhibitory agent has an IC₅₀ for inhibiting the activity of PDE1, PDE2, PDE3, PDE4, PDE8, PDE10 and PDE11 of greater than 10 times the lesser of the IC₅₀ for inhibiting PDE7A activity and the IC₅₀ for inhibiting PDE7B activity.

2. A compound for use as claimed in claim 1 for use in the treatment of Parkinson's disease.

3. A compound for use as claimed in claim 1 wherein the movement disorder is at least one of tremor at rest, rigidity, brady kinesis or deficiency of postural reflexes.

4. A compound for use as claimed in claims 1 or 2 which is an inhibitor of PDE7A.

5. A compound for use as claimed in claims 1 or 2 which is an inhibitor of PDE7B.

6. A compound for use as claimed in claim 1 which is compound (1):

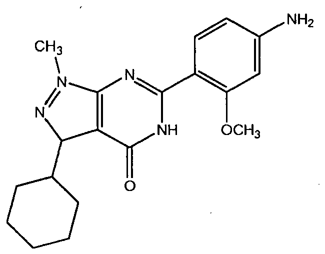

7. A compound for use as claimed in claim 1 which is compound (2):

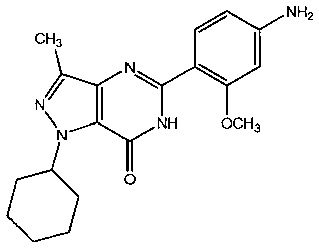

8. A compound for use as claimed in claim 1 which is compound (3):

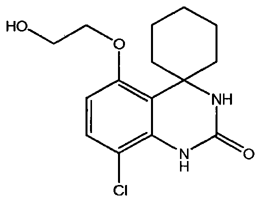

9. A compound for use as claimed in claim 1 which is compound (4):

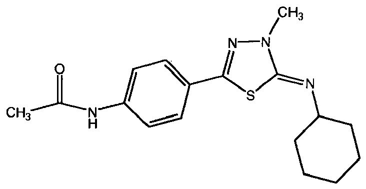

10. A compound for use as claimed in claim 1 for use together with a dopaminergic agent, a therapeutic agent that activates the dopamine D1 receptor, a therapeutic agent that increases the concentration of dopamine in the nigrostriatal nerve terminals, or a therapeutic agent that increases the concentration of dopamine in the nigrostriatal synaptic cleft.

11. A compound for use as claimed in claim 1 together with L-DOPA.

12. A pharmaceutical composition for use in the treatment as set forth in claim 1 which comprises a PDE7 inhibitor as set forth in any of claims 1 and 6 to 9.

## Patentansprüche

1. Verbindung, bei der es sich um eine PDE7 hemmende Substanz handelt, zur Verwendung in der Behandlung von Bewegungsstörungen der Parkinson-Krankheit, des Postenzephalitischen Parkinson-Syndroms, Dopa-Responsiver Dystonie, des Shy-Drager-Syndroms, der Period Limb Movement Disorder (PLMD), von Periodic Limb Movements in Sleep (PLMS) und dem Restless-Leg(s)-Syndrom durch Hemmung der enzymatischen Aktivität von PDE7, wobei die PDE7 hemmende Substanz eine IC₅₀ zur Hemmung der Aktivität von PDE1, PDE2, PDE3, PDE4, PDE8, PDE10 und PDE11 von mehr als dem Zehnfachen des kleineren Wertes der IC₅₀ für die Hemmung der PDE7A-Aktivät und des IC₅₀ für die Hemmung der PDE7B-Aktivität aufweist.

2. Verbindung zur Verwendung nach Anspruch 1, zur Verwendung in der Behandlung der Parkinson-Krankheit.

3. Verbindung zur Verwendung nach Anspruch 1, wobei die Bewegungsstörung wenigstens eine ist aus Ruhetremor, Steifigkeit, Bradykinese oder Posturale Instabilität.

4. Verbindung zur Verwendung nach Anspruch 1 oder 2, die ein Inhibitor von PDE7A ist.

5. Verbindung zur Verwendung nach Anspruch 1 oder 2, die ein Inhibitor von PDE7B ist.

6. Verbindung zur Verwendung nach Anspruch 1, bei der es sich um Verbindung (1) handelt:

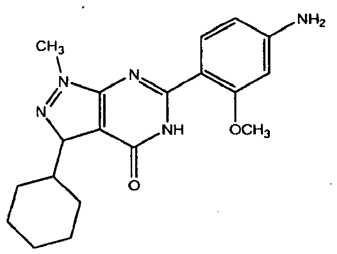

7. Verbindung zur Verwendung nach Anspruch 1, bei der es sich um Verbindung (2) handelt:

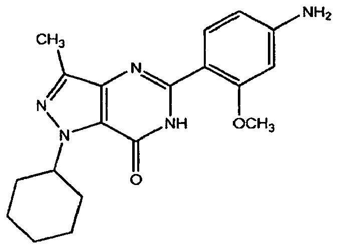

8. Verbindung zur Verwendung nach Anspruch 1, bei der es sich um Verbindung (3) handelt:

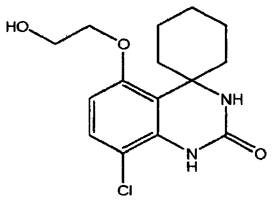

9. Verbindung zur Verwendung nach Anspruch 1, bei der es sich um Verbindung (4) handelt:

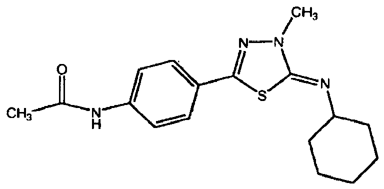

10. Verbindung zur Verwendung nach Anspruch 1 zur gemeinsamen Verwendung mit einem Dopaminergikum, einem Therapeutikum, das den Dopamin-Rezeptor D1 aktiviert, einem Therapeutikum, das die Dopaminkonzentration in den nigrostriatalen Nervenendigungen erhöht oder ein Therapeutikum, das die Dopaminkonzentration in des nigrostriatalen synaptischen Spalts erhöht.

11. Verbindung zur Verwendung nach Anspruch in Verbindung mit L-DOPA.

12. Pharmazeutische Zusammensetzung zur Verwendung nach Anspruch 1, umfassend einen PDE7-Inhibitor nach einem der Ansprüche 1 und 6 bis 9.

## Revendications

1. Composé qui est agent inhibiteur de PDE7 destiné à une utilisation dans le traitement des mouvements anormaux tels que présents dans la maladie de Parkinson, le parkinsonisme post-encéphalitique, la dystonie qui répond à la dopamine, le syndrome de Shy et Drager, le syndrome du mouvement périodique des membres (PLMD), le syndrome du mouvement périodique des membres pendant le sommeil (PLMS) et le syndrome de l'impatience du (des) membre(s) inférieur(s) (RLS), par l'inhibition de l'activité enzymatique de PDE7, lequel agent inhibiteur de PDE7 possède une IC50 pour l'inhibition de l'activité de PDE1, PDE2, PDE3, PDE4, PDE8, pDE10 et PDE11 supérieure à 10 fois à l'IC50 la moins élevée pour l'inhibition de l'activité de PDE7A et l'IC50 pour l'inhibition de l'activité de PDE7B.

2. Composé destiné à une utilisation selon la revendication 1 destiné à l'utilisation dans le traitement de la maladie de Parkinson.

3. Composé destiné à une utilisation selon la revendication 1, dans lequel le trouble des mouvements est au moins un de tremblement au repos, la kinésie corporelle ou la déficience des réflexes posturaux.

4. Composé destiné à une utilisation selon la revendication 1 ou la revendication 2 qui est un inhibiteur de PDEE7A.

5. Composé destiné à une utilisation selon la revendication 1 ou la revendication 2 qui est un inhibiteur de PDEE7B.

6. Composé destiné à une utilisation selon la revendication 1 qui est le composé (1) :

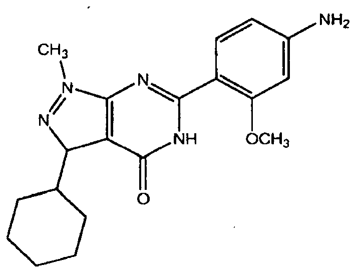

7. Composé destiné à une utilisation selon la revendication 1 qui est le composé (2) :

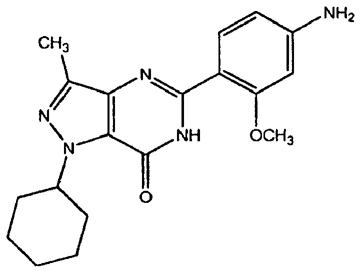

8. Composé destiné à une utilisation selon la revendication 1 qui est le composé (3) :

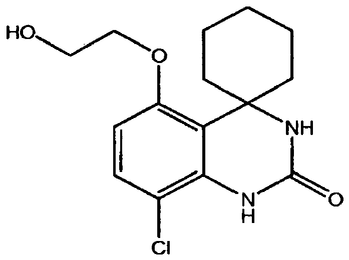

9. Composé destiné à une utilisation selon la revendication 1 qui est le composé (4) :

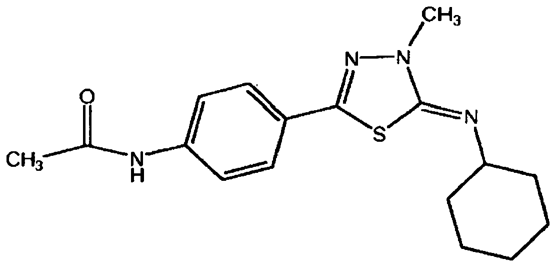

10. Composé destiné à une utilisation selon la revendication 1 pour une utilisation ensemble avec un agent dopaminergique, un agent thérapeutique qui active le récepteur à dopamine D1, un agent thérapeutique qui augmente la concentration de dopamine dans les terminaux des nerfs nigrostriés, ou un agent thérapeutique qui la concentration de dopamine dans la fente synaptique nigrostriée.

11. Composé destiné à une utilisation selon la revendication 1 ensemble avec L-DOPA.

12. Composition pharmaceutique destinée à une utilisation dans le traitement tel qu'exposé selon la revendication 1 qui comprend un inhibiteur de PDE7 tel qu'exposé selon l'une quelconque des revendications 1 et 6 à 9.


### SEQUENCE LISTING

<110> Omeros Corporation Bergmann, J.E. Cutshall, N.S. Demopulos, G.A. Florio, V. Gaitanaris, G. Gray, P. Hohman, J. Onrust, R. Zeng, H.
<120> THE USE OF PDE7 INHIBITORS FOR THE TREATMENT OF MOVEMENT DISORDERS
<130> OMER-1-31122
<150> US 60/920,496
   <151> 2007-03-27
<160> 6
<170> PatentIn version 3.5
<210> 1
   <211> 1739
   <212> DNA
   <213> Homo sapiens
<220>
   <221> CDS
   <222> (157)..(1527)
<400> 1
   ggggatcact gttggaaggc agctgcttga ggtccaaggc agtcagtgtc ccctctcttt      60
   tgcctcggga cagctggtat ttatcagact cctaagaagt tttccttgct ccctagtaga     120
   agagagagat tatgcagcgg gcttttgatt gatcca atg gga att aca ttg atc       174
                                           Met Gly Ile Thr Leu Ile
                                           1               5
   tgg tgt ctg gcc ttg gtt ctt atc aag tgg atc acc tct aag agg cgt       222
   Trp Cys Leu Ala Leu Val Leu Ile Lys Trp Ile Thr Ser Lys Arg Arg
               10                  15                  20
   gga gct att tcc tat gac agt tct gat cag act gca tta tac att cgt       270
   Gly Ala Ile Ser Tyr Asp Ser Ser Asp Gln Thr Ala Leu Tyr Ile Arg
           25                  30                  35
   atg cta gga gat gta cgt gta agg agc cga gca gga ttt gaa tca gaa       318
   Met Leu Gly Asp Val Arg Val Arg Ser Arg Ala Gly Phe Glu Ser Glu
       40                  45                  50
   aga aga ggt tct cac cca tat att gat ttt cgt att ttc cac tct caa       366
   Arg Arg Gly Ser His Pro Tyr Ile Asp Phe Arg Ile Phe His Ser Gln
   55                  60                  65                  70
   tct gaa att gaa gtg tct gtc tct gca agg aat atc aga agg cta cta       414
   Ser Glu Ile Glu Val Ser Val Ser Ala Arg Asn Ile Arg Arg Leu Leu
                   75                  80                  85
   agt ttc cag cga tat ctt aga tct tca cgc ttt ttt cgt ggt act gcg      462
   Ser Phe Gln Arg Tyr Leu Arg Ser Ser Arg Phe Phe Arg Gly Thr Ala
               90                  95                  100
   gtt tca aat tcc cta aac att tta gat gat gat tat aat gga caa gcc      510
   Val Ser Asn Ser Leu Asn Ile Leu Asp Asp Asp Tyr Asn Gly Gln Ala
           105                 110                 115
   aag tgt atg ctg gaa aaa gtt gga aat tgg aat ttt gat atc ttt cta      558
   Lys Cys Met Leu Glu Lys Val Gly Asn Trp Asn Phe Asp Ile Phe Leu
       120                 125                 130
   ttt gat aga cta aca aat gga aat agt cta gta agc tta acc ttt cat      606
   Phe Asp Arg Leu Thr Asn Gly Asn Ser Leu Val Ser Leu Thr Phe His
   135                 140                 145                 150
   tta ttt agt ctt cat gga tta att gag tac ttc cat tta gat atg atg      654
   Leu Phe Ser Leu His Gly Leu Ile Glu Tyr Phe His Leu Asp Met Met
                   155                 160                 165
   aaa ctt cgt aga ttt tta gtt atg att caa gaa gat tac cac agt caa      702
   Lys Leu Arg Arg Phe Leu Val Met Ile Gln Glu Asp Tyr His Ser Gln
               170                 175                 180
   aat cct tac cat aac gca gtc cac gct gcg gat gtt act cag gcc atg      750
   Asn Pro Tyr His Asn Ala Val His Ala Ala Asp Val Thr Gln Ala Met
           185                 190                 195
   cac tgt tac tta aag gaa cct aag ctt gcc aat tct gta act cct tgg      798
   His Cys Tyr Leu Lys Glu Pro Lys Leu Ala Asn Ser Val Thr Pro Trp
       200                 205                 210
   gat atc ttg ctg agc tta att gca gct gcc act cat gat ctg gat cat      846
   Asp Ile Leu Leu Ser Leu Ile Ala Ala Ala Thr His Asp Leu Asp His
   215                 220                 225                 230
   cca ggt gtt aat caa cct ttc ctt att aaa act aac cat tac ttg gca      894
   Pro Gly Val Asn Gln Pro Phe Leu Ile Lys Thr Asn His Tyr Leu Ala
                   235                 240                 245
   act tta tac aag aat acc tca gta ctg gaa aat cac cac tgg aga tct      942
   Thr Leu Tyr Lys Asn Thr Ser Val Leu Glu Asn His His Trp Arg Ser
               250                 255                 260
   gca gtg ggc tta ttg aga gaa tca ggc tta ttc tca cat ctg cca tta      990
   Ala Val Gly Leu Leu Arg Glu Ser Gly Leu Phe Ser His Leu Pro Leu
           265                 270                 275
   gaa agc agg caa caa atg gag aca cag ata ggt gct ctg ata cta gcc     1038
   Glu Ser Arg Gln Gln Met Glu Thr Gln Ile Gly Ala Leu Ile Leu Ala
       280                 285                 290
   aca gac atc agt cgc cag aat gag tat ctg tct ttg ttt agg tcc cat     1086
   Thr Asp Ile Ser Arg Gln Asn Glu Tyr Leu Ser Leu Phe Arg Ser His
   295                 300                 305                 310
   ttg gat aga ggt gat tta tgc cta gaa gac acc aga cac aga cat ttg     1134
   Leu Asp Arg Gly Asp Leu Cys Leu Glu Asp Thr Arg His Arg His Leu
                   315                 320                 325
   gtt tta cag atg gct ttg aaa tgt gct gat att tgt aac cca tgt cgg     1182
   Val Leu Gln Met Ala Leu Lys Cys Ala Asp Ile Cys Asn Pro Cys Arg
                   330                 335                 340
   acg tgg gaa tta agc aag cag tgg agt gaa aaa gta acg gag gaa ttc       1230
   Thr Trp Glu Leu Ser Lys Gln Trp Ser Glu Lys Val Thr Glu Glu Phe
               345                 350                 355
   ttc cat caa gga gat ata gaa aaa aaa tat cat ttg ggt gtg agt cca       1278
   Phe His Gln Gly Asp Ile Glu Lys Lys Tyr His Leu Gly Val Ser Pro
           360                 365                 370
   ctt tgc gat cgt cac act gaa tct att gcc aac atc cag att ggt ttt       1326
   Leu Cys Asp Arg His Thr Glu Ser Ile Ala Asn Ile Gln Ile Gly Phe
   375                 380                 385                 390
   atg act tac cta gtg gag cct tta ttt aca gaa tgg gcc agg ttt tcc       1374
   Met Thr Tyr Leu Val Glu Pro Leu Phe Thr Glu Trp Ala Arg Phe Ser
                   395                 400                 405
   aat aca agg cta tcc cag aca atg ctt gga cac gtg ggg ctg aat aaa       1422
   Asn Thr Arg Leu Ser Gln Thr Met Leu Gly His Val Gly Leu Asn Lys
               410                 415                 420
   gcc agc tgg aag gga ctg cag aga gaa cag tcg agc agt gag gac act       1470
   Ala Ser Trp Lys Gly Leu Gln Arg Glu Gln Ser Ser Ser Glu Asp Thr
           425                 430                 435
   gat gct gca ttt gag ttg aac tca cag tta tta cct cag gaa aat cgg       1518
   Asp Ala Ala Phe Glu Leu Asn Ser Gln Leu Leu Pro Gln Glu Asn Arg
       440                 445                 450
   tta tca taa cccccagaac cagtgggaca aactgcctcc tggaggtttt               1567
   Leu Ser
   455
   tagaaatgtg aaatggggtc ttgaggtgag agaacttaac tcttgactgc caaggtttcc     1627
   aagtgagtga tgccagccag cattatttat ttccaagatt tcctctgttg gatcatttga     1687
   acccacttgt taattgcaag acccgaacat acagcaatat gaatttggct tt             1739
<210> 2
   <211> 456
   <212> PRT
   <213> Homo sapiens
<400> 2
   Met Gly Ile Thr Leu Ile Trp Cys Leu Ala Leu Val Leu Ile Lys Trp
   1               5                   10                  15
   Ile Thr Ser Lys Arg Arg Gly Ala Ile Ser Tyr Asp Ser Ser Asp Gln
               20                  25                  30
   Thr Ala Leu Tyr Ile Arg Met Leu Gly Asp Val Arg Val Arg Ser Arg
           35                  40                  45
   Ala Gly Phe Glu Ser Glu Arg Arg Gly Ser His Pro Tyr Ile Asp Phe
       50                  55                  60
   Arg Ile Phe His Ser Gln Ser Glu Ile Glu Val Ser Val Ser Ala Arg
   65 70 75 80
   Asn Ile Arg Arg Leu Leu Ser Phe Gln Arg Tyr Leu Arg Ser Ser Arg
   85 90 95
   Phe Phe Arg Gly Thr Ala Val Ser Asn Ser Leu Asn Ile Leu Asp Asp
   100 105 110
   Asp Tyr Asn Gly Gln Ala Lys Cys Met Leu Glu Lys Val Gly Asn Trp
   115 120 125
   Asn Phe Asp Ile Phe Leu Phe Asp Arg Leu Thr Asn Gly Asn Ser Leu
   130 135 140
   Val Ser Leu Thr Phe His Leu Phe Ser Leu His Gly Leu Ile Glu Tyr
   145 150 155 160
   Phe His Leu Asp Met Met Lys Leu Arg Arg Phe Leu Val Met Ile Gln
   165 170 175
   Glu Asp Tyr His Ser Gln Asn Pro Tyr His Asn Ala Val His Ala Ala
   180 185 190
   Asp Val Thr Gln Ala Met His Cys Tyr Leu Lys Glu Pro Lys Leu Ala
   195 200 205
   Asn Ser Val Thr Pro Trp Asp Ile Leu Leu Ser Leu Ile Ala Ala Ala
   210 215 220
   Thr His Asp Leu Asp His Pro Gly Val Asn Gln Pro Phe Leu Ile Lys
   225 230 235 240
   Thr Asn His Tyr Leu Ala Thr Leu Tyr Lys Asn Thr Ser Val Leu Glu
   245 250 255
   Asn His His Trp Arg Ser Ala Val Gly Leu Leu Arg Glu Ser Gly Leu
   260 265 270
   Phe Ser His Leu Pro Leu Glu Ser Arg Gln Gln Met Glu Thr Gln Ile
   275 280 285
   Gly Ala Leu Ile Leu Ala Thr Asp Ile Ser Arg Gln Asn Glu Tyr Leu
   290 295 300
   Ser Leu Phe Arg Ser His Leu Asp Arg Gly Asp Leu Cys Leu Glu Asp
   305 310 315 320
   Thr Arg His Arg His Leu Val Leu Gln Met Ala Leu Lys Cys Ala Asp
                   325                 330                 335
   Ile Cys Asn Pro Cys Arg Thr Trp Glu Leu Ser Lys Gln Trp Ser Glu
               340                 345                 350
   Lys Val Thr Glu Glu Phe Phe His Gln Gly Asp Ile Glu Lys Lys Tyr
           355                 360                 365
   His Leu Gly Val Ser Pro Leu Cys Asp Arg His Thr Glu Ser Ile Ala
       370                 375                 380
   Asn Ile Gln Ile Gly Phe Met Thr Tyr Leu Val Glu Pro Leu Phe Thr
   385                 390                 395                 400
   Glu Trp Ala Arg Phe Ser Asn Thr Arg Leu Ser Gln Thr Met Leu Gly
                   405                 410                 415
   His Val Gly Leu Asn Lys Ala Ser Trp Lys Gly Leu Gln Arg Glu Gln
               420                 425                 430
   Ser Ser Ser Glu Asp Thr Asp Ala Ala Phe Glu Leu Asn Ser Gln Leu
           435                 440                 445
   Leu Pro Gln Glu Asn Arg Leu Ser
       450                 455
<210> 3
   <211> 2990
   <212> DNA
   <213> Homo sapiens
<220>
   <221> CDS
   <222> (1)..(1275)
<400> 3
   atg gaa gtg tgt tac cag ctg ccg gta ctg ccc ctg gac agg ccg gtc      48
   Met Glu Val Cys Tyr Gln Leu Pro Val Leu Pro Leu Asp Arg Pro Val
   1               5                   10                  15
   ccc cag cac gtc ctc agc cgc cga gga gcc atc agc ttc agc tcc agc      96
   Pro Gln His Val Leu Ser Arg Arg Gly Ala Ile Ser Phe Ser Ser Ser
               20                  25                  30
   tcc gct ctc ttc ggc tgc ccc aat ccc cgg cag ctc tct cag agg cgt     144
   Ser Ala Leu Phe Gly Cys Pro Asn Pro Arg Gln Leu Ser Gln Arg Arg
           35                  40                  45
   gga gct att tcc tat gac agt tct gat cag act gca tta tac att cgt     192
   Gly Ala Ile Ser Tyr Asp Ser Ser Asp Gln Thr Ala Leu Tyr Ile Arg
           50                  55                  60
   atg cta gga gat gta cgt gta agg agc cga gca gga ttt gaa tca gaa      240
   Met Leu Gly Asp Val Arg Val Arg Ser Arg Ala Gly Phe Glu Ser Glu
   65                  70                  75                  80
   aga aga ggt tct cac cca tat att gat ttt cgt att ttc cac tct caa      288
   Arg Arg Gly Ser His Pro Tyr Ile Asp Phe Arg Ile Phe His Ser Gln
                   85                  90                  95
   tct gaa att gaa gtg tct gtc tct gca agg aat atc aga agg cta cta      336
   Ser Glu Ile Glu Val Ser Val Ser Ala Arg Asn Ile Arg Arg Leu Leu
               100                 105                 110
   agt ttc cag cga tat ctt aga tct tca cgc ttt ttt cgt ggt act gcg      384
   Ser Phe Gln Arg Tyr Leu Arg Ser Ser Arg Phe Phe Arg Gly Thr Ala
           115                 120                 125
   gtt tca aat tcc cta aac att tta gat gat gat tat aat gga caa gcc      432
   Val Ser Asn Ser Leu Asn Ile Leu Asp Asp Asp Tyr Asn Gly Gln Ala
       130                 135                 140
   aag tgt atg ctg gaa aaa gtt gga aat tgg aat ttt gat atc ttt cta      480
   Lys Cys Met Leu Glu Lys Val Gly Asn Trp Asn Phe Asp Ile Phe Leu
   145                 150                 155                 160
   ttt gat aga cta aca aat gga aat agt cta gta agc tta acc ttt cat      528
   Phe Asp Arg Leu Thr Asn Gly Asn Ser Leu Val Ser Leu Thr Phe His
                   165                 170                 175
   tta ttt agt ctt cat gga tta att gag tac ttc cat tta gat atg atg      576
   Leu Phe Ser Leu His Gly Leu Ile Glu Tyr Phe His Leu Asp Met Met
               180                 185                 190
   aaa ctt cgt aga ttt tta gtt atg att caa gaa gat tac cac agt caa      624
   Lys Leu Arg Arg Phe Leu Val Met Ile Gln Glu Asp Tyr His Ser Gln
           195                 200                 205
   aat cct tac cat aac gca gtc cac gct gcg gat gtt act cag gcc atg      672
   Asn Pro Tyr His Asn Ala Val His Ala Ala Asp Val Thr Gln Ala Met
       210                 215                 220
   cac tgt tac tta aag gaa cct aag ctt gcc aat tct gta act cct tgg      720
   His Cys Tyr Leu Lys Glu Pro Lys Leu Ala Asn Ser Val Thr Pro Trp
   225                 230                 235                 240
   gat atc ttg ctg agc tta att gca gct gcc act cat gat ctg gat cat      768
   Asp Ile Leu Leu Ser Leu Ile Ala Ala Ala Thr His Asp Leu Asp His
                   245                 250                 255
   cca ggt gtt aat caa cct ttc ctt att aaa act aac cat tac ttg gca      816
   Pro Gly Val Asn Gln Pro Phe Leu Ile Lys Thr Asn His Tyr Leu Ala
               260                 265                 270
   act tta tac aag aat acc tca gta ctg gaa aat cac cac tgg aga tct      864
   Thr Leu Tyr Lys Asn Thr Ser Val Leu Glu Asn His His Trp Arg Ser
           275                 280                 285
   gca gtg ggc tta ttg aga gaa tca ggc tta ttc tca cat ctg cca tta      912
   Ala Val Gly Leu Leu Arg Glu Ser Gly Leu Phe Ser His Leu Pro Leu
       290                 295                 300
   gaa agc agg caa caa atg gag aca cag ata ggt gct ctg ata cta gcc      960
   Glu Ser Arg Gln Gln Met Glu Thr Gln Ile Gly Ala Leu Ile Leu Ala
   305                 310                 315                 320
   aca gac atc agt cgc cag aat gag tat ctg tct ttg ttt agg tcc cat      1008
   Thr Asp Ile Ser Arg Gln Asn Glu Tyr Leu Ser Leu Phe Arg Ser His
                   325                 330                 335
   ttg gat aga ggt gat tta tgc cta gaa gac acc aga cac aga cat ttg      1056
   Leu Asp Arg Gly Asp Leu Cys Leu Glu Asp Thr Arg His Arg His Leu
               340                 345                 350
   gtt tta cag atg gct ttg aaa tgt gct gat att tgt aac cca tgt cgg      1104
   Val Leu Gln Met Ala Leu Lys Cys Ala Asp Ile Cys Asn Pro Cys Arg
           355                 360                 365
   acg tgg gaa tta agc aag cag tgg agt gaa aaa gta acg gag gaa ttc      1152
   Thr Trp Glu Leu Ser Lys Gln Trp Ser Glu Lys Val Thr Glu Glu Phe
       370                 375                 380
   ttc cat caa gga gat ata gaa aaa aaa tat cat ttg ggt gtg agt cca      1200
   Phe His Gln Gly Asp Ile Glu Lys Lys Tyr His Leu Gly Val Ser Pro
   385                 390                 395                 400
   ctt tgc gat cgt cac act gaa tct att gcc aac atc cag att ggt aac      1248
   Leu Cys Asp Arg His Thr Glu Ser Ile Ala Asn Ile Gln Ile Gly Asn
                   405                 410                 415
   tat aca tat tta gat ata gct ggt tag aaaaatgcca ctgtttttat            1295
   Tyr Thr Tyr Leu Asp Ile Ala Gly
               420
   caagaaggga aatatatttg aaatataaaa tattaaaatt atgctcattt ctatttttaa    1355
   aaataattta agaaatttta cccttgtttt cccttgttat ggctcttcta attctcattt    1415
   aattttagga tgtaaaaagt atatttttgc agaacaggca gcagcaataa cttgtttctg    1475
   ttcttatgta aataagaatc cattattcgc tcatgtggaa gcttcttttg catcatttgg    1535
   gactgccatt taaaaaagga taggtaaaca aagaaatgac aaaaataaaa taaataaaat    1595
   aaaaatggat aggtggtgac ccactgagcc tgatcataat acgaagacca gcttctgcca    1655
   ctgcctttcc agactcttac cactgcctgt tgattaaatc taactcttca acatcctaga    1715
   caggccctta taatcttgct tcaaatgctg tgcagccatc ttgcctcaac ttccctctca    1775
   tttgcctaca gcatctcggg acgcttctgt gtttcccaag tatacgctgt tctttcgctc    1835
   tttgtgcttc gccagtgctt tccatgtgcc tcgtagagtt atttttcttg aagaggcagc    1895
   tcaaatgtca ccttctccag aagctgctct ccacttgctt taggcagagt cagtcacttt    1955
   tcttctagat tccaaagtgc ctgatccact tggttgtgga ttcctggagc ctagcaccac    2015
   accagaagca cgaggccctt gagaactgtg tgttgagtga actaataact gtattataga    2075
   aagcataatg aaaatgtcct gtgactgaag tatgtgtagc ttgttgcagg agtcacagga    2135
   aagttgacta ggattgagtg tgttgggctt tgggtataaa ggagggggat tctacggggg    2195
   cagtagctca acaaggaata gagggaggag tgtaattttg gtagctggtg ttgaataggg    2255
   cctttgagaa tcagactgaa cacagtgaaa tatgtgccca aagttcagaa agatgaagtt 2315
   tccagaaact aagaaggtag cacaatatgt ggcatcatac tcagaaagga agaccatgcc 2375
   atggggccag aaattcagaa acgtaattct tacattgtga ttgcaatgga tactcatgaa 2435
   agaaagtggg tagtggccga tttgccttca gagtgacagg tagagaaggg aagagcgtgt 2495
   agaactgtgg ccatacttta ggagtgtgag ggatgctgaa tctcccagag agctcacact 2555
   ggccaggaat gctgagagta gcagatgctt ttcttttggg aggatagtaa aacaatttag 2615
   aaccagatat gctttgtctt gattctcaag tagaataatc ttcaaatgca aaagaataca 2675
   ttagaaatgg acaaaagtgg ccaggagcgg tagctcatac ttgtaaccca gcactttggg 2735
   aagccgaggc gggctgatcg cttgaggtca ggagttcgag accagcctgg ccaaaatagt 2795
   gaaactcacg tttctactaa aaatacaaaa attagctggg tgtgatggcc acttgggagg 2855
   ctgagatagg agaatcgctt gaacctggga ggcagaggtt gcagtgagcc aatatcgtgc 2915
   cactgcattc cagcctgggt gacagaatga aactccatca ctccatctca aaaaaaaaaa 2975
   aaaaaaaaaa aaaaa 2990
<210> 4
   <211> 424
   <212> PRT
   <213> Homo sapiens
<400> 4
   Met Glu Val Cys Tyr Gln Leu Pro Val Leu Pro Leu Asp Arg Pro Val
   1               5                   10                  15
   Pro Gln His Val Leu Ser Arg Arg Gly Ala Ile Ser Phe Ser Ser Ser
               20                  25                  30
   Ser Ala Leu Phe Gly Cys Pro Asn Pro Arg Gln Leu Ser Gln Arg Arg
           35                  40                  45
   Gly Ala Ile Ser Tyr Asp Ser Ser Asp Gln Thr Ala Leu Tyr Ile Arg
       50                  55                  60
   Met Leu Gly Asp Val Arg Val Arg Ser Arg Ala Gly Phe Glu Ser Glu
   65                  70                  75                  80
   Arg Arg Gly Ser His Pro Tyr Ile Asp Phe Arg Ile Phe His Ser Gln
                   85                  90                  95
   Ser Glu Ile Glu Val Ser Val Ser Ala Arg Asn Ile Arg Arg Leu Leu
               100                 105                 110
   Ser Phe Gln Arg Tyr Leu Arg Ser Ser Arg Phe Phe Arg Gly Thr Ala
           115                 120                 125
   Val Ser Asn Ser Leu Asn Ile Leu Asp Asp Asp Tyr Asn Gly Gln Ala
       130                 135                 140
   Lys Cys Met Leu Glu Lys Val Gly Asn Trp Asn Phe Asp Ile Phe Leu
   145                 150                 155                 160
   Phe Asp Arg Leu Thr Asn Gly Asn Ser Leu Val Ser Leu Thr Phe His
                   165                 170                 175
   Leu Phe Ser Leu His Gly Leu Ile Glu Tyr Phe His Leu Asp Met Met
               180                 185                 190
   Lys Leu Arg Arg Phe Leu Val Met Ile Gln Glu Asp Tyr His Ser Gln
           195                 200                 205
   Asn Pro Tyr His Asn Ala Val His Ala Ala Asp Val Thr Gln Ala Met
       210                 215                 220
   His Cys Tyr Leu Lys Glu Pro Lys Leu Ala Asn Ser Val Thr Pro Trp
   225                 230                 235                 240
   Asp Ile Leu Leu Ser Leu Ile Ala Ala Ala Thr His Asp Leu Asp His
                   245                 250                 255
   Pro Gly Val Asn Gln Pro Phe Leu Ile Lys Thr Asn His Tyr Leu Ala
               260                 265                 270
   Thr Leu Tyr Lys Asn Thr Ser Val Leu Glu Asn His His Trp Arg Ser
           275                 280                 285
   Ala Val Gly Leu Leu Arg Glu Ser Gly Leu Phe Ser His Leu Pro Leu
       290                 295                 300
   Glu Ser Arg Gln Gln Met Glu Thr Gln Ile Gly Ala Leu Ile Leu Ala
   305                 310                 315                 320
   Thr Asp Ile Ser Arg Gln Asn Glu Tyr Leu Ser Leu Phe Arg Ser His
                   325                 330                 335
   Leu Asp Arg Gly Asp Leu Cys Leu Glu Asp Thr Arg His Arg His Leu
               340                 345                 350
   Val Leu Gln Met Ala Leu Lys Cys Ala Asp Ile Cys Asn Pro Cys Arg
           355                 360                 365
   Thr Trp Glu Leu Ser Lys Gln Trp Ser Glu Lys Val Thr Glu Glu Phe
       370                 375                 380
   Phe His Gln Gly Asp Ile Glu Lys Lys Tyr His Leu Gly Val Ser Pro
   385                 390                 395                 400
   Leu Cys Asp Arg His Thr Glu Ser Ile Ala Asn Ile Gln Ile Gly Asn
                   405                 410                 415
   Tyr Thr Tyr Leu Asp Ile Ala Gly
               420
<210> 5
   <211> 2100
   <212> DNA
   <213> Homo sapiens
<220>
   <221> CDS
   <222> (304)..(1656)
<400> 5
   cagtcagttg gtctgggcac tgcagcaggc tcggctctgt cccagcactt gtctgggaga      60
   aaagtggtgt tactcaccca gggagagtct ctctttctac cttccttctt tctcgatctc     120
   cttgtgtgct tttgtgtttc tttatttctt ttcctttttt ttcttttttt ttttttgtta      180
   cttaattata ttcctaatcc tggatgaagt tgctggattc tgcagcacaa gtcttcatga     240
   acaagcagca ccgctcagag atttcacggc attcaaaggt cacagaactg ccactatggt     300
   taa atg tct tgt tta atg gtt gag agg tgt ggc gaa atc ttg ttt gag       348
       Met Ser Cys Leu Met Val Glu Arg Cys Gly Glu Ile Leu Phe Glu
       1               5                   10                  15
   aac ccc gat cag aat gcc aaa tgt gtt tgc atg ctg gga gat ata cga       396
   Asn Pro Asp Gln Asn Ala Lys Cys Val Cys Met Leu Gly Asp Ile Arg
                   20                  25                  30
   cta agg ggt cag acg ggg gtt cgt gct gaa cgc cgt ggc tcc tac cca       444
   Leu Arg Gly Gln Thr Gly Val Arg Ala Glu Arg Arg Gly Ser Tyr Pro
               35                  40                  45
   ttc att gac ttc cgc cta ctt aac agt aca aca tac tca ggg gag att       492
   Phe Ile Asp Phe Arg Leu Leu Asn Ser Thr Thr Tyr Ser Gly Glu Ile
           50                  55                  60
   ggc acc aag aaa aag gtg aaa aga cta tta agc ttt caa aga tac ttc       540
   Gly Thr Lys Lys Lys Val Lys Arg Leu Leu Ser Phe Gln Arg Tyr Phe
       65                  70                  75
   cat gca tca agg ctg ctt cgt gga att ata cca caa gcc cct ctg cac       588
   His Ala Ser Arg Leu Leu Arg Gly Ile Ile Pro Gln Ala Pro Leu His
   80                  85                  90                  95
   ctg ctg gat gaa gac tac ctt gga caa gca agg cat atg ctc tcc aaa       636
   Leu Leu Asp Glu Asp Tyr Leu Gly Gln Ala Arg His Met Leu Ser Lys
                   100                 105                 110
   gtg gga atg tgg gat ttt gac att ttc ttg ttt gat cgc ttg aca aat        684
   Val Gly Met Trp Asp Phe Asp Ile Phe Leu Phe Asp Arg Leu Thr Asn
               115                 120                 125
   gga aac agc ctg gta aca ctg ttg tgc cac ctc ttc aat acc cat gga        732
   Gly Asn Ser Leu Val Thr Leu Leu Cys His Leu Phe Asn Thr His Gly
           130                 135                 140
   ctc att cac cat ttc aag tta gat atg gtg acc tta cac cga ttt tta        780
   Leu Ile His His Phe Lys Leu Asp Met Val Thr Leu His Arg Phe Leu
       145                 150                 155
   gtc atg gtt caa gaa gat tac cac agc caa aac ccg tat cac aat gct        828
   Val Met Val Gln Glu Asp Tyr His Ser Gln Asn Pro Tyr His Asn Ala
   160                 165                 170                 175
   gtt cac gca gcc gac gtc acc cag gcc atg cac tgc tac ctg aaa gag        876
   Val His Ala Ala Asp Val Thr Gln Ala Met His Cys Tyr Leu Lys Glu
                   180                 185                 190
   cca aag ctt gcc agc ttc ctc acg cct ctg gac atc atg ctt gga ctg        924
   Pro Lys Leu Ala Ser Phe Leu Thr Pro Leu Asp Ile Met Leu Gly Leu
               195                 200                 205
   ctg gct gca gca gca cac gat gtg gac cac cca ggg gtg aac cag cca        972
   Leu Ala Ala Ala Ala His Asp Val Asp His Pro Gly Val Asn Gln Pro
           210                 215                 220
   ttt ttg ata aaa act aac cac cat ctt gca aac cta tat cag aat atg       1020
   Phe Leu Ile Lys Thr Asn His His Leu Ala Asn Leu Tyr Gln Asn Met
       225                 230                 235
   tct gtg ctg gag aat cat cac tgg cga tct aca att ggc atg ctt cga       1068
   Ser Val Leu Glu Asn His His Trp Arg Ser Thr Ile Gly Met Leu Arg
   240                 245                 250                 255
   gaa tca agg ctt ctt gct cat ttg cca aag gaa atg aca cag gat att       1116
   Glu Ser Arg Leu Leu Ala His Leu Pro Lys Glu Met Thr Gln Asp Ile
                   260                 265                 270
   gaa cag cag ctg ggc tcc ttg atc ttg gca aca gac atc aac agg cag       1164
   Glu Gln Gln Leu Gly Ser Leu Ile Leu Ala Thr Asp Ile Asn Arg Gln
               275                 280                 285
   aat gaa ttt ttg acc aga ttg aaa gct cac ctc cac aat aaa gac tta       1212
   Asn Glu Phe Leu Thr Arg Leu Lys Ala His Leu His Asn Lys Asp Leu
           290                 295                 300
   aga ctg gag gat gca cag gac agg cac ttt atg ctt cag atc gcc ttg       1260
   Arg Leu Glu Asp Ala Gln Asp Arg His Phe Met Leu Gln Ile Ala Leu
       305                 310                 315
   aag tgt gct gac att tgc aat cct tgt aga atc tgg gag atg agc aag       1308
   Lys Cys Ala Asp Ile Cys Asn Pro Cys Arg Ile Trp Glu Met Ser Lys
   320                 325                 330                 335
   cag tgg agt gaa agg gtc tgt gaa gaa ttc tac agg caa ggt gaa ctt       1356
   Gln Trp Ser Glu Arg Val Cys Glu Glu Phe Tyr Arg Gln Gly Glu Leu
                   340                 345                 350
   gaa cag aaa ttt gaa ctg gaa atc agt cct ctt tgt aat caa cag aaa       1404
   Glu Gln Lys Phe Glu Leu Glu Ile Ser Pro Leu Cys Asn Gln Gln Lys
               355                 360                 365
   gat tcc atc cct agt ata caa att ggt ttc atg agc tac atc gtg gag     1452
   Asp Ser Ile Pro Ser Ile Gln Ile Gly Phe Met Ser Tyr Ile Val Glu
           370                 375                 380
   ccg ctc ttc cgg gaa tgg gcc cat ttc acg ggt aac agc acc ctg tcg     1500
   Pro Leu Phe Arg Glu Trp Ala His Phe Thr Gly Asn Ser Thr Leu Ser
       385                 390                 395
   gag aac atg ctg ggc cac ctc gca cac aac aag gcc cag tgg aag agc     1548
   Glu Asn Met Leu Gly His Leu Ala His Asn Lys Ala Gln Trp Lys Ser
   400                 405                 410                 415
   ctg ttg ccc agg cag cac aga agc agg ggc agc agt ggc agc ggg cct     1596
   Leu Leu Pro Arg Gln His Arg Ser Arg Gly Ser Ser Gly Ser Gly Pro
                   420                 425                 430
   gac cac gac cac gca ggc caa ggg act gag agc gag gag cag gaa ggc     1644
   Asp His Asp His Ala Gly Gln Gly Thr Glu Ser Glu Glu Gln Glu Gly
               435                 440                 445
   gac agc ccc tag gggccggccc aacttagacg cggctctcct ccggcagggc         1696
   Asp Ser Pro
           450
   ccccagaggg cagaagcagc gtggaggggc cctcacgcag cagcccagcc actttctgag   1756
   tgttgtcctg gggctctttg gaacgccatc ttcctcccac ttacctgcct cccctccttt    1816
   tcgcaaatgt acagaagcca tttgtcacct cagcattcgc tgccgaaatg agcaactcca   1876
   ttcagtaacg tgggagctga tcccacgggc aggctctccc tgctccagga gaagactagg   1936
   aggaagaatg aggtgctcct gccgtgtccg ccttgttccg ggtcgcactg gaacaggcag   1996
   caattcctaa gtccggagcg tttgagcgtt tgctatctga ctgctgatct gcgtgacaga   2056
   aacaccagca tatttgcaac gccaaggata ttggtcttaa gtgc                    2100
<210> 6
   <211> 450
   <212> PRT
   <213> Homo sapiens
<400> 6
   Met Ser Cys Leu Met Val Glu Arg Cys Gly Glu Ile Leu Phe Glu Asn
   1 5 10 15
   Pro Asp Gln Asn Ala Lys Cys Val Cys Met Leu Gly Asp Ile Arg Leu
   20 25 30
   Arg Gly Gln Thr Gly Val Arg Ala Glu Arg Arg Gly Ser Tyr Pro Phe
   35 40 45
   Ile Asp Phe Arg Leu Leu Asn Ser Thr Thr Tyr Ser Gly Glu Ile Gly
   50 55 60
   Thr Lys Lys Lys Val Lys Arg Leu Leu Ser Phe Gln Arg Tyr Phe His
   65                  70                  75                  80
   Ala Ser Arg Leu Leu Arg Gly Ile Ile Pro Gln Ala Pro Leu His Leu
                   85                  90                  95
   Leu Asp Glu Asp Tyr Leu Gly Gln Ala Arg His Met Leu Ser Lys Val
               100                 105                 110
   Gly Met Trp Asp Phe Asp Ile Phe Leu Phe Asp Arg Leu Thr Asn Gly
           115                 120                 125
   Asn Ser Leu Val Thr Leu Leu Cys His Leu Phe Asn Thr His Gly Leu
       130                 135                 140
   Ile His His Phe Lys Leu Asp Met Val Thr Leu His Arg Phe Leu Val
   145                 150                 155                 160
   Met Val Gln Glu Asp Tyr His Ser Gln Asn Pro Tyr His Asn Ala Val
                   165                 170                 175
   His Ala Ala Asp Val Thr Gln Ala Met His Cys Tyr Leu Lys Glu Pro
               180                 185                 190
   Lys Leu Ala Ser Phe Leu Thr Pro Leu Asp Ile Met Leu Gly Leu Leu
           195                 200                 205
   Ala Ala Ala Ala His Asp Val Asp His Pro Gly Val Asn Gln Pro Phe
       210                 215                 220
   Leu Ile Lys Thr Asn His His Leu Ala Asn Leu Tyr Gln Asn Met Ser
   225                 230                 235                 240
   Val Leu Glu Asn His His Trp Arg Ser Thr Ile Gly Met Leu Arg Glu
                   245                 250                 255
   Ser Arg Leu Leu Ala His Leu Pro Lys Glu Met Thr Gln Asp Ile Glu
               260                 265                 270
   Gln Gln Leu Gly Ser Leu Ile Leu Ala Thr Asp Ile Asn Arg Gln Asn
           275                 280                 285
   Glu Phe Leu Thr Arg Leu Lys Ala His Leu His Asn Lys Asp Leu Arg
       290                 295                 300
   Leu Glu Asp Ala Gln Asp Arg His Phe Met Leu Gln Ile Ala Leu Lys
   305                 310                 315                 320
   Cys Ala Asp Ile Cys Asn Pro Cys Arg Ile Trp Glu Met Ser Lys Gln
                   325                 330                 335
   Trp Ser Glu Arg Val Cys Glu Glu Phe Tyr Arg Gln Gly Glu Leu Glu
               340                 345                 350
   Gln Lys Phe Glu Leu Glu Ile Ser Pro Leu Cys Asn Gln Gln Lys Asp
           355                 360                 365
   Ser Ile Pro Ser Ile Gln Ile Gly Phe Met Ser Tyr Ile Val Glu Pro
       370                 375                 380
   Leu Phe Arg Glu Trp Ala His Phe Thr Gly Asn Ser Thr Leu Ser Glu
   385                 390                 395                 400
   Asn Met Leu Gly His Leu Ala His Asn Lys Ala Gln Trp Lys Ser Leu
                   405                 410                 415
   Leu Pro Arg Gln His Arg Ser Arg Gly Ser Ser Gly Ser Gly Pro Asp
               420                 425                 430
   His Asp His Ala Gly Gln Gly Thr Glu Ser Glu Glu Gln Glu Gly Asp
           435                 440                 445
   Ser Pro
       450